# EUROPEAN PATENT SPECIFICATION

(11) **EP 2 155 881 B1**
(45) Date of publication and mention of the grant of the patent: **08.07.2015**
(21) Application number: 08760280.1
(22) Date of filing: 30.05.2008
(51) Int. Cl.: C12N 15/82, A01H 5/00, C12N 9/22

(54) **METHOD OF EXCISING A NUCLEIC ACID SEQUENCE FROM A PLANT GENOME**
VERFAHREN ZUR EXZISION EINER NUKLEINSÄURESEQUENZ AUS EINEM PFLANZENGENOM
PROCÉDÉ D'EXCISION D'UNE SÉQUENCE D'ACIDE NUCLÉIQUE PROVENANT DU GÉNOME D'UN VÉGÉTAL

(30) Priority: 31.05.2007 US 941227 P
(43) Date of publication of application: 24.02.2010
(73) Proprietor: BASF Plant Science GmbH, 67056 Ludwigshafen (DE)
(72) Inventor: SONG, Hee-Sook, Raleigh, North Carolina 27606 (US); LAI, Fang-Ming, Cary, North Carolina 27519 (US); ROCHE, Christina E., Youngsville, North Carolina 27596 (US); BROWN, Jeffrey A., Apex, North Carolina 27502 (US)
(86) International application number: PCT/EP2008/056691
(87) International publication number: WO 2008/145731

(56) References cited:
- WO-A-03/004659
- WO-A-2006/105946
- WO-A-2007/135022
- WO-A-2008/037436
- PUCHTA H: "The repair of double-strand breaks in plants: mechanisms and consequences for genome evolution" JOURNAL OF EXPERIMENTAL BOTANY, OXFORD UNIVERSITY PRESS, GB, vol. 56, no. 409, 22 November 2004 (2004-11-22), pages 1-14, XP002394840 ISSN: 0022-0957
- SIEBERT R & PUCHTA H: "Efficient Repair of Genomic Double-Strand Breaks by Homologous Recombination between Directly Repeated Sequences in the Plant Genome" PLANT CELL, AMERICAN SOCIETY OF PLANT PHYSIOLOGISTS, ROCKVILLE, MD, US, vol. 14, 1 May 2002 (2002-05-01), pages 1121-1131, XP002236853 ISSN: 1040-4651
- GREEN C E ET AL: "PLANT REGENERATION FROM TISSUE CULTURES OF MAIZE" CROP SCIENCE, CROP SCIENCE SOCIETY OF AMERICA, MADISON, WI, US, vol. 15, no. 3, 1 January 1975 (1975-01-01), pages 417-421, XP008053283 ISSN: 0011-183X cited in the application

### SEQUENCE LISTING

<110> BASF Plant Science
<120> Method of excising a nucleic acid sequence from a plant genome
<130> B 8673 / RN
<150> 60/941,227 <151> 31.05.2007
<160> 29
<170> PatentIn version 3.3
<210> 1
   <211> 14869
   <212> DNA
   <213> Artificial
<220>
   <223> Composite binary vector: LB-Ubiquitin promoter/intron::ZmAHAS cds::ZmAHAS 3'/term-[t-nos::I-SceI cds::Zm ubiquitin promoter/intron] reverse-RB
<220>
   <221> terminator
   <222> (81)..(347)
   <223> NOS terminator (reverse orientation)
<220>
   <221> misc_feature
   <222> (363)..(1067)
   <223> I-SceI CDS (reverse orientation)
<220>
   <221> promoter
   <222> (1152)..(3139)
   <223> Zea mays Ubiquitin promoter/intron
<220>
   <221> misc_signal
   <222> (3277)..(3302)
   <223> T-DNA Left border
<220>
   <221> misc_signal
   <222> (9305)..(9330)
   <223> T-DNA Right border
<220>
   <221> promoter
   <222> (9423)..(11418)
   <223> Zea mays ubiquitin promoter and intron
<220>
   <221> misc_feature
   <222> (11451)..(13367)
   <223> AHAS CDS
<220>
   <221> terminator
   <222> (13368)..(14809)
   <223> AHAS CDS
<220>
   <221> misc_feature
   <222> (13894)..(13894)
   <223> n is a, c, g, or t
<220>
   <221> misc_feature
   <222> (13968)..(13968)
   <223> n is a, c, g, or t
<400> 1
   aaactgaagg cgggaaacga caatcagatc tagtaggaaa cagctatgac catgattacg     60
   ccaagctgcg atcgccaatt cccgatctag taacatagat gacaccgcgc gcgataattt    120
   atcctagttt gcgcgctata ttttgttttc tatcgcgtat taaatgtata attgcgggac    180
   tctaatcata aaaacccatc tcataaataa cgtcatgcat tacatgttaa ttattacatg    240
   cttaacgtaa ttcaacagaa attatatgat aatcatcgca agaccggcaa caggattcaa    300
   tcttaagaaa ctttattgcc aaatgtttga acgatcgggg aaattcgagc tcctgcaggt    360
   tatttcagga aagtttcgga ggagatagtg ttcggcagtt tgtacatcat ctgcgggatc    420
   aggtacggtt tgatcaggtt gtagaagatc aggtaagaca tagaatcgat gtagatgatc    480
   ggtttgtttt tgttgatttt tacgtaacag ttcagttgga atttgttacg cagaccctta    540
   accaggtatt ctacttcttc gaaagtgaaa gactgggtgt tcagtacgat cgatttgttg    600
   gtagagtttt tgttgtaatc ccatttacca ccatcatcca tgaaccagta tgccagagac    660
   atcggggtca ggtagttttc aaccaggttg ttcgggatgg tttttttgtt gttaacgatg    720
   aacaggctag ccagtttgtt gaaagcttgg tgtttgaaag tctgggcgcc ccaggtgatt    780
   accaggttac ccaggtggtt aacacgttct tttttgtgcg gcggggacag tacccactga    840
   tcgtacagca gacatacgtg gtccatgtat gctttgtttt tccactcgaa ctgcatacag    900
   taggttttac cttcatcacg agaacggatg taagcatcac ccaggatcag accgatacct    960
   gcttcgaact gttcgatgtt cagttcgatc agctgggatt tgtattcttt cagcagttta   1020
   gagttcggac ccaggttcat tacctggttt tttttgatgt ttttcatggc gcgggggatc   1080
   ctctagagtc gacctgcagg catgcaagct tggcgcgcct taattaaagg cctgttgggg   1140
   gtaccctgca gaagtaacac caaacaacag ggtgagcatc gacaaaagaa acagtaccaa   1200
   gcaaataaat agcgtatgaa ggcagggcta aaaaaatcca catatagctg ctgcatatgc   1260
   catcatccaa gtatatcaag atcgaaataa ttataaaaca tacttgttta ttataataga   1320
   taggtactca aggttagagc atatgaatag atgctgcata tgccatcatg tatatgcatc   1380
   agtaaaaccc acatcaacat gtatacctat cctagatcga tatttccatc catcttaaac   1440
   tcgtaactat gaagatgtat gacacacaca tacagttcca aaattaataa atacaccagg   1500
   tagtttgaaa cagtattcta ctccgatcta gaacgaatga acgaccgccc aaccacacca   1560
   catcatcaca accaagcgaa caaaaagcat ctctgtatat gcatcagtaa aacccgcatc   1620
   aacatgtata cctatcctag atcgatattt ccatccatca tcttcaattc gtaactatga   1680
   atatgtatgg cacacacata cagatccaaa attaataaat ccaccaggta gtttgaaaca   1740
   gaattctact ccgatctaga acgaccgccc aaccagacca catcatcaca accaagacaa 1800
   aaaaaagcat gaaaagatga cccgacaaac aagtgcacgg catatattga aataaaggaa 1860
   aagggcaaac caaaccctat gcaacgaaac aaaaaaaatc atgaaatcga tcccgtctgc 1920
   ggaacggcta gagccatccc aggattcccc aaagagaaac actggcaagt tagcaatcag 1980
   aacgtgtctg acgtacaggt cgcatccgtg tacgaacgct agcagcacgg atctaacaca 2040
   aacacggatc taacacaaac atgaacagaa gtagaactac cgggccctaa ccatggaccg 2100
   gaacgccgat ctagagaagg tagagagggg gggggggggg gaggacgagc ggcgtacctt 2160
   gaagcggagg tgccgacggg tggatttggg ggagatctgg ttgtgtgtgt gtgcgctccg 2220
   aacaacacga ggttggggaa agagggtgtg gagggggtgt ctatttatta cggcgggcga 2280
   ggaagggaaa gcgaaggagc ggtgggaaag gaatcccccg tagctgccgg tgccgtgaga 2340
   ggaggaggag gccgcctgcc gtgccggctc acgtctgccg ctccgccacg caatttctgg 2400
   atgccgacag cggagcaagt ccaacggtgg agcggaactc tcgagagggg tccagaggca 2460
   gcgacagaga tgccgtgccg tctgcttcgc ttggcccgac gcgacgctgc tggttcgctg 2520
   gttggtgtcc gttagactcg tcgacggcgt ttaacaggct ggcattatct actcgaaaca 2580
   agaaaaatgt ttccttagtt tttttaattt cttaaagggt atttgtttaa tttttagtca 2640
   ctttatttta ttctatttta tatctaaact attaaataaa aaaactaaaa tagagtttta 2700
   gttttcttaa tttagaggct aaaatagaat aaaatagatg tactaaaaaa attagtctat 2760
   aaaaaccatt aaccctaaac cctaaatgga tgtactaata aaatggatga agtattatat 2820
   aggtgaagct atttgcaaaa aaaaaggaga acacatgcac actaaaaaga taaaactgta 2880
   gagtcctgtt gtcaaaatac tcaattgtcc tttagaccat gtctaactgt tcatttatat 2940
   gattctctaa aacactgata ttattgtagt actatagatt atattattcg tagagtaaag 3000
   tttaaatata tgtataaaga tagataaact gcacttcaaa caagtgtgac aaaaaaaata 3060
   tgtggtaatt ttttataact tagacatgca atgctcatta tctctagaga ggggcacgac 3120
   cgggtcacgc tgcactgcag ccgcggaagc ttgcatgcct gcaggcatgc aagcttggcg 3180
   cgccttaatt aaaggcctgt taacagccgc gccactagtc aattcagtac attaaaaacg 3240
   tccgcaatgt gttattaagt tgtctaagcg tcaatttgtt tacaccacaa tatatcctgc 3300
   caccagccag ccaacagctc cccgaccggc agctcggcac aaaatcacca ctcgatacag 3360
   gcagcccatc agtccgggac ggcgtcagcg ggagagccgt tgtaaggcgg cagactttgc 3420
   tcatgttacc gatgctattc ggaagaacgg caactaagct gccgggtttg aaacacggat 3480
   gatctcgcgg agggtagcat gttgattgta acgatgacag agcgttgctg cctgtgatca 3540
   aatatcatct ccctcgcaga gatccgaatt atcagccttc ttattcattt ctcgcttaac 3600
   cgtgacaggc tgtcgatctt gagaactatg ccgacataat aggaaatcgc tggataaagc 3660
   cgctgaggaa gctgagtggc gctatttctt tagaagtgaa cgttgacgat cgtcgaccgt 3720
   accccgatga attaattcgg acgtacgttc tgaacacagc tggatactta cttgggcgat 3780
   tgtcatacat gacatcaaca atgtacccgt ttgtgtaacc gtctcttgga ggttcgtatg 3840
   acactagtgg ttcccctcag cttgcgacta gatgttgagg cctaacattt tattagagag 3900
   caggctagtt gcttagatac atgatcttca ggccgttatc tgtcagggca agcgaaaatt 3960
   ggccatttat gacgaccaat gccccgcaga agctcccatc tttgccgcca tagacgccgc 4020
   gccccccttt tggggtgtag aacatccttt tgccagatgt ggaaaagaag ttcgttgtcc 4080
   cattgttggc aatgacgtag tagccggcga aagtgcgaga cccatttgcg ctatatataa 4140
   gcctacgatt tccgttgcga ctattgtcgt aattggatga actattatcg tagttgctct 4200
   cagagttgtc gtaatttgat ggactattgt cgtaattgct tatggagttg tcgtagttgc 4260
   ttggagaaat gtcgtagttg gatggggagt agtcataggg aagacgagct tcatccacta 4320
   aaacaattgg caggtcagca agtgcctgcc ccgatgccat cgcaagtacg aggcttagaa 4380
   ccaccttcaa cagatcgcgc atagtcttcc ccagctctct aacgcttgag ttaagccgcg 4440
   ccgcgaagcg gcgtcggctt gaacgaattg ttagacatta tttgccgact accttggtga 4500
   tctcgccttt cacgtagtga acaaattctt ccaactgatc tgcgcgcgag gccaagcgat 4560
   cttcttgtcc aagataagcc tgcctagctt caagtatgac gggctgatac tgggccggca 4620
   ggcgctccat tgcccagtcg gcagcgacat ccttcggcgc gattttgccg gttactgcgc 4680
   tgtaccaaat gcgggacaac gtaagcacta catttcgctc atcgccagcc cagtcgggcg 4740
   gcgagttcca tagcgttaag gtttcattta gcgcctcaaa tagatcctgt tcaggaaccg 4800
   gatcaaagag ttcctccgcc gctggaccta ccaaggcaac gctatgttct cttgcttttg 4860
   tcagcaagat agccagatca atgtcgatcg tggctggctc gaagatacct gcaagaatgt 4920
   cattgcgctg ccattctcca aattgcagtt cgcgcttagc tggataacgc cacggaatga 4980
   tgtcgtcgtg cacaacaatg gtgacttcta cagcgcggag aatctcgctc tctccagggg 5040
   aagccgaagt ttccaaaagg tcgttgatca aagctcgccg cgttgtttca tcaagcctta 5100
   cggtcaccgt aaccagcaaa tcaatatcac tgtgtggctt caggccgcca tccactgcgg 5160
   agccgtacaa atgtacggcc agcaacgtcg gttcgagatg gcgctcgatg acgccaacta 5220
   cctctgatag ttgagtcgat acttcggcga tcaccgcttc cctcatgatg tttaactcct 5280
   gaattaagcc gcgccgcgaa gcggtgtcgg cttgaatgaa ttgttaggcg tcatcctgtg 5340
   ctcccgagaa ccagtaccag tacatcgctg tttcgttcga gacttgaggt ctagttttat 5400
   acgtgaacag gtcaatgccg ccgagagtaa agccacattt tgcgtacaaa ttgcaggcag 5460
   gtacattgtt cgtttgtgtc tctaatcgta tgccaaggag ctgtctgctt agtgcccact 5520
   ttttcgcaaa ttcgatgaga ctgtgcgcga ctcctttgcc tcggtgcgtg tgcgacacaa 5580
   caatgtgttc gatagaggct agatcgttcc atgttgagtt gagttcaatc ttcccgacaa 5640
   gctcttggtc gatgaatgcg ccatagcaag cagagtcttc atcagagtca tcatccgaga 5700
   tgtaatcctt ccggtagggg ctcacacttc tggtagatag ttcaaagcct tggtcggata 5760
   ggtgcacatc gaacacttca cgaacaatga aatggttctc agcatccaat gtttccgcca 5820
   cctgctcagg gatcaccgaa atcttcatat gacgcctaac gcctggcaca gcggatcgca 5880
   aacctggcgc ggcttttggc acaaaaggcg tgacaggttt gcgaatccgt tgctgccact 5940
   tgttaaccct tttgccagat ttggtaacta taatttatgt tagaggcgaa gtcttgggta 6000
   aaaactggcc taaaattgct ggggatttca ggaaagtaaa catcaccttc cggctcgatg 6060
   tctattgtag atatatgtag tgtatctact tgatcggggg atctgctgcc tcgcgcgttt 6120
   cggtgatgac ggtgaaaacc tctgacacat gcagctcccg gagacggtca cagcttgtct 6180
   gtaagcggat gccgggagca gacaagcccg tcagggcgcg tcagcgggtg ttggcgggtg 6240
   tcggggcgca gccatgaccc agtcacgtag cgatagcgga gtgtatactg gcttaactat 6300
   gcggcatcag agcagattgt actgagagtg caccatatgc ggtgtgaaat accgcacaga 6360
   tgcgtaagga gaaaataccg catcaggcgc tcttccgctt cctcgctcac tgactcgctg 6420
   cgctcggtcg ttcggctgcg gcgagcggta tcagctcact caaaggcggt aatacggtta 6480
   tccacagaat caggggataa cgcaggaaag aacatgtgag caaaaggcca gcaaaaggcc 6540
   aggaaccgta aaaaggccgc gttgctggcg tttttccata ggctccgccc ccctgacgag 6600
   catcacaaaa atcgacgctc aagtcagagg tggcgaaacc cgacaggact ataaagatac 6660
   caggcgtttc cccctggaag ctccctcgtg cgctctcctg ttccgaccct gccgcttacc 6720
   ggatacctgt ccgcctttct cccttcggga agcgtggcgc tttctcatag ctcacgctgt 6780
   aggtatctca gttcggtgta ggtcgttcgc tccaagctgg gctgtgtgca cgaacccccc 6840
   gttcagcccg accgctgcgc cttatccggt aactatcgtc ttgagtccaa cccggtaaga 6900
   cacgacttat cgccactggc agcagccact ggtaacagga ttagcagagc gaggtatgta 6960
   ggcggtgcta cagagttctt gaagtggtgg cctaactacg gctacactag aaggacagta 7020
   tttggtatct gcgctctgct gaagccagtt accttcggaa aaagagttgg tagctcttga 7080
   tccggcaaac aaaccaccgc tggtagcggt ggtttttttg tttgcaagca gcagattacg 7140
   cgcagaaaaa aaggatctca agaagatcct ttgatctttt ctacggggtc tgacgctcag 7200
   tggaacgaaa actcacgtta agggattttg gtcatgagat tatcaaaaag gatcttcacc 7260
   tagatccttt taaattaaaa atgaagtttt aaatcaatct aaagtatata tgagtaaact 7320
   tggtctgaca gttaccaatg cttaatcagt gaggcaccta tctcagcgat ctgtctattt 7380
   cgttcatcca tagttgcctg actccccgtc gtgtagataa ctacgatacg ggagggctta 7440
   ccatctggcc ccagtgctgc aatgataccg cgagacccac gctcaccggc tccagattta 7500
   tcagcaataa accagccagc cggaagggcc gagcgcagaa gtggtcctgc aactttatcc 7560
   gcctccatcc agtctattaa ttgttgccgg gaagctagag taagtagttc gccagttaat 7620
   agtttgcgca acgttgttgc cattgctgca gggggggggg ggggggggga cttccattgt 7680
   tcattccacg gacaaaaaca gagaaaggaa acgacagagg ccaaaaagcc tcgctttcag 7740
   cacctgtcgt ttcctttctt ttcagagggt attttaaata aaaacattaa gttatgacga 7800
   agaagaacgg aaacgcctta aaccggaaaa ttttcataaa tagcgaaaac ccgcgaggtc 7860
   gccgccccgt aacctgtcgg atcaccggaa aggacccgta aagtgataat gattatcatc 7920
   tacatatcac aacgtgcgtg gaggccatca aaccacgtca aataatcaat tatgacgcag 7980
   gtatcgtatt aattgatctg catcaactta acgtaaaaac aacttcagac aatacaaatc 8040
   agcgacactg aatacggggc aacctcatgt cccccccccc cccccccctg caggcatcgt 8100
   ggtgtcacgc tcgtcgtttg gtatggcttc attcagctcc ggttcccaac gatcaaggcg 8160
   agttacatga tcccccatgt tgtgcaaaaa agcggttagc tccttcggtc ctccgatcgt 8220
   tgtcagaagt aagttggccg cagtgttatc actcatggtt atggcagcac tgcataattc 8280
   tcttactgtc atgccatccg taagatgctt ttctgtgact ggtgagtact caaccaagtc 8340
   attctgagaa tagtgtatgc ggcgaccgag ttgctcttgc ccggcgtcaa cacgggataa 8400
   taccgcgcca catagcagaa ctttaaaagt gctcatcatt ggaaaacgtt cttcggggcg 8460
   aaaactctca aggatcttac cgctgttgag atccagttcg atgtaaccca ctcgtgcacc 8520
   caactgatct tcagcatctt ttactttcac cagcgtttct gggtgagcaa aaacaggaag 8580
   gcaaaatgcc gcaaaaaagg gaataagggc gacacggaaa tgttgaatac tcatactctt 8640
   cctttttcaa tattattgaa gcatttatca gggttattgt ctcatgagcg gatacatatt 8700
   tgaatgtatt tagaaaaata aacaaatagg ggttccgcgc acatttcccc gaaaagtgcc 8760
   acctgacgtc taagaaacca ttattatcat gacattaacc tataaaaata ggcgtatcac 8820
   gaggcccttt cgtcttcaag aattggtcga cgatcttgct gcgttcggat attttcgtgg 8880
   agttcccgcc acagacccgg attgaaggcg agatccagca actcgcgcca gatcatcctg 8940
   tgacggaact ttggcgcgtg atgactggcc aggacgtcgg ccgaaagagc gacaagcaga 9000
   tcacgctttt cgacagcgtc ggatttgcga tcgaggattt ttcggcgctg cgctacgtcc 9060
   gcgaccgcgt tgagggatca agccacagca gcccactcga ccttctagcc gacccagacg 9120
   agccaaggga tctttttgga atgctgctcc gtcgtcaggc tttccgacgt ttgggtggtt 9180
   gaacagaagt cattatcgta cggaatgcca agcactcccg aggggaaccc tgtggttggc 9240
   atgcacatac aaatggacga acggataaac cttttcacgc ccttttaaat atccgttatt 9300
   ctaataaacg ctcttttctc ttaggtttac ccgccaatat atcctgtcaa acactgatag 9360
   tttgtggaat tcgagctcgg tacccgggga tcctctagag tcgacctgca ggcatgcaag 9420
   ctttgcagtg cagcgtgacc cggtcgtgcc cctctctaga gataatgagc attgcatgtc 9480
   taagttataa aaaattacca catatttttt ttgtcacact tgtttgaagt gcagtttatc 9540
   tatctttata catatattta aactttactc tacgaataat ataatctata gtactacaat 9600
   aatatcagtg ttttagagaa tcatataaat gaacagttag acatggtcta aaggacaatt 9660
   gagtattttg acaacaggac tctacagttt tatcttttta gtgtgcatgt gttctccttt 9720
   ttttttgcaa atagcttcac ctatataata cttcatccat tttattagta catccattta 9780
   gggtttaggg ttaatggttt ttatagacta atttttttag tacatctatt ttattctatt 9840
   ttagcctcta aattaagaaa actaaaactc tattttagtt tttttattta atagtttaga 9900
   tataaaatag aataaaataa agtgactaaa aattaaacaa atacccttta agaaattaaa 9960
   aaaactaagg aaacattttt cttgtttcga gtagataatg ccagcctgtt aaacgccgtc 10020
   gacgagtcta acggacacca accagcgaac cagcagcgtc gcgtcgggcc aagcgaagca 10080
   gacggcacgg catctctgtc gctgcctctg gacccctctc gagagttccg ctccaccgtt 10140
   ggacttgctc cgctgtcggc atccagaaat tgcgtggcgg agcggcagac gtgagccggc 10200
   acggcaggcg gcctcctcct cctctcacgg caccggcagc tacgggggat tcctttccca 10260
   ccgctccttc gctttccctt cctcgcccgc cgtaataaat agacaccccc tccacaccct 10320
   ctttccccaa cctcgtgttg ttcggagcgc acacacacac aaccagatct cccccaaatc 10380
   cacccgtcgg cacctccgct tcaaggtacg ccgctcgtcc tccccccccc cccccctctc 10440
   taccttctct agatcggcgt tccggtccat ggttagggcc cggtagttct acttctgttc 10500
   atgtttgtgt tagatccgtg tttgtgttag atccgtgctg ctagcgttcg tacacggatg 10560
   cgacctgtac gtcagacacg ttctgattgc taacttgcca gtgtttctct ttggggaatc 10620
   ctgggatggc tctagccgtt ccgcagacgg gatcgatttc atgatttttt ttgtttcgtt 10680
   gcatagggtt tggtttgccc ttttccttta tttcaatata tgccgtgcac ttgtttgtcg 10740
   ggtcatcttt tcatgctttt ttttgtcttg gttgtgatga tgtggtctgg ttgggcggtc 10800
   gttctagatc ggagtagaat tctgtttcaa actacctggt ggatttatta attttggatc 10860
   tgtatgtgtg tgccatacat attcatagtt acgaattgaa gatgatggat ggaaatatcg 10920
   atctaggata ggtatacatg ttgatgcggg ttttactgat gcatatacag agatgctttt 10980
   tgttcgcttg gttgtgatga tgtggtgtgg ttgggcggtc gttcattcgt tctagatcgg 11040
   agtagaatac tgtttcaaac tacctggtgt atttattaat tttggaactg tatgtgtgtg 11100
   tcatacatct tcatagttac gagtttaaga tggatggaaa tatcgatcta ggataggtat 11160
   acatgttgat gtgggtttta ctgatgcata tacatgatgg catatgcagc atctattcat 11220
   atgctctaac cttgagtacc tatctattat aataaacaag tatgttttat aattatttcg 11280
   atcttgatat acttggatga tggcatatgc agcagctata tgtggatttt tttagccctg 11340
   ccttcatacg ctatttattt gcttggtact gtttcttttg tcgatgctca ccctgttgtt 11400
   tggtgttact tctgcagggt acccccgggg atccactagt tctagaaacc atggccaccg 11460
   ccgccgccgc gtctaccgcg ctcactggcg ccactaccgc tgcgcccaag gcgaggcgcc 11520
   gggcgcacct cctggccacc cgccgcgccc tcgccgcgcc catcaggtgc tcagcggcgt 11580
   cacccgccat gccgatggct cccccggcca ccccgctccg gccgtggggc cccaccgatg 11640
   cccgcaaggg tgctgacatc ctcgtcgagt ccctcgagcg ctgcggcgtc cgcgacgtct 11700
   tcgcctaccc cggcggcgcg tccatggaga tccaccaggc actcacccgc tcccccgtca 11760
   tcgccaacca cctcttccgc cacgagcaag gggaggcctt tgccgcctcc ggctacgcgc 11820
   gctcctcggg ccgcgtcggc gtctgcatcg ccacctccgg ccccggcgcc accaacctag 11880
   tctccgcgct cgccgacgcg ctgctcgatt ccgtccccat ggtcgccatc acgggacagg 11940
   tgccgcgacg catgattggc accgacgcct tccaggagac gcccatcgtc gaggtcaccc 12000
   gctccatcac caagcacaac tacctggtcc tcgacgtcga cgacatcccc cgcgtcgtgc 12060
   aggaggcttt cttcctcgcc tcctctggtc gaccagggcc ggtgcttgtc gacatcccca 12120
   aggacatcca gcagcagatg gcggtgcctg tctgggacaa gcccatgagt ctgcctgggt 12180
   acattgcgcg ccttcccaag ccccctgcga ctgagttgct tgagcaggtg ctgcgtcttg 12240
   ttggtgaatc gcggcgccct gttctttatg tgggcggtgg ctgcgcagca tctggtgagg 12300
   agttgcgacg ctttgtggag ctgactggaa tcccggtcac aactactctt atgggcctcg 12360
   gcaacttccc cagcgacgac ccactgtctc tgcgcatgct aggtatgcat gggacggtgt 12420
   atgcaaatta tgcagtggat aaggccgatc tgttgcttgc acttggtgtg cggtttgatg 12480
   atcgcgtgac agggaagatt gaggcttttg caagcagggc taagattgtg cacgttgata 12540
   ttgatccggc tgagattggc aagaacaagc agccacatgt gtccatctgt gcagatgtta 12600
   agcttgcttt gcagggcatg aatgctcttc ttgaaggaag cacatcaaag aagagctttg 12660
   actttggctc atggaacgat gagttggatc agcagaagag ggaattcccc cttgggtata 12720
   aaacatctaa tgaggagatc cagccacaat atgctattca ggttcttgat gagctgacga 12780
   aaggcgaggc catcatcggc acaggtgttg ggcagcacca gatgtgggcg gcacagtact 12840
   acacttacaa gcggccaagg cagtggttgt cttcagctgg tcttggggct atgggatttg 12900
   gtttgccggc tgctgctggt gcttctgtgg caaacccagg tgtcactgtt gttgacatcg 12960
   atggagatgg tagctttctc atgaacgttc aggagctagc tatgatccga attgagaacc 13020
   tcccagtgaa ggtctttgtg ctaaacaacc agcacctggg gatggtggtg cagtgggagg 13080
   acaggttcta taaggccaac agagcgcaca catacttggg aaacccagag aatgaaagtg 13140
   agatatatcc agatttcgtg acgatcgcca aagggttcaa cattccagcg gtccgtgtga 13200
   caaagaagaa cgaagtccgc gcagcgataa agaagatgct cgagactcca gggccgtacc 13260
   tcttggatat aatcgtccca caccaggagc atgtgttgcc tatgatccct aatggtgggg 13320
   ctttcaagga tatgatcctg gatggtgatg gcaggactgt gtactgatct aaaatccagc 13380
   aagcaactga tctaaaatcc agcaagcacc gcctccctgc tagtacaagg gtgatatgtt 13440
   ttatctgtgt gatgttctcc tgtgttctat ctttttttgt aggccgtcag ctatctgtta 13500
   tggtaatcct atgtagcttc cgaccttgta attgtgtagg tctgttgttt tccttctggc 13560
   atgtgtcata agagatcatt taagtgtcct tttgctacat ataaataaga taataagcac 13620
   tgctatcgag tggttctgaa ttggcttctg ttgccaaatt taagtgtcca actggtcctt 13680
   gcttttgttt tcgctatttt tttttccttt tttagttatt attatattgg taatttcaac 13740
   tcaacatatg atgtatggaa taatactagg gctgcaattt caaactattt tacaaactag 13800
   aatggcattt tcgtggtttg aggggggtga aaaaatgatc aagagtggca tttgactgaa 13860
   ttagttacct gatccaaaga aataatccat tggngcccca cttgcatccc taaggctagt 13920
   ttgaaaacat aaatctttaa tccctaaagc taatttagaa ccattggngc cccacttgca 13980
   tccctaaggc tagtttgaaa acataaatct ttaatcccta aagctaattt attttcgtgg 14040
   ttgaggggag tgaaaaaaaa tgaggcattt gactgaatta gttacctgat ccattttcgt 14100
   ggtttggatc attggaatta aattccattc taataatagt aattttggca tatatcaatt 14160
   aagttaattc ggttttatgc aaaatatatt tgtatactat tattatcaag atgtcggaga 14220
   tatttatatg ctacattttt actatacagg agtgagatga agagtgtcat gtaagttaca 14280
   cagtagaaac aaattctatt aatgcataaa atcatttcca tcatccaccc tatgaatttg 14340
   agatagacct atatctaaac tttgaaaagt ggttgaatat caaattccaa attaaataag 14400
   ttattttatt gagtgaattc taatttctct aaaacgaagg gatctaaacg ccctctaaag 14460
   ctaatttgga aactcaaact ttsttagcat tggaggggat tgagaaaaaa tattaattca 14520
   ttttcatctc aatcattcaa tctccaaaga gatttgagtt ccttattagt ctgttccatg 14580
   catcaaatcg gctcaatgtg tcattatttg ccatgacgat tgacgagttg ttctggggcc 14640
   tagcgctttc cacgccgatg tgctggggcc tggtcctgga gaagacagct tgatatttaa 14700
   agctatcaat tgtttcaatt gattcccact tcatttttct aaatgtagaa aacggtgacg  14760
   tataagaaaa agaatgaatt aggcttttat tccgtacact aatctagagc ggccccttaa  14820
   gagcgctgcg atcgcgttaa cagcttgctg aggaggcctc ggaccgtta            14869
<210> 2
   <211> 14180
   <212> DNA
   <213> Artificial
<220>
   <223> Composite binary vector: RB-Ubiquitin promoter/intron::ZmAHAS cds::ZmAHAS 3'/term-ScBV promoter::I-SceI::Nos terminator-LB
<220>
   <221> promoter
   <222> (1)..(1403)
   <223> ScBV promoter
<220>
   <221> misc_feature
   <222> (1450)..(2154)
   <223> I-SceI CDS
<220>
   <221> terminator
   <222> (2170)..(2436)
   <223> NOS terminator
<220>
   <221> misc_signal
   <222> (2586)..(2611)
   <223> T-DNA Left border
<220>
   <221> misc_signal
   <222> (8614)..(8639)
   <223> T-DNA Right border
<220>
   <221> promoter
   <222> (8732)..(10727)
   <223> Zea mays Ubiquitin promoter/intron
<220>
   <221> misc_feature
   <222> (10760)..(12676)
   <223> Zea mays AHAS cds
<220>
   <221> terminator
   <222> (12677)..(14118)
   <223> Zea mays AHAS terminator
<220>
   <221> misc_feature
   <222> (13203)..(13203)
   <223> n is a, c, g, or t
<220>
   <221> misc_feature
   <222> (13277)..(13277)
   <223> n is a, c, g, or t
<400> 2
<210> 3
   <211> 15801
   <212> DNA
   <213> Artificial
<220>
   <223> Composite binary vector: RB-Ubiquitin promoter/intron::ZmAHAS cds::ZmAHAS 3'/term-ScBV promoter::GU fragment:I-SceI site:US fragment::Nos terminator-LB
<220>
   <221> misc_signal
   <222> (176)..(199)
   <223> T-DNA Right border
<220>
   <221> promoter
   <222> (282)..(2269)
   <223> Zea mays Ubiquitin promoter/intron
<220>
   <221> misc_feature
   <222> (2309)..(4225)
   <223> Zea mays AHAS cds
<220>
   <221> terminator
   <222> (4226)..(5441)
   <223> Zea mays AHAS terminator
<220>
   <221> promoter
   <222> (5505)..(6902)
   <223> ScBV promoter
<220>
   <221> misc_feature
   <222> (6968)..(7588)
   <223> "GU" portion of GUS cds
<220>
   <221> repeat_region
   <222> (6979)..(7588)
   <223> HR target repeat
<220>
   <221> misc_signal
   <222> (7601)..(7618)
   <223> I-SceI recognition site
<220>
   <221> misc_feature
   <222> (7706)..(9505)
   <223> "US" portion of GUS cds
<220>
   <221> repeat_region
   <222> (7706)..(8315)
   <223> HR target repeat
<220>
   <221> terminator
   <222> (9611)..(9786)
   <223> NOS terminator
<220>
   <221> misc_signal
   <222> (9929)..(9953)
   <223> T-DNA Left border
<400> 3
   gtcgtcaggc tttccgacgt ttgggtggtt gaacagaagt cattatcgca cggaatgcca      60
   agcactcccg aggggaaccc tgtggttggc atgcacatac aaatggacga acggataaac     120
   cttttcacgc ccttttaaat atccgattat tctaataaac gctcttttct cttaggttta     180
   cccgccaata tatcctgtca aacactgata gtttgtggaa ttcgagctcg gtacccgggg     240
   atcctctaga gtcgacctgc aggcatgcaa gcttccgcgg ctgcagtgca gcgtgacccg     300
   gtcgtgcccc tctctagaga taatgagcat tgcatgtcta agttataaaa aattaccaca     360
   tatttttttt gtcacacttg tttgaagtgc agtttatcta tctttataca tatatttaaa      420
   ctttactcta cgaataatat aatctatagt actacaataa tatcagtgtt ttagagaatc     480
   atataaatga acagttagac atggtctaaa ggacaattga gtattttgac aacaggactc     540
   tacagtttta tctttttagt gtgcatgtgt tctccttttt ttttgcaaat agcttcacct     600
   atataatact tcatccattt tattagtaca tccatttagg gtttagggtt aatggttttt     660
   atagactaat ttttttagta catctatttt attctatttt agcctctaaa ttaagaaaac     720
   taaaactcta ttttagtttt tttatttaat agtttagata taaaatagaa taaaataaag     780
   tgactaaaaa ttaaacaaat accctttaag aaattaaaaa aactaaggaa acatttttct      840
   tgtttcgagt agataatgcc agcctgttaa acgccgtcga cgagtctaac ggacaccaac     900
   cagcgaacca gcagcgtcgc gtcgggccaa gcgaagcaga cggcacggca tctctgtcgc     960
   tgcctctgga cccctctcga gagttccgct ccaccgttgg acttgctccg ctgtcggcat    1020
   ccagaaattg cgtggcggag cggcagacgt gagccggcac ggcaggcggc ctcctcctcc    1080
   tctcacggca ccggcagcta cgggggattc ctttcccacc gctccttcgc tttcccttcc    1140
   tcgcccgccg taataaatag acaccccctc cacaccctct ttccccaacc tcgtgttgtt    1200
   cggagcgcac acacacacaa ccagatctcc cccaaatcca cccgtcggca cctccgcttc    1260
   aaggtacgcc gctcgtcctc cccccccccc cccctctcta ccttctctag atcggcgttc 1320
   cggtccatgg ttagggcccg gtagttctac ttctgttcat gtttgtgtta gatccgtgtt 1380
   tgtgttagat ccgtgctgct agcgttcgta cacggatgcg acctgtacgt cagacacgtt 1440
   ctgattgcta acttgccagt gtttctcttt ggggaatcct gggatggctc tagccgttcc 1500
   gcagacggga tcgatttcat gatttttttt gtttcgttgc atagggtttg gtttgccctt 1560
   ttcctttatt tcaatatatg ccgtgcactt gtttgtcggg tcatcttttc atgctttttt 1620
   ttgtcttggt tgtgatgatg tggtctggtt gggcggtcgt tctagatcgg agtagaattc 1680
   tgtttcaaac tacctggtgg atttattaat tttggatctg tatgtgtgtg ccatacatat 1740
   tcatagttac gaattgaaga tgatggatgg aaatatcgat ctaggatagg tatacatgtt 1800
   gatgcgggtt ttactgatgc atatacagag atgctttttg ttcgcttggt tgtgatgatg 1860
   tggtgtggtt gggcggtcgt tcattcgttc tagatcggag tagaatactg tttcaaacta 1920
   cctggtgtat ttattaattt tggaactgta tgtgtgtgtc atacatcttc atagttacga 1980
   gtttaagatg gatggaaata tcgatctagg ataggtatac atgttgatgt gggttttact 2040
   gatgcatata catgatggca tatgcagcat ctattcatat gctctaacct tgagtaccta 2100
   tctattataa taaacaagta tgttttataa ttatttcgat cttgatatac ttggatgatg 2160
   gcatatgcag cagctatatg tggatttttt tagccctgcc ttcatacgct atttatttgc 2220
   ttggtactgt ttcttttgtc gatgctcacc ctgttgtttg gtgttacttc tgcagggtac 2280
   ccccggggat ccactagttc tagaaaccat ggccaccgcc gccgccgcgt ctaccgcgct 2340
   cactggcgcc actaccgctg cgcccaaggc gaggcgccgg gcgcacctcc tggccacccg 2400
   ccgcgccctc gccgcgccca tcaggtgctc agcggcgtca cccgccatgc cgatggctcc 2460
   cccggccacc ccgctccggc cgtggggccc caccgatccc cgcaagggcg ccgacatcct 2520
   cgtcgagtcc ctcgagcgct gcggcgtccg cgacgtcttc gcctaccccg gcggcgcgtc 2580
   catggagatc caccaggcac tcacccgctc ccccgtcatc gccaaccacc tcttccgcca 2640
   cgagcaaggg gaggcctttg cggcctccgg ctacgcgcgc tcctcgggcc gcgtcggcgt 2700
   ctgcatcgcc acctccggcc ccggcgccac caaccttgtc tccgcgctcg ccgacgcgct 2760
   gctcgattcc gtccccatgg tcgccatcac gggacaggtg ccgcgacgca tgattggcac 2820
   cgacgccttc caggagacgc ccatcgtcga ggtcacccgc tccatcacca agcacaacta 2880
   cctggtcctc gacgtcgacg acatcccccg cgtcgtgcag gaggctttct tcctcgcctc 2940
   ctctggtcga ccggggccgg tgcttgtcga catccccaag gacatccagc agcagatggc 3000
   ggtgcctgtc tgggacaagc ccatgagtct gcctgggtac attgcgcgcc ttcccaagcc 3060
   ccctgcgact gagttgcttg agcaggtgct gcgtcttgtt ggtgaatccc ggcgccctgt 3120
   tctttatgtt ggcggtggct gcgcagcatc tggtgaggag ttgcgacgct ttgtggagct 3180
   gactggaatc ccggtcacaa ctactcttat gggcctcggc aacttcccca gcgacgaccc 3240
   actgtctctg cgcatgctag gtatgcatgg cacggtgtat gcaaattatg cagtggataa 3300
   ggccgatctg ttgcttgcac ttggtgtgcg gtttgatgat cgtgtgacag ggaagattga 3360
   ggcttttgca agcagggcta agattgtgca cgttgatatt gatccggctg agattggcaa 3420
   gaacaagcag ccacatgtgt ccatctgtgc agatgttaag cttgctttgc agggcatgaa 3480
   tgctcttctt gaaggaagca catcaaagaa gagctttgac tttggctcat ggaacgatga 3540
   gttggatcag cagaagaggg aattccccct tgggtataaa acatctaatg aggagatcca 3600
   gccacaatat gctattcagg ttcttgatga gctgacgaaa ggcgaggcca tcatcggcac 3660
   aggtgttggg cagcaccaga tgtgggcggc acagtactac acttacaagc ggccaaggca 3720
   gtggttgtct tcagctggtc ttggggctat gggatttggt ttgccggctg ctgctggtgc 3780
   ttctgtggcc aacccaggtg ttactgttgt tgacatcgat ggagatggta gctttctcat 3840
   gaacgttcag gagctagcta tgatccgaat tgagaacctc ccggtgaagg tctttgtgct 3900
   aaacaaccag cacctgggga tggtggtgca gtgggaggac aggttctata aggccaacag 3960
   agcgcacaca tacttgggaa acccagagaa tgaaagtgag atatatccag atttcgtgac 4020
   gatcgccaaa gggttcaaca ttccagcggt ccgtgtgaca aagaagaacg aagtccgcgc 4080
   agcgataaag aagatgctcg agactccagg gccgtacctc ttggatataa tcgtcccaca 4140
   ccaggagcat gtgttgccta tgatccctaa tggtggggct ttcaaggata tgatcctgga 4200
   tggtgatggc aggactgtgt actgatctaa aatccagcaa gcaactgatc taaaatccag 4260
   caagcaccgc ctccctgcta gtacaagggt gatatgtttt tatctgtgtg atgttctcct 4320
   gtattctatc tttttttgta ggccgtcagc tatctgttat ggtaatccta tgtagcttcc 4380
   gaccttgtaa ttgtgtagtc tgttgttttc cttctggcat gtgtcataag agatcattta 4440
   agtgcctttt gctacatata aataagataa taagcactgc tatgcagtgg ttctgaattg 4500
   gcttctgttg ccaaatttaa gtgtccaact ggtccttgct tttgttttcg ctattttttt 4560
   ccttttttag ttattattat attggtaatt tcaactcaac atatgatgta tggaataatg 4620
   ctagggctgc aatttcaaac tattttacaa accagaatgg cattttcgtg gtttgagggg 4680
   agtgaaaaaa aatgaggcat ttgactgaat tagttacctg atccattttc gtggtttgga 4740
   tcattggaat taaattccat tctaataata gtaattttgg catatatcaa ttaagttaat 4800
   tcggttttat gcaaaatata tttgtatact attattatca agatgtcgga gatatttata 4860
   tgctacattt ttactataca ggagtgagat gaagagtgtc atgtaagtta cacagtagaa 4920
   acaaattcta ttaatgcata aaatcatttc catcatccac cctatgaatt tgagatagac 4980
   ctatatctaa actttgaaaa gtggttgaat atcaaattcc aaattaaata agttatttta 5040
   ttgagtgaat tctaatttct ctaaaacgaa gggatctaaa cgccctctaa agctaatttg 5100
   gaaactcaaa ctttcttagc attggagggg attgagaaaa aatattaatt cattttcatc 5160
   tcaatcattc aatctccaaa gagatttgag ttccttatta gtctgttcca tgcatcaaat 5220
   cggctcaatg tgtcattatt tgccatgacg attgacgagt tgttctgggg cctagcgctt 5280
   tccacgccga tgtgctgggg cctggtcctg gagaagacag cttgatattt aaagctatca 5340
   attgtttcaa ttgattccca cttcattttt ctaaatgtag aaaacggtga cgtataagaa 5400
   aaagaatgaa ttaggacttt tattccgtac actaatctag agcggcccct taaggcgctg 5460
   cgatcgcgtt aacagcttgc tgaggaggcc tcggaccgtt aattaatcct ggctagcaac 5520
   actgaactat gccagaaacc acatcaaaga tatgggcaag cttcttggcc cattatatcc 5580
   aaagacctca gagaaaggtg agcgaaggct caattcagaa gattggaagc tgatcaatag 5640
   gatcaagaca atggtgagaa cgcttccaaa tctcactatt ccaccagaag atgcatacat 5700
   tatcattgaa acagatgcat gtgcaactgg atggggagca gtatgcaagt ggaagaaaaa 5760
   caaggcagac ccaagaaata cagagcaaat ctgtaggtat gccagtggaa aatttgataa 5820
   gccaaaagga acctgtgatg cagaaatcta tggggttatg aatggcttag aaaagatgag 5880
   attgttctac ttggacaaaa gagagatcac agtcagaact gacagtagtg caatcgaaag 5940
   gttctacaac aagagtgctg aacacaagcc ttctgagatc agatggatca ggttcatgga 6000
   ctacatcact ggtgcaggac cagagatagt cattgaacac ataaaaggga agagcaatgg 6060
   tttagctgac atcttgtcca ggctcaaagc caaattagct cagaatgaac caacggaaga 6120
   gatgatcctg cttacacaag ccataaggga agtaattcct tatccagatc atccatacac 6180
   tgagcaactc agagaatggg gaaacaaaat tctggatcca ttccccacat tcaagaagga 6240
   catgttcgaa agaacagagc aagcttttat gctaacagag gaaccagttc tactctgtgc 6300
   atgcaggaag cctgcaattc agttagtgtc cagaacatct gccaacccag gaaggaaatt 6360
   cttcaagtgc gcaatgaaca aatgccattg ctggtactgg gcagatctca ttgaagaaca 6420
   cattcaagac agaattgatg aatttctcaa gaatcttgaa gttctgaaga ccggtggcgt 6480
   gcaaacaatg gaggaggaac ttatgaagga agtcaccaag ctgaagatag aagagcagga 6540
   gttcgaggaa taccaggcca caccaagggc tatgtcgcca gtagccgcag aagatgtgct 6600
   agatctccaa gacgtaagca atgacgattg aggaggcatt gacgtcaggg atgaccgcag 6660
   cggagagtac tgggcccatt cagtggatgc tccactgagt tgtattattg tgtgcttttc 6720
   ggacaagtgt gctgtccact ttcttttggc acctgtgcca ctttattcct tgtctgccac 6780
   gatgcctttg cttagcttgt aagcaaggat cgcagtgcgt gtgtgacacc accccccttc 6840
   cgacgctctg cctatataag gcaccgtctg taagctctta cgatcatcgg tagttcacca 6900
   aggtaccggg ccccccctcg aggtcgacgg tatcgataag cttgatctag tctagagtcg 6960
   atcgaccatg gtacgtcctg tagaaacccc aacccgtgaa atcaaaaaac tcgacggcct 7020
   gtgggcattc agtctggatc gcgaaaactg tggaattggt cagcgttggt gggaaagcgc 7080
   gttacaagaa agccgggcaa ttgctgtgcc aggcagtttt aacgatcagt tcgccgatgc 7140
   agatattcgt aattatgcgg gcaacgtctg gtatcagcgc gaagtcttta taccgaaagg 7200
   ttgggcaggc cagcgtatcg tgctgcgttt cgatgcggtc actcattacg gcaaagtgtg 7260
   ggtcaataat caggaagtga tggagcatca gggcggctat acgccatttg aagccgatgt 7320
   cacgccgtat gttattgccg ggaaaagtgt acgtatcacc gtttgtgtga acaacgaact 7380
   gaactggcag actatcccgc cgggaatggt gattaccgac gaaaacggca agaaaaagca 7440
   gtcttacttc catgatttct ttaactatgc cggaatccat cgcagcgtaa tgctctacac 7500
   cacgccgaac acctgggtgg acgatatcac cgtggtgacg catgtcgcgc aagactgtaa 7560
   ccacgcgtct gttgactggc aggtggtgcc agcggccgcc tagggataac agggtaatag 7620
   tctagtccga aaacgccgtg agacatattg gttacgatcc taaggtagcg aaattcaccc 7680
   ggtaactctg tgccagctag agtcctgtag aaaccccaac ccgtgaaatc aaaaaactcg 7740
   acggcctgtg ggcattcagt ctggaccgcg aaaactgtgg aattgatcag cgttggtggg 7800
   aaagcgcgtt acaagaaagc cgggcaattg ctgtgccagg cagttttaac gatcagttcg 7860
   ccgatgcaga tattcgtaat tatgcgggca acgtctggta tcagcgcgaa gtctttatac 7920
   cgaaaggttg ggcaggccag cgtatcgtgc tgcgtttcga tgcggtcact cattacggca 7980
   aagtgtgggt caataatcag gaagtgatgg agcatcaggg cggctatacg ccatttgaag 8040
   ccgatgtcac gccgtatgtt attgccggga aaagtgtacg tatcaccgtt tgtgtgaaca 8100
   acgaactgaa ctggcagact atcccgccgg gaatggtgat taccgacgaa aacggcaaga 8160
   aaaagcagtc ttacttccat gatttcttta actatgccgg aatccatcgc agcgtaatgc 8220
   tctacaccac gccgaacacc tgggtggacg atatcaccgt ggtgacgcat gtcgcgcaag 8280
   actgtaacca cgcgtctgtt gactggcagg tggtggccaa tggtgatgtc agcgttgaac 8340
   tgcgtgatgc ggatcaacag gtggttgcaa ctggacaagg cactagcggg actttgcaag 8400
   tggtgaatcc gcacctctgg caccgggtga aggttatctc tatgaactgt gcgtcacagc 8460
   caaaagccag acagagtgtg atatctaccc gcttcgcgtc ggcatccggt cagtggcagt 8520
   gaagggcgaa cagttcctga ttaaccacaa accgttctac tttactggct ttggtcgtca 8580
   tgaagatgcg gacttgcgtg gcaaaggatt cgataacgtg ctgatggtgc acgaccacgc 8640
   attaatggac tggattgggg ccaactccta ccgtacctcg cattaccctt acgctgaaga 8700
   gatgctcgac tgggcagatg aacatggcat cgtggtgatt gatgaaactg ctgctgtcgg 8760
   ctttaacctc tctttaggca ttggtttcga agcgggcaac aagccgaaag aactgtacag 8820
   cgaagaggca gtcaacgggg aaactcagca agcgcactta caggcgatta aagagctgat 8880
   agcgcgtgac aaaaaccacc caagcgtggt gatgtggagt attgccaacg aaccggatac 8940
   ccgtccgcaa ggtgcacggg aatatttcgc gccactggcg gaagcaacgc gtaaactcga 9000
   cccgacgcgt ccgatcacct gcgtcaatgt aatgttctgc gacgctcaca ccgataccat 9060
   cagcgatctc tttgatgtgc tgtgcctgaa ccgttattac ggatggtatg tccaaagcgg 9120
   cgatttggaa gcggcagaga aggtactgga aaaagaactt ctggcctggc aggagaaact 9180
   gcatcagccg attatcatca ccgaatacgg cgtggatacg ttagccgggc tgcactcaat 9240
   gtacaccgac atgtggagtg aagagtatca gtgtgcatgg ctggatatgt atcaccgcgt 9300
   ctttgatcgc gtcagcgccg tcgtcggtga acaggtatgg aatttcgccg attttgcgac 9360
   ctcgcaaggc atattgcgcg ttggcggtaa caagaaaggg atcttcactc gcgaccgcaa 9420
   accgaagtcg gcggcttttc tgctgcaaaa acgctggact ggcatgaact tcggtgaaaa 9480
   accgcagcag ggaggcaaac aatgaatcaa caactctcct ggcgcaccat cgtcggctac 9540
   agcctcggga attgctaccg agctcgaatt tccccgatcg ttcaaacatt tggcaataaa 9600
   gtttcttaag attgaatcct gttgccggac ttgcgatgat tatcatataa tttctgttga 9660
   attacgttaa gcatgtaata attaacatgt aatgcatgac gttatttatg agatgggttt 9720
   ttatgattag agtcccgcaa ttatacattt aatacgcgat agaaaacaaa atatagcgcg 9780
   caaactagga taaattatcg cgcgcggtgt catctatgtt actagatcgg aataagctta 9840
   tcgataccgt cgacctcgag cgccactagt caattcagta cattaaaaac gtccgcaatg 9900
   tgttattaag ttgtctaagc gtcaatttgt ttacaccaca atatatcctg ccaccagcca 9960
   gccaacagct ccccgaccgg cagctcggca caaaatcacc actcgataca ggcagcccat 10020
   cagtccggga cggcgtcagc gggagagccg ttgtaaggcg gcagactttg ctcatgttac 10080
   cgatgctatt cggaagaacg gcaactaagc tgccgggttt gaaacacgga tgatctcgcg 10140
   gagggtagca tgttgattgt aacgatgaca gagcgttgct gcctgtgatc aaatatcatc 10200
   tccctcgcag agatccgaat tatcagcctt cttattcatt tctcgcttaa ccgtgacagg 10260
   ctgtcgatct tgagaactat gccgacataa taggaaatcg ctggataaag ccgctgagga 10320
   agctgagtgg cgctatttct ttagaagtga acgttgacga tcgtcgaccg taccccgatg 10380
   aattaattcg gacgtacgtt ctgaacacag ctggatactt acttgggcga ttgtcataca 10440
   tgacatcaac aatgtacccg tttgtgtaac cgtctcttgg aggttcgtat gacactagtg 10500
   gttcccctca gcttgcgact agatgttgag gcctaacatt ttattagaga gcaggctagt 10560
   tgcttagata catgatcttc aggccgttat ctgtcagggc aagcgaaaat tggccattta 10620
   tgacgaccaa tgccccgcag aagctcccat ctttgccgcc atagacgccg cgcccccctt 10680
   ttggggtgta gaacatcctt ttgccagatg tggaaaagaa gttcgttgtc ccattgttgg 10740
   caatgacgta gtagccggcg aaagtgcgag acccatttgc gctatatata agcctacgat 10800
   ttccgttgcg actattgtcg taattggatg aactattatc gtagttgctc tcagagttgt 10860
   cgtaatttga tggactattg tcgtaattgc ttatggagtt gtcgtagttg cttggagaaa 10920
   tgtcgtagtt ggatggggag tagtcatagg gaagacgagc ttcatccact aaaacaattg 10980
   gcaggtcagc aagtgcctgc cccgatgcca tcgcaagtac gaggcttaga accaccttca 11040
   acagatcgcg catagtcttc cccagctctc taacgcttga gttaagccgc gccgcgaagc 11100
   ggcgtcggct tgaacgaatt gttagacatt atttgccgac taccttggtg atctcgcctt 11160
   tcacgtagtg aacaaattct tccaactgat ctgcgcgcga ggccaagcga tcttcttgtc 11220
   caagataagc ctgcctagct tcaagtatga cgggctgata ctgggccggc aggcgctcca 11280
   ttgcccagtc ggcagcgaca tccttcggcg cgattttgcc ggttactgcg ctgtaccaaa 11340
   tgcgggacaa cgtaagcact acatttcgct catcgccagc ccagtcgggc ggcgagttcc 11400
   atagcgttaa ggtttcattt agcgcctcaa atagatcctg ttcaggaacc ggatcaaaga 11460
   gttcctccgc cgctggacct accaaggcaa cgctatgttc tcttgctttt gtcagcaaga 11520
   tagccagatc aatgtcgatc gtggctggct cgaagatacc tgcaagaatg tcattgcgct 11580
   gccattctcc aaattgcagt tcgcgcttag ctggataacg ccacggaatg atgtcgtcgt 11640
   gcacaacaat ggtgacttct acagcgcgga gaatctcgct ctctccaggg gaagccgaag 11700
   tttccaaaag gtcgttgatc aaagctcgcc gcgttgtttc atcaagcctt acggtcaccg 11760
   taaccagcaa atcaatatca ctgtgtggct tcaggccgcc atccactgcg gagccgtaca 11820
   aatgtacggc cagcaacgtc ggttcgagat ggcgctcgat gacgccaact acctctgata 11880
   gttgagtcga tacttcggcg atcaccgctt ccctcatgat gtttaactcc tgaattaagc 11940
   cgcgccgcga agcggtgtcg gcttgaatga attgttaggc gtcatcctgt gctcccgaga 12000
   accagtacca gtacatcgct gtttcgttcg agacttgagg tctagtttta tacgtgaaca 12060
   ggtcaatgcc gccgagagta aagccacatt ttgcgtacaa attgcaggca ggtacattgt 12120
   tcgtttgtgt ctctaatcgt atgccaagga gctgtctgct tagtgcccac tttttcgcaa 12180
   attcgatgag actgtgcgcg actcctttgc ctcggtgcgt gtgcgacaca acaatgtgtt 12240
   cgatagaggc tagatcgttc catgttgagt tgagttcaat cttcccgaca agctcttggt 12300
   cgatgaatgc gccatagcaa gcagagtctt catcagagtc atcatccgag atgtaatcct 12360
   tccggtaggg gctcacactt ctggtagata gttcaaagcc ttggtcggat aggtgcacat 12420
   cgaacacttc acgaacaatg aaatggttct cagcatccaa tgtttccgcc acctgctcag 12480
   ggatcaccga aatcttcata tgacgcctaa cgcctggcac agcggatcgc aaacctggcg 12540
   cggcttttgg cacaaaaggc gtgacaggtt tgcgaatccg ttgctgccac ttgttaaccc 12600
   ttttgccaga tttggtaact ataatttatg ttagaggcga agtcttgggt aaaaactggc 12660
   ctaaaattgc tggggatttc aggaaagtaa acatcacctt ccggctcgat gtctattgta 12720
   gatatatgta gtgtatctac ttgatcgggg gatctgctgc ctcgcgcgtt tcggtgatga 12780
   cggtgaaaac ctctgacaca tgcagctccc ggagacggtc acagcttgtc tgtaagcgga 12840
   tgccgggagc agacaagccc gtcagggcgc gtcagcgggt gttggcgggt gtcggggcgc 12900
   agccatgacc cagtcacgta gcgatagcgg agtgtatact ggcttaacta tgcggcatca 12960
   gagcagattg tactgagagt gcaccatatg cggtgtgaaa taccgcacag atgcgtaagg 13020
   agaaaatacc gcatcaggcg ctcttccgct tcctcgctca ctgactcgct gcgctcggtc 13080
   gttcggctgc ggcgagcggt atcagctcac tcaaaggcgg taatacggtt atccacagaa 13140
   tcaggggata acgcaggaaa gaacatgtga gcaaaaggcc agcaaaaggc caggaaccgt 13200
   aaaaaggccg cgttgctggc gtttttccat aggctccgcc cccctgacga gcatcacaaa 13260
   aatcgacgct caagtcagag gtggcgaaac ccgacaggac tataaagata ccaggcgttt 13320
   ccccctggaa gctccctcgt gcgctctcct gttccgaccc tgccgcttac cggatacctg 13380
   tccgcctttc tcccttcggg aagcgtggcg ctttctcata gctcacgctg taggtatctc 13440
   agttcggtgt aggtcgttcg ctccaagctg ggctgtgtgc acgaaccccc cgttcagccc 13500
   gaccgctgcg ccttatccgg taactatcgt cttgagtcca acccggtaag acacgactta 13560
   tcgccactgg cagcagccac tggtaacagg attagcagag cgaggtatgt aggcggtgct 13620
   acagagttct tgaagtggtg gcctaactac ggctacacta gaaggacagt atttggtatc 13680
   tgcgctctgc tgaagccagt taccttcgga aaaagagttg gtagctcttg atccggcaaa 13740
   caaaccaccg ctggtagcgg tggttttttt gtttgcaagc agcagattac gcgcagaaaa 13800
   aaaggatctc aagaagatcc tttgatcttt tctacggggt ctgacgctca gtggaacgaa 13860
   aactcacgtt aagggatttt ggtcatgaga ttatcaaaaa ggatcttcac ctagatcctt 13920
   ttaaattaaa aatgaagttt taaatcaatc taaagtatat atgagtaaac ttggtctgac 13980
   agttaccaat gcttaatcag tgaggcacct atctcagcga tctgtctatt tcgttcatcc 14040
   atagttgcct gactccccgt cgtgtagata actacgatac gggagggctt accatctggc 14100
   cccagtgctg caatgatacc gcgagaccca cgctcaccgg ctccagattt atcagcaata 14160
   aaccagccag ccggaagggc cgagcgcaga agtggtcctg caactttatc cgcctccatc 14220
   cagtctatta attgttgccg ggaagctaga gtaagtagtt cgccagttaa tagtttgcgc 14280
   aacgttgttg ccattgctgc aggggggggg gggggggggg acttccattg ttcattccac 14340
   ggacaaaaac agagaaagga aacgacagag gccaaaaagc ctcgctttca gcacctgtcg 14400
   tttcctttct tttcagaggg tattttaaat aaaaacatta agttatgacg aagaagaacg 14460
   gaaacgcctt aaaccggaaa attttcataa atagcgaaaa cccgcgaggt cgccgccccg 14520
   taacctgtcg gatcaccgga aaggacccgt aaagtgataa tgattatcat ctacatatca 14580
   caacgtgcgt ggaggccatc aaaccacgtc aaataatcaa ttatgacgca ggtatcgtat 14640
   taattgatct gcatcaactt aacgtaaaaa caacttcaga caatacaaat cagcgacact 14700
   gaatacgggg caacctcatg tccccccccc ccccccccct gcaggcatcg tggtgtcacg 14760
   ctcgtcgttt ggtatggctt cattcagctc cggttcccaa cgatcaaggc gagttacatg 14820
   atcccccatg ttgtgcaaaa aagcggttag ctccttcggt cctccgatcg ttgtcagaag 14880
   taagttggcc gcagtgttat cactcatggt tatggcagca ctgcataatt ctcttactgt 14940
   catgccatcc gtaagatgct tttctgtgac tggtgagtac tcaaccaagt cattctgaga 15000
   atagtgtatg cggcgaccga gttgctcttg cccggcgtca acacgggata ataccgcgcc 15060
   acatagcaga actttaaaag tgctcatcat tggaaaacgt tcttcggggc gaaaactctc 15120
   aaggatctta ccgctgttga gatccagttc gatgtaaccc actcgtgcac ccaactgatc 15180
   ttcagcatct tttactttca ccagcgtttc tgggtgagca aaaacaggaa ggcaaaatgc 15240
   cgcaaaaaag ggaataaggg cgacacggaa atgttgaata ctcatactct tcctttttca 15300
   atattattga agcatttatc agggttattg tctcatgagc ggatacatat ttgaatgtat 15360
   ttagaaaaat aaacaaatag gggttccgcg cacatttccc cgaaaagtgc cacctgacgt 15420
   ctaagaaacc attattatca tgacattaac ctataaaaat aggcgtatca cgaggccctt 15480
   tcgtcttcaa gaattggtcg acgatcttgc tgcgttcgga tattttcgtg gagttcccgc 15540
   cacagacccg gattgaaggc gagatccagc aactcgcgcc agatcatcct gtgacggaac  15600
   tttggcgcgt gatgactggc caggacgtcg gccgaaagag cgacaagcag atcacgcttt  15660
   tcgacagcgt cggatttgcg atcgaggatt tttcggcgct gcgctacgtc cgcgaccgcg  15720
   ttgagggatc aagccacagc agcccactcg accttctagc cgacccagac gagccaaggg  15780
   atctttttgg aatgctgctc c                                            15801
<210> 4
   <211> 16447
   <212> DNA
   <213> Artificial
<220>
   <223> Composite binary vector: LB-Ubiquitin promoter/intron::ZmAHAS cds::ZmAHAS 3'/term-ScBV promoter::GUS cds::Nos terminator-HR repeat-RB
<220>
   <221> misc_signal
   <222> (1)..(25)
   <223> T-DNA Left border
<220>
   <221> promoter
   <222> (135)..(2122)
   <223> Zea mays Ubiquitin promoter/intron
<220>
   <221> misc_feature
   <222> (2131)..(4102)
   <223> Zea mays AHAS cds
<220>
   <221> repeat_region
   <222> (4098)..(4905)
<220>
   <221> terminator
   <222> (4103)..(5314)
   <223> Zea mays AHAS terminator
<220>
   <221> misc_signal
   <222> (5412)..(5429)
   <223> I-SceI recognition site
<220>
   <221> promoter
   <222> (5461)..(6881)
   <223> ScBV promoter
<220>
   <221> misc_feature
   <222> (6982)..(8902)
   <223> GUS CDS with embedded PIV2 intron
<220>
   <221> Intron
   <222> (7287)..(7475)
   <223> PIV2 intron
<220>
   <221> terminator
   <222> (8973)..(9225)
   <223> NOS terminator
<220>
   <221> misc_signal
   <222> (9488)..(9505)
   <223> I-SceI recognition site
<220>
   <221> repeat_region
   <222> (9521)..(10351)
<220>
   <221> misc_signal
   <222> (10406)..(10429)
   <223> I-SceI recognition site
<400> 4
   tggcaggata tattgtggtg taaacaaatt gacgcttaga caacttaata acacattgcg      60
   gacgttttta atgtactgaa ttgactagtg gcgcgccaag cttgcatgcc tgcaggcatg     120
   caagcttccg cggctgcagt gcagcgtgac ccggtcgtgc ccctctctag agataatgag     180
   cattgcatgt ctaagttata aaaaattacc acatattttt tttgtcacac ttgtttgaag     240
   tgcagtttat ctatctttat acatatattt aaactttact ctacgaataa tataatctat     300
   agtactacaa taatatcagt gttttagaga atcatataaa tgaacagtta gacatggtct     360
   aaaggacaat tgagtatttt gacaacagga ctctacagtt ttatcttttt agtgtgcatg     420
   tgttctcctt tttttttgca aatagcttca cctatataat acttcatcca ttttattagt     480
   acatccattt agggtttagg gttaatggtt tttatagact aattttttta gtacatctat     540
   tttattctat tttagcctct aaattaagaa aactaaaact ctattttagt tttttatttt     600
   aatagtttag atataaaata gaataaaata aagtgactaa aaattaaaca aatacccttt     660
   aagaaattaa aaaaactaag gaaacatttt tcttgtttcg agtagataat gccagcctgt     720
   taaacgccgt cgacgagtct aacggacacc aaccagcgaa ccagcagcgt cgcgtcgggc     780
   caagcgaagc agacggcacg gcatctctgt cgctgcctct ggacccctct cgagagttcc     840
   gctccaccgt tggacttgct ccgctgtcgg catccagaaa ttgcgtggcg gagcggcaga     900
   cgtgagccgg cacggcaggc ggcctcctcc tcctctcacg gcaccggcag ctacggggga     960
   ttcctttccc accgctcctt cgctttccct tcctcgcccg ccgtaataaa tagacacccc    1020
   ctccacaccc tctttcccca acctcgtgtt gttcggagcg cacacacaca caaccagatc    1080
   tcccccaaat ccacccgtcg gcacctccgc ttcaaggtac gccgctcgtc ctcccccccc    1140
   ccccccctct ctaccttctc tagatcggcg ttccggtcca tggttagggc ccggtagttc    1200
   tacttctgtt catgtttgtg ttagatccgt gtttgtgtta gatccgtgct gctagcgttc    1260
   gtacacggat gcgacctgta cgtcagacac gttctgattg ctaacttgcc agtgtttctc    1320
   tttggggaat cctgggatgg ctctagccgt tccgcagacg ggatcgattt catgattttt    1380
   tttgtttcgt tgcatagggt ttggtttgcc cttttccttt atttcaatat atgccgtgca    1440
   cttgtttgtc gggtcatctt ttcatgcttt tttttgtctt ggttgtgatg atgtggtctg    1500
   gttgggcggt cgttctagat cggagtagaa ttctgtttca aactacctgg tggatttatt 1560
   aattttggat ctgtatgtgt gtgccataca tattcatagt tacgaattga agatgatgga 1620
   tggaaatatc gatctaggat aggtatacat gttgatgcgg gttttactga tgcatataca 1680
   gagatgcttt ttgttcgctt ggttgtgatg atgtggtgtg gttgggcggt cgttcattcg 1740
   ttctagatcg gagtagaata ctgtttcaaa ctacctggtg tatttattaa ttttggaact 1800
   gtatgtgtgt gtcatacatc ttcatagtta cgagtttaag atggatggaa atatcgatct 1860
   aggataggta tacatgttga tgtgggtttt actgatgcat atacatgatg gcatatgcag 1920
   catctattca tatgctctaa ccttgagtac ctatctatta taataaacaa gtatgtttta 1980
   taattatttc gatcttgata tacttggatg atggcatatg cagcagctat atgtggattt 2040
   ttttagccct gccttcatac gctatttatt tgcttggtac tgtttctttt gtcgatgctc 2100
   accctgttgt ttggtgttac ttctgcaggg taccccgggg ggatccacta gttctagaaa 2160
   ccatggccac cgccgccgcc gcgtctaccg cgctcactgg cgccactacc gctgcgccca 2220
   aggcgaggcg ccgggcgcac ctcctggcca cccgccgcgc cctcgccgcg cccatcaggt 2280
   gctcagcggc gtcacccgcc atgccgatgg ctcccccggc caccccgctc cggccgtggg 2340
   gccccaccga tccccgcaag ggcgccgaca tcctcgtcga gtccctcgag cgctgcggcg 2400
   tccgcgacgt cttcgcctac ccgggcggcg cgtccatgga gatccaccag gcactcaccc 2460
   gctcccccgt catcgccaac cacctcttcc gccacgagca aggggaggcc tttgcggcct 2520
   ccggctacgc gcgctcctcg ggccgcgtcg gcgtctgcat cgccacctcc ggccccggcg 2580
   ccaccaacct tgtctccgcg ctcgccgacg cgctgctcga ttccgtcccc atggtcgcca 2640
   tcacgggaca ggtgccgcga cgcatgattg gcaccgacgc cttccaggag acgcccatcg 2700
   tcgaggtcac ccgctccatc accaagcaca actacctggt cctcgacgtc gacgacatcc 2760
   cccgcgtcgt gcaggaggct ttcttcctcg cctcctctgg tcgaccgggg ccggtgcttg 2820
   tcgacatccc caaggacatc cagcagcaga tggcggtgcc tgtctgggac aagcccatga 2880
   gtctgcctgg gtacattgcg cgccttccca agccccctgc gactgagttg cttgagcagg 2940
   tgctgcgtct tgttggtgaa tcccggcgcc ctgttcttta tgttggcggt ggctgcgcag 3000
   catctggtga ggagttgcga cgctttgtgg agctgactgg aatcccggtc acaactactc 3060
   ttatgggcct cggcaacttc cccagcgacg acccactgtc tctgcgcatg ctaggtatgc 3120
   atggcacggt gtatgcaaat tatgcagtgg ataaggccga tctgttgctt gcacttggtg 3180
   tgcggtttga tgatcgtgtg acagggaaga ttgaggcttt tgcaagcagg gctaagattg 3240
   tgcacgttga tattgatccg gctgagattg gcaagaacaa gcagccacat gtgtccatct 3300
   gtgcagatgt taagcttgct ttgcagggca tgaatgctct tcttgaagga agcacatcaa 3360
   agaagagctt tgactttggc tcatggaacg atgagttgga tcagcagaag agggaattcc 3420
   cccttgggta taaaacatct aatgaggaga tccagccaca atatgctatt caggttcttg 3480
   atgagctgac gaaaggcgag gccatcatcg gcacaggtgt tgggcagcac cagatgtggg 3540
   cggcacagta ctacacttac aagcggccaa ggcagtggtt gtcttcagct ggtcttgggg 3600
   ctatgggatt tggtttgccg gctgctgctg gtgcttctgt ggccaaccca ggtgttactg 3660
   ttgttgacat cgatggagat ggtagctttc tcatgaacgt tcaggagcta gctatgatcc 3720
   gaattgagaa cctcccggtg aaggtctttg tgctaaacaa ccagcacctg gggatggtgg 3780
   tgcagtggga ggacaggttc tataaggcca acagagcgca cacatacttg ggaaacccag 3840
   agaatgaaag tgagatatat ccagatttcg tgacgatcgc caaagggttc aacattccag 3900
   cggtccgtgt gacaaagaag aacgaagtcc gcgcagcgat aaagaagatg ctcgagactc 3960
   cagggccgta cctcttggat ataatcgtcc cacaccagga gcatgtgttg cctatgatcc 4020
   ctaatggtgg ggctttcaag gatatgatcc tggatggtga tggcaggact gtgtactgat 4080
   ctaaaatcca gcaagcaact gatctaaaat ccagcaagca ccgcctccct gctagtacaa 4140
   gggtgatatg tttttatctg tgtgatgttc tcctgtattc tatctttttt tgtaggccgt 4200
   cagctatctg ttatggtaat cctatgtagc ttccgacctt gtaattgtgt agtctgttgt 4260
   tttccttctg gcatgtgtca taagagatca tttaagtgcc ttttgctaca tataaataag 4320
   ataataagca ctgctatgca gtggttctga attggcttct gttgccaaat ttaagtgtcc 4380
   aactggtcct tgcttttgtt ttcgctattt ttttcctttt ttagttatta ttatattggt 4440
   aatttcaact caacatatga tgtatggaat aatgctaggg ctgcaatttc aaactatttt 4500
   acaaaccaga atggcatttt cgtggtttga ggggagtgaa aaaaaatgag gcatttgact 4560
   gaattagtta cctgatccat tttcgtggtt tggatcattg gaattaaatt ccattctaat 4620
   aatagtaatt ttggcatata tcaattaagt taattcggtt ttatgcaaaa tatatttgta 4680
   tactattatt atcaagatgt cggagatatt tatatgctac atttttacta tacaggagtg 4740
   agatgaagag tgtcatgtaa gttacacagt agaaacaaat tctattaatg cataaaatca 4800
   tttccatcat ccaccctatg aatttgagat agacctatat ctaaactttg aaaagtggtt 4860
   gaatatcaaa ttccaaatta aataagttat tttattgagt gaattctaat ttctctaaaa 4920
   cgaagggatc taaacgccct ctaaagctaa tttggaaact caaactttct tagcattgga 4980
   ggggattgag aaaaaatatt aattcatttt catctcaatc attcaatctc caaagagatt 5040
   tgagttcctt attagtctgt tccatgcatc aaatcggctc aatgtgtcat tatttgccat 5100
   gacgattgac gagttgttct ggggcctagc gctttccacg ccgatgtgct ggggcctggt 5160
   cctggagaag acagcttgat atttaaagct atcaattgtt tcaattgatt cccacttcat 5220
   ttttctaaat gtagaaaacg gtgacgtata agaaaaagaa tgaattagga cttttattcc 5280
   gtacactaat ctagagcggc cgccaccgcg gtggagctcg aattccacgt gttaattaac 5340
   ggtccgaggc ctcctcagca agctgttaac gcgatcgcag cgccttaagg ggcccgttta 5400
   acaggccttt aattaccctg ttatccctaa ttaaggcgcg ccaagcttgc atgcctgcag 5460
   gaagttgaag acaaagaagg tcttaaatcc tggctagcaa cactgaacta tgccagaaac 5520
   cacatcaaag atatgggcaa gcttcttggc ccattatatc caaagacctc agagaaaggt 5580
   gagcgaaggc tcaattcaga agattggaag ctgatcaata ggatcaagac aatggtgaga 5640
   acgcttccaa atctcactat tccaccagaa gatgcataca ttatcattga aacagatgca 5700
   tgtgcaactg gatggggagc agtatgcaag tggaagaaaa acaaggcaga cccaagaaat 5760
   acagagcaaa tctgtaggta tgccagtgga aaatttgata agccaaaagg aacctgtgat 5820
   gcagaaatct atggggttat gaatggctta gaaaagatga gattgttcta cttggacaaa 5880
   agagagatca cagtcagaac tgacagtagt gcaatcgaaa ggttctacaa caagagtgct 5940
   gaacacaagc cttctgagat cagatggatc aggttcatgg actacatcac tggtgcagga 6000
   ccagagatag tcattgaaca cataaaaggg aagagcaatg gtttagctga catcttgtcc 6060
   aggctcaaag ccaaattagc tcagaatgaa ccaacggaag agatgatcct gcttacacaa 6120
   gccataaggg aagtaattcc ttatccagat catccataca ctgagcaact cagagaatgg 6180
   ggaaacaaaa ttctggatcc attccccaca ttcaagaagg acatgttcga aagaacagag 6240
   caagctttta tgctaacaga ggaaccagtt ctactctgtg catgcaggaa gcctgcaatt 6300
   cagttagtgt ccagaacatc tgccaaccca ggaaggaaat tcttcaagtg cgcaatgaac 6360
   aaatgccatt gctggtactg ggcagatctc attgaagaac acattcaaga cagaattgat 6420
   gaatttctca agaatcttga agttctgaag accggtggcg tgcaaacaat ggaggaggaa 6480
   cttatgaagg aagtcaccaa gctgaagata gaagagcagg agttcgagga ataccaggcc 6540
   acaccaaggg ctatgtcgcc agtagccgca gaagatgtgc tagatctcca agacgtaagc 6600
   aatgacgatt gaggaggcat tgacgtcagg gatgaccgca gcggagagta ctgggcccat 6660
   tcagtggatg ctccactgag ttgtattatt gtgtgctttt cggacaagtg tgctgtccac 6720
   tttcttttgg cacctgtgcc actttattcc ttgtctgcca cgatgccttt gcttagcttg 6780
   taagcaagga tcgcagtgcg tgtgtgacac cacccccctt ccgacgctct gcctatataa 6840
   ggcaccgtct gtaagctctt acgatcatcg gtagttcacc aagggggtag gtcagtccct 6900
   tatgttacgt cctgtagaaa ccccaacccg tgaaatcaaa aaactcgacg gcctgtgggc 6960
   attcagtctg gatcgcgaaa actgtggaat tggtcagcgt tggtgggaaa gcgcgttaca 7020
   agaaagccgg gcaattgctg tgccaggcag ttttaacgat cagttcgccg atgcagatat 7080
   tcgtaattat gcgggcaacg tctggtatca gcgcgaagtc tttataccga aaggttgggc 7140
   aggccagcgt atcgtgctgc gtttcgatgc ggtcactcat tacggcaaag tgtgggtcaa 7200
   taatcaggaa gtgatggagc atcagggcgg ctatacgcca tttgaagccg atgtcacgcc 7260
   gtatgttatt gccgggaaaa gtgtacgtaa gtttctgctt ctacctttga tatatatata 7320
   ataattatca ttaattagta gtaatataat atttcaaata tttttttcaa aataaaagaa 7380
   tgtagtatat agcaattgct tttctgtagt ttataagtgt gtatatttta atttataact 7440
   tttctaatat atgaccaaaa tttgttgatg tgcaggtatc accgtttgtg tgaacaacga 7500
   actgaactgg cagactatcc cgccgggaat ggtgattacc gacgaaaacg gcaagaaaaa 7560
   gcagtcttac ttccatgatt tctttaacta tgccggaatc catcgcagcg taatgctcta 7620
   caccacgccg aacacctggg tggacgatat caccgtggtg acgcatgtcg cgcaagactg 7680
   taaccacgcg tctgttgact ggcaggtggt ggccaatggt gatgtcagcg ttgaactgcg 7740
   tgatgcggat caacaggtgg ttgcaactgg acaaggcact agcgggactt tgcaagtggt 7800
   gaatccgcac ctctggcaac cgggtgaagg ttatctctat gaactgtgcg tcacagccaa 7860
   aagccagaca gagtgtgata tctacccgct tcgcgtcggc atccggtcag tggcagtgaa 7920
   gggcgaacag ttcctgatta accacaaacc gttctacttt actggctttg gtcgtcatga 7980
   agatgcggac ttgcgtggca aaggattcga taacgtgctg atggtgcacg accacgcatt 8040
   aatggactgg attggggcca actcctaccg tacctcgcat tacccttacg ctgaagagat 8100
   gctcgactgg gcagatgaac atggcatcgt ggtgattgat gaaactgctg ctgtcggctt 8160
   taacctctct ttaggcattg gtttcgaagc gggcaacaag ccgaaagaac tgtacagcga 8220
   agaggcagtc aacggggaaa ctcagcaagc gcacttacag gcgattaaag agctgatagc 8280
   gcgtgacaaa aaccacccaa gcgtggtgat gtggagtatt gccaacgaac cggatacccg 8340
   tccgcaaggt gcacgggaat atttcgcgcc actggcggaa gcaacgcgta aactcgaccc 8400
   gacgcgtccg atcacctgcg tcaatgtaat gttctgcgac gctcacaccg ataccatcag 8460
   cgatctcttt gatgtgctgt gcctgaaccg ttattacgga tggtatgtcc aaagcggcga 8520
   tttggaaacg gcagagaagg tactggaaaa agaacttctg gcctggcagg agaaactgca 8580
   tcagccgatt atcatcaccg aatacggcgt ggatacgtta gccgggctgc actcaatgta 8640
   caccgacatg tggagtgaag agtatcagtg tgcatggctg gatatgtatc accgcgtctt 8700
   tgatcgcgtc agcgccgtcg tcggtgaaca ggtatggaat ttcgccgatt ttgcgacctc 8760
   gcaaggcata ttgcgcgttg gcggtaacaa gaaagggatc ttcactcgcg accgcaaacc 8820
   gaagtcggcg gcttttctgc tgcaaaaacg ctggactggc atgaacttcg gtgaaaaacc 8880
   gcagcaggga ggcaaacaat gaatcaacaa ctctcctggc gcaccatcgt cggctacagc 8940
   ctcgggaatt gctaccgagc tcgaatttcc ccgatcgttc aaacatttgg caataaagtt 9000
   tcttaagatt gaatcctgtt gccggtcttg cgatgattat catataattt ctgttgaatt 9060
   acgttaagca tgtaataatt aacatgtaat gcatgacgtt atttatgaga tgggttttta 9120
   tgattagagt cccgcaatta tacatttaat acgcgataga aaacaaaata tagcgcgcaa 9180
   actaggataa attatcgcgc gcggtgtcat ctatgttact agatcgggaa ttggcatgca 9240
   agcttggcac tggccgtcgt tttacaacgt cgtgactggg aaaaccctgg cgttacccaa 9300
   cttaatcgcc ttgcagcaca tccccctttc gccagctggc gtaatagcga agaggcccgc 9360
   accgatcgcc cttcccaaca gttgcgcagc ctgaatggcg aatgctagag cagcttgagc 9420
   ttggatcaga ttgtcgtttc ccgccttcag tttgtgggcg cgccaagctt gcatgcctgc 9480
   aggtcgatag ggataacagg gtaatctaga ggatccccgg gtacctctaa aatccagcaa 9540
   gcaactgatc taaaatccag caagcaccgc ctccctgcta gtacaagggt gatatgtttt 9600
   tatctgtgtg atgttctcct gtattctatc tttttttgta ggccgtcagc tatctgttat 9660
   ggtaatccta tgtagcttcc gaccttgtaa ttgtgtagtc tgttgttttc cttctggcat 9720
   gtgtcataag agatcattta agtgcctttt gctacatata aataagataa taagcactgc 9780
   tatgcagtgg ttctgaattg gcttctgttg ccaaatttaa gtgtccaact ggtccttgct 9840
   tttgttttcg ctattttttt ccttttttag ttattattat attggtaatt tcaactcaac 9900
   atatgatgta tggaataatg ctagggctgc aatttcaaac tattttacaa accagaatgg 9960
   cattttcgtg gtttgagggg agtgaaaaaa aatgaggcat ttgactgaat tagttacctg 10020
   atccattttc gtggtttgga tcattggaat taaattccat tctaataata gtaattttgg 10080
   catatatcaa ttaagttaat tcggttttat gcaaaatata tttgtatact attattatca 10140
   agatgtcgga gatatttata tgctacattt ttactataca ggagtgagat gaagagtgtc 10200
   atgtaagtta cacagtagaa acaaattcta ttaatgcata aaatcatttc catcatccac 10260
   cctatgaatt tgagatagac ctatatctaa actttgaaaa gtggttgaat atcaaattcc 10320
   aaattaaata agttatttta ttgagtgaat tccacgtgcg gtccgcctca gcgcgatcgc 10380
   cttaaggttt aaactatcag tgtttgacag gatatattgg cgggtaaacc taagagaaaa 10440
   gagcgtttat tagaataatc ggatatttaa aagggcgtga aaaggtttat ccgttcgtcc 10500
   atttgtatgt gcatgccaac cacagggttc ccctcgggag tgcttggcat tccgtgcgat 10560
   aatgacttct gttcaaccac ccaaacgtcg gaaagcctga cgacggagca gcattccaaa 10620
   aagatccctt ggctcgtctg ggtcggctag aaggtcgagt gggctgctgt ggcttgatcc 10680
   ctcaacgcgg tcgcggacgt agcgcagcgc cgaaaaatcc tcgatcgcaa atccgacgct 10740
   gtcgaaaagc gtgatctgct tgtcgctctt tcggccgacg tcctggccag tcatcacgcg 10800
   ccaaagttcc gtcacaggat gatctggcgc gagttgctgg atctcgcctt caatccgggt 10860
   ctgtggcggg aactccacga aaatatccga acgcagcaag atcgtcgacc aattcttgaa 10920
   gacgaaaggg cctcgtgata cgcctatttt tataggttaa tgtcatgata ataatggttt 10980
   cttagacgtc aggtggcact tttcggggaa atgtgcgcgg aacccctatt tgtttatttt 11040
   tctaaataca ttcaaatatg tatccgctca tgagacaata accctgataa atgcttcaat 11100
   aatattgaaa aaggaagagt atgagtattc aacatttccg tgtcgccctt attccctttt 11160
   ttgcggcatt ttgccttcct gtttttgctc acccagaaac gctggtgaaa gtaaaagatg 11220
   ctgaagatca gttgggtgca cgagtgggtt acatcgaact ggatctcaac agcggtaaga 11280
   tccttgagag ttttcgcccc gaagaacgtt ttccaatgat gagcactttt aaagttctgc 11340
   tatgtggcgc ggtattatcc cgtgttgacg ccgggcaaga gcaactcggt cgccgcatac 11400
   actattctca gaatgacttg gttgagtact caccagtcac agaaaagcat cttacggatg 11460
   gcatgacagt aagagaatta tgcagtgctg ccataaccat gagtgataac actgcggcca 11520
   acttacttct gacaacgatc ggaggaccga aggagctaac cgcttttttg cacaacatgg 11580
   gggatcatgt aactcgcctt gatcgttggg aaccggagct gaatgaagcc ataccaaacg 11640
   acgagcgtga caccacgatg cctgcagggg gggggggggg gggacatgag gttgccccgt 11700
   attcagtgtc gctgatttgt attgtctgaa gttgttttta cgttaagttg atgcagatca 11760
   attaatacga tacctgcgtc ataattgatt atttgacgtg gtttgatggc ctccacgcac 11820
   gttgtgatat gtagatgata atcattatca ctttacgggt cctttccggt gatccgacag 11880
   gttacggggc ggcgacctcg cgggttttcg ctatttatga aaattttccg gtttaaggcg 11940
   tttccgttct tcttcgtcat aacttaatgt ttttatttaa aataccctct gaaaagaaag 12000
   gaaacgacag gtgctgaaag cgagcttttt ggcctctgtc gtttcctttc tctgtttttg 12060
   tccgtggaat gaacaatgga accccccccc ccccccccct gcagcaatgg caacaacgtt 12120
   gcgcaaacta ttaactggcg aactacttac tctagcttcc cggcaacaat taatagactg 12180
   gatggaggcg gataaagttg caggaccact tctgcgctcg gcccttccgg ctggctggtt 12240
   tattgctgat aaatctggag ccggtgagcg tgggtctcgc ggtatcattg cagcactggg 12300
   gccagatggt aagccctccc gtatcgtagt tatctacacg acggggagtc aggcaactat 12360
   ggatgaacga aatagacaga tcgctgagat aggtgcctca ctgattaagc attggtaact 12420
   gtcagaccaa gtttactcat atatacttta gattgattta aaacttcatt tttaatttaa 12480
   aaggatctag gtgaagatcc tttttgataa tctcatgacc aaaatccctt aacgtgagtt 12540
   ttcgttccac tgagcgtcag accccgtaga aaagatcaaa ggatcttctt gagatccttt 12600
   ttttctgcgc gtaatctgct gcttgcaaac aaaaaaacca ccgctaccag cggtggtttg 12660
   tttgccggat caagagctac caactctttt tccgaaggta actggcttca gcagagcgca 12720
   gataccaaat actgtccttc tagtgtagcc gtagttaggc caccacttca agaactctgt 12780
   agcaccgcct acatacctcg ctctgctaat cctgttacca gtggctgctg ccagtggcga 12840
   taagtcgtgt cttaccgggt tggactcaag acgatagtta ccggataagg cgcagcggtc 12900
   gggctgaacg gggggttcgt gcacacagcc cagcttggag cgaacgacct acaccgaact 12960
   gagataccta cagcgtgagc tatgagaaag cgccacgctt cccgaaggga gaaaggcgga 13020
   caggtatccg gtaagcggca gggtcggaac aggagagcgc acgagggagc ttccaggggg 13080
   aaacgcctgg tatctttata gtcctgtcgg gtttcgccac ctctgacttg agcgtcgatt 13140
   tttgtgatgc tcgtcagggg ggcggagcct atggaaaaac gccagcaacg cggccttttt 13200
   acggttcctg gccttttgct ggccttttgc tcacatgttc tttcctgcgt tatcccctga 13260
   ttctgtggat aaccgtatta ccgcctttga gtgagctgat accgctcgcc gcagccgaac 13320
   gaccgagcgc agcgagtcag tgagcgagga agcggaagag cgcctgatgc ggtattttct 13380
   ccttacgcat ctgtgcggta tttcacaccg catatggtgc actctcagta caatctgctc 13440
   tgatgccgca tagttaagcc agtatacact ccgctatcgc tacgtgactg ggtcatggct 13500
   gcgccccgac acccgccaac acccgctgac gcgccctgac gggcttgtct gctcccggca 13560
   tccgcttaca gacaagctgt gaccgtctcc gggagctgca tgtgtcagag gttttcaccg 13620
   tcatcaccga aacgcgcgag gcagcagatc ccccgatcaa gtagatacac tacatatatc 13680
   tacaatagac atcgagccgg aaggtgatgt ttactttcct gaaatcccca gcaattttag 13740
   gccagttttt acccaagact tcgcctctaa cataaattat agttaccaaa tctggcaaaa 13800
   gggttaacaa gtggcagcaa cggattcgca aacctgtcac gccttttgtg ccaaaagccg 13860
   cgccaggttt gcgatccgct gtgccaggcg ttaggcgtca tatgaagatt tcggtgatcc 13920
   ctgagcaggt ggcggaaaca ttggatgctg agaaccattt cattgttcgt gaagtgttcg 13980
   atgtgcacct atccgaccaa ggctttgaac tatctaccag aagtgtgagc ccctaccgga 14040
   aggattacat ctcggatgat gactctgatg aagactctgc ttgctatggc gcattcatcg 14100
   accaagagct tgtcgggaag attgaactca actcaacatg gaacgatcta gcctctatcg 14160
   aacacattgt tgtgtcgcac acgcaccgag gcaaaggagt cgcgcacagt ctcatcgaat 14220
   ttgcgaaaaa gtgggcacta agcagacagc tccttggcat acgattagag acacaaacga 14280
   acaatgtacc tgcctgcaat ttgtacgcaa aatgtggctt tactctcggc ggcattgacc 14340
   tgttcacgta taaaactaga cctcaagtct cgaacgaaac agcgatgtac tggtactggt 14400
   tctcgggagc acaggatgac gcctaacaat tcattcaagc cgacaccgct tcgcggcgcg 14460
   gcttaattca ggagttaaac atcatgaggg aagcggtgat cgccgaagta tcgactcaac 14520
   tatcagaggt agttggcgtc atcgagcgcc atctcgaacc gacgttgctg gccgtacatt 14580
   tgtacggctc cgcagtggat ggcggcctga agccacacag tgatattgat ttgctggtta 14640
   cggtgaccgt aaggcttgat gaaacaacgc ggcgagcttt gatcaacgac cttttggaaa 14700
   cttcggcttc ccctggagag agcgagattc tccgcgctgt agaagtcacc attgttgtgc 14760
   acgacgacat cattccgtgg cgttatccag ctaagcgcga actgcaattt ggagaatggc 14820
   agcgcaatga cattcttgca ggtatcttcg agccagccac gatcgacatt gatctggcta 14880
   tcttgctgac aaaagcaaga gaacatagcg ttgccttggt aggtccagcg gcggaggaac 14940
   tctttgatcc ggttcctgaa caggatctat ttgaggcgct aaatgaaacc ttaacgctat 15000
   ggaactcgcc gcccgactgg gctggcgatg agcgaaatgt agtgcttacg ttgtcccgca 15060
   tttggtacag cgcagtaacc ggcaaaatcg cgccgaagga tgtcgctgcc gactgggcaa 15120
   tggagcgcct gccggcccag tatcagcccg tcatacttga agctaggcag gcttatcttg 15180
   gacaagaaga tcgcttggcc tcgcgcgcag atcagttgga agaatttgtt cactacgtga 15240
   aaggcgagat caccaaggta gtcggcaaat aatgtctaac aattcgttca agccgacgcc 15300
   gcttcgcggc gcggcttaac tcaagcgtta gagagctggg gaagactatg cgcgatctgt 15360
   tgaaggtggt tctaagcctc gtacttgcga tggcatcggg gcaggcactt gctgacctgc 15420
   caattgtttt agtggatgaa gctcgtcttc cctatgacta ctccccatcc aactacgaca 15480
   tttctccaag caactacgac aactccataa gcaattacga caatagtcca tcaaattacg 15540
   acaactctga gagcaactac gataatagtt catccaatta cgacaatagt cgcaacggaa 15600
   atcgtaggct tatatatagc gcaaatgggt ctcgcacttt cgccggctac tacgtcattg 15660
   ccaacaatgg gacaacgaac ttcttttcca catctggcaa aaggatgttc tacaccccaa 15720
   aaggggggcg cggcgtctat ggcggcaaag atgggagctt ctgcggggca ttggtcgtca 15780
   taaatggcca attttcgctt gccctgacag ataacggcct gaagatcatg tatctaagca 15840
   actagcctgc tctctaataa aatgttaggc ctcaacatct agtcgcaagc tgaggggaac 15900
   cactagtgtc atacgaacct ccaagagacg gttacacaaa cgggtacatt gttgatgtca 15960
   tgtatgacaa tcgcccaagt aagtatccag ctgtgttcag aacgtacgtc cgaattaatt 16020
   catcggggta cggtcgacga tcgtcaacgt tcacttctaa agaaatagcg ccactcagct 16080
   tcctcagcgg ctttatccag cgatttccta ttatgtcggc atagttctca agatcgacag 16140
   cctgtcacgg ttaagcgaga aatgaataag aaggctgata attcggatct ctgcgaggga 16200
   gatgatattt gatcacaggc agcaacgctc tgtcatcgtt acaatcaaca tgctaccctc 16260
   cgcgagatca tccgtgtttc aaacccggca gcttagttgc cgttcttccg aatagcatcg 16320
   gtaacatgag caaagtctgc cgccttacaa cggctctccc gctgacgccg tcccggactg 16380
   atgggctgcc tgtatcgagt ggtgattttg tgccgagctg ccggtcgggg agctgttggc 16440
   tggctgg                                                           16447
<210> 5
   <211> 11457
   <212> DNA
   <213> Artificial
<220>
   <223> Composite binary vector: LB-Ubiquitin promoter/intron::Ec DsdA cds::OCS3 3'/term-[t-nos::I-SceI cds::super promoter] reverse-RB
<220>
   <221> misc_signal
   <222> (1)..(128)
   <223> T-DNA Left border
<220>
   <221> promoter
   <222> (162)..(2149)
   <223> Zea mays Ubiquitin promoter/intron
<220>
   <221> misc_signal
   <222> (2192)..(3520)
   <223> E. coli Dsda CDS
<220>
   <221> terminator
   <222> (3562)..(4270)
   <223> A. tumefaciens Octapine Synthase 3 terminator
<220>
   <221> terminator
   <222> (4419)..(4671)
   <223> A. tumefaciens Nopaline synthase terminator (reverse orientation)
<220>
   <221> misc_signal
   <222> (4695)..(5402)
   <223> I-SceI CDS (reverse orientation)
<220>
   <221> promoter
   <222> (5458)..(6569)
   <223> Super promoter (reverse orientation)
<220>
   <221> misc_signal
   <222> (6650)..(6794)
   <223> T-DNA Right border
<400> 5
   gtgattttgt gccgagctgc cggtcgggga gctgttggct ggctggtggc aggatatatt      60
   gtggtgtaaa caaattgacg cttagacaac ttaataacac attgcggacg tttttaatgt     120
   actgaattgg atccgcccgg gcggtaccaa gcttccgcgg ctgcagtgca gcgtgacccg     180
   gtcgtgcccc tctctagaga taatgagcat tgcatgtcta agttataaaa aattaccaca     240
   tatttttttt gtcacacttg tttgaagtgc agtttatcta tctttataca tatatttaaa     300
   ctttactcta cgaataatat aatctatagt actacaataa tatcagtgtt ttagagaatc     360
   atataaatga acagttagac atggtctaaa ggacaattga gtattttgac aacaggactc     420
   tacagtttta tctttttagt gtgcatgtgt tctccttttt ttttgcaaat agcttcacct     480
   atataatact tcatccattt tattagtaca tccatttagg gtttagggtt aatggttttt     540
   atagactaat ttttttagta catctatttt attctatttt agcctctaaa ttaagaaaac     600
   taaaactcta ttttagtttt tttatttaat agtttagata taaaatagaa taaaataaag     660
   tgactaaaaa ttaaacaaat accctttaag aaattaaaaa aactaaggaa acatttttct     720
   tgtttcgagt agataatgcc agcctgttaa acgccgtcga cgagtctaac ggacaccaac     780
   cagcgaacca gcagcgtcgc gtcgggccaa gcgaagcaga cggcacggca tctctgtcgc     840
   tgcctctgga cccctctcga gagttccgct ccaccgttgg acttgctccg ctgtcggcat     900
   ccagaaattg cgtggcggag cggcagacgt gagccggcac ggcaggcggc ctcctcctcc     960
   tctcacggca ccggcagcta cgggggattc ctttcccacc gctccttcgc tttcccttcc    1020
   tcgcccgccg taataaatag acacccccct cacaccctct ttccccaacc tcgtgttgtt    1080
   cggagcgcac acacacacaa ccagatctcc cccaaatcca cccgtcggca cctccgcttc    1140
   aaggtacgcc gctcgtcctc cccccccccc cccctctcta ccttctctag atcggcgttc    1200
   cggtccatgg ttagggcccg gtagttctac ttctgttcat gtttgtgtta gatccgtgtt    1260
   tgtgttagat ccgtgctgct agcgttcgta cacggatgcg acctgtacgt cagacacgtt    1320
   ctgattgcta acttgccagt gtttctcttt ggggaatcct gggatggctc tagccgttcc    1380
   gcagacggga tcgatttcat gatttttttt gtttcgttgc atagggtttg gtttgccctt    1440
   ttcctttatt tcaatatatg ccgtgcactt gtttgtcggg tcatcttttc atgctttttt    1500
   ttgtcttggt tgtgatgatg tggtctggtt gggcggtcgt tctagatcgg agtagaattc    1560
   tgtttcaaac tacctggtgg atttattaat tttggatctg tatgtgtgtg ccatacatat    1620
   tcatagttac gaattgaaga tgatggatgg aaatatcgat ctaggatagg tatacatgtt    1680
   gatgcgggtt ttactgatgc atatacagag atgctttttg ttcgcttggt tgtgatgatg    1740
   tggtgtggtt gggcggtcgt tcattcgttc tagatcggag tagaatactg tttcaaacta    1800
   cctggtgtat ttattaattt tggaactgta tgtgtgtgtc atacatcttc atagttacga 1860
   gtttaagatg gatggaaata tcgatctagg ataggtatac atgttgatgt gggttttact 1920
   gatgcatata catgatggca tatgcagcat ctattcatat gctctaacct tgagtaccta 1980
   tctattataa taaacaagta tgttttataa ttatttcgat cttgatatac ttggatgatg 2040
   gcatatgcag cagctatatg tggatttttt tagccctgcc ttcatacgct atttatttgc 2100
   ttggtactgt ttcttttgtc gatgctcacc ctgttgtttg gtgttacttc tgcagggtac 2160
   ggatcctcat ctaagcgcaa agagacgtac tatggaaaac gctaaaatga actcgctcat 2220
   cgcccagtat ccgttggtaa aggatctggt tgctcttaaa gaaaccacct ggtttaatcc 2280
   tggcacgacc tcattggctg aaggtttacc ttatgttggc ctgaccgaac aggatgttca 2340
   ggacgcccat gcgcgcttat cccgttttgc accctatctg gcaaaagcat ttcctgaaac 2400
   tgctgccact gggggattta ttgaatcaga actggttgcc attccagcta tgcaaaaacg 2460
   gctggaaaaa gaatatcagc aaccgatcag cgggcaactg ttactgaaaa aagatagcca 2520
   tttgcccatt tccggctcca taaaagcacg cggcgggatt tatgaagtcc tggcacacgc 2580
   agaaaaactg gctctggaag cggggttgct gacgcttgat gatgactaca gcaaactgct 2640
   ttctccggag tttaaacagt tctttagcca atacagcatt gctgtgggct caaccggaaa 2700
   tctggggtta tcaatcggca ttatgagcgc ccgcattggc tttaaggtga cagttcatat 2760
   gtctgctgat gcccgggcat ggaaaaaagc gaaactgcgc agccatggcg ttacggtcgt 2820
   ggaatatgag caagattatg gtgttgccgt cgaggaagga cgtaaagcag cgcagtctga 2880
   cccgaactgt ttctttattg atgacgaaaa ttcccgcacg ttgttccttg ggtattccgt 2940
   cgctggccag cgtcttaaag cgcaatttgc ccagcaaggc cgtatcgtcg atgctgataa 3000
   ccctctgttt gtctatctgc cgtgtggtgt tggcggtggt cctggtggcg tcgcattcgg 3060
   gcttaaactg gcgtttggcg atcatgttca ctgctttttt gccgaaccaa cgcactcccc 3120
   ttgtatgttg ttaggcgtcc atacaggatt acacgatcag atttctgttc aggatattgg 3180
   tatcgacaac cttaccgcag cggatggcct tgcagttggt cgcgcatcag gctttgtcgg 3240
   gcgggcaatg gagcgtctgc tggatggctt ctataccctt agcgatcaaa ccatgtatga 3300
   catgcttggc tggctggcgc aggaagaagg tattcgtctt gaaccttcgg cactggcggg 3360
   tatggccgga cctcagcgcg tgtgtgcatc agtaagttac caacagatgc acggtttcag 3420
   cgcagaacaa ctgcgtaata ccactcatct ggtgtgggcg acgggaggtg gaatggtgcc 3480
   ggaagaagag atgaatcaat atctggcaaa aggccgttaa taacgtttca acgcagcatg 3540
   gatcgtaccg agctcaatcg atcctgcttt aatgagatat gcgagacgcc tatgatcgca 3600
   tgatatttgc tttcaattct gttgtgcacg ttgtaaaaaa cctgagcatg tgtagctcag 3660
   atccttaccg ccggtttcgg ttcattctaa tgaatatatc acccgttact atcgtatttt 3720
   tatgaataat attctccgtt caatttactg attgtaccct actacttata tgtacaatat 3780
   taaaatgaaa acaatatatt gtgctgaata ggtttatagc gacatctatg atagagcgcc 3840
   acaataacaa acaattgcgt tttattatta caaatccaat tttaaaaaaa gcggcagaac 3900
   cggtcaaacc taaaagactg attacataaa tcttattcaa atttcaaaag tgccccaggg 3960
   gctagtatct acgacacacc gagcggcgaa ctaataacgc tcactgaagg gaactccggt 4020
   tccccgccgg cgcgcatggg tgagattcct tgaagttgag tattggccgt ccgctctacc 4080
   gaaagttacg ggcaccattc aacccggtcc agcacggcgg ccgggtaacc gacttgctgc 4140
   cccgagaatt atgcagcatt tttttggtgt atgtgggccc caaatgaagt gcaggtcaaa 4200
   ccttgacagt gacgacaaat cgttgggcgg gtccagggcg aattttgcga caacatgtcg 4260
   aggctcagca ggatgggccc aggtacagaa ttcgcggccg tacaacgcgt accggttaat 4320
   taatctagag gcgcgaaact gaaggcggga aacgacaatc agatctagta ggaaacagct 4380
   atgaccatga ttacgccaag ctgcgatcgc caattcccga tctagtaaca tagatgacac 4440
   cgcgcgcgat aatttatcct agtttgcgcg ctatattttg ttttctatcg cgtattaaat 4500
   gtataattgc gggactctaa tcataaaaac ccatctcata aataacgtca tgcattacat 4560
   gttaattatt acatgcttaa cgtaattcaa cagaaattat atgataatca tcgcaagacc 4620
   ggcaacagga ttcaatctta agaaacttta ttgccaaatg tttgaacgat cggggaaatt 4680
   cgagctcctg caggttattt caggaaagtt tcggaggaga tagtgttcgg cagtttgtac 4740
   atcatctgcg ggatcaggta cggtttgatc aggttgtaga agatcaggta agacatagaa 4800
   tcgatgtaga tgatcggttt gtttttgttg atttttacgt aacagttcag ttggaatttg 4860
   ttacgcagac ccttaaccag gtattctact tcttcgaaag tgaaagactg ggtgttcagt 4920
   acgatcgatt tgttggtaga gtttttgttg taatcccatt taccaccatc atccatgaac 4980
   cagtatgcca gagacatcgg ggtcaggtag ttttcaacca ggttgttcgg gatggttttt 5040
   ttgttgttaa cgatgaacag gctagccagt ttgttgaaag cttggtgttt gaaggtctgg 5100
   gcgccccagg tgattaccag gttacccagg tggttaacac gttctttttt gtgcggcggg 5160
   gacagtaccc actgatcgta cagcagacat acgtggtcca tgtatgcttt gtttttccac 5220
   tcgaactgca tacagtaggt tttaccttca tcacgagaac ggatgtaagc atcacccagg 5280
   atcagaccga tacctgcttc gaactgttcg atgttcagtt cgatcagctg ggatttgtat 5340
   tctttcagca gtttagagtt cggacccagg ttcattacct ggtttttttt gatgtttttc 5400
   atggcgcggg ggatcctcta gagtcgacct gcaggcatgc aagcttggcg cggatcttcg 5460
   atttggtgta tcgagattgg ttatgaaatt cagatgctag tgtaatgtat tggtaatttg 5520
   ggaagatata ataggaagca aggctattta tccatttctg aaaaggcgaa atggcgtcac 5580
   cgcgagcgtc acgcgcattc cgttcttgct gtaaagcgtt gtttggtaca cttttgacta 5640
   gcgaggcttg gcgtgtcagc gtatctattc aaaagtcgtt aatggctgcg gatcaagaaa 5700
   aagttggaat agaaacagaa tacccgcgaa attcaggccc ggttgccatg tcctacacgc 5760
   cgaaataaac gaccaaatta gtagaaaaat aaaaactgac tcggatactt acgtcacgtc 5820
   ttgcgcactg atttgaaaaa tctccctcga tcgagaaaga gatcaatgtt gagctgcttc 5880
   aaaagcaatg ggattgacca gctcgcggat cctacaggcc aaattcgctc ttagccgtac    5940
   aatattactc accggtgcga tgccccccat cgtaggtgaa ggtggaaatt aatgatccat    6000
   cttgagacca caggcccaca acagctacca gtttcctcaa gggtccacca aaaacgtaag    6060
   cgcttacgta catggtcgat aagaaaaggc aatttgtaga tgttaacatc caacgtcgct    6120
   ttcagggatc ctacaggcca aattcgctct tagccgtaca atattactca ccggtgcgat    6180
   gccccccatc gtaggtgaag gtggaaatta atgatccatc ttgagaccac aggcccacaa    6240
   cagctaccag tttcctcaag ggtccaccaa aaacgtaagc gcttacgtac atggtcgata    6300
   agaaaaggca atttgtagat gttaacatcc aacgtcgctt tcagggatcc tacaggccaa    6360
   attcgctctt agccgtacaa tattactcac cggtgcgatg ccccccatcg taggtgaagg    6420
   tggaaattaa tgatccatct tgagaccaca ggcccacaac agctaccagt ttcctcaagg    6480
   gtccaccaaa aacgtaagcg cttacgtaca tggtcgataa gaaaaggcaa tttgtagatg    6540
   ttaacatcca acgtcgcttt cagggatccg cgagcttatc gataccgtcg aatctagagg    6600
   atccgcccaa agctgcgcct tacgcgccgg gcccggccgg ccagatcttg attgtcgttt    6660
   cccgccttca gtttaaacta tcagtgtttg acaggatata ttggcgggta aacctaagag    6720
   aaaagagcgt ttattagaat aatcggatat ttaaaagggc gtgaaaaggt ttatccgttc    6780
   gtccatttgt atgtgcatgc caaccacagg gttcccctcg ggagtgcttg gcattccgtg    6840
   cgataatgac ttctgttcaa ccacccaaac gtcggaaagc ctgacgacgg agcagcattc    6900
   caaaaagatc ccttggctcg tctgggtcgg ctagaaggtc gagtgggctg ctgtggcttg    6960
   atccctcaac gcggtcgcgg acgtagcgca gcgccgaaaa atcctcgatc gcaaatccga    7020
   cgctgtcgaa aagcgtgatc tgcttgtcgc tctttcggcc gacgtcctgg ccagtcatca    7080
   cgcgccaaag ttccgtcaca ggatgatctg gcgcgagttg ctggatctcg ccttcaatcc    7140
   gggtctgtgg cgggaactcc acgaaaatat ccgaacgcag caagatcgtc gaccaattct    7200
   tgaagacgaa agggcctcgt gatacgccta tttttatagg ttaatgtcat gataataatg    7260
   gtttcttaga cgtcaggtgg cacttttcgg ggaaatgtgc gcggaacccc tatttgttta    7320
   tttttctaaa tacattcaaa tatgtatccg ctcatgagac aataaccctg ataaatgctt    7380
   caataatatt gaaaaaggaa gagtatgagt attcaacatt tccgtgtcgc ccttattccc    7440
   ttttttgcgg cattttgcct tcctgttttt gctcacccag aaacgctggt gaaagtaaaa    7500
   gatgctgaag atcagttggg tgcacgagtg ggttacatcg aactggatct caacagcggt    7560
   aagatccttg agagttttcg ccccgaagaa cgttttccaa tgatgagcac ttttaaagtt    7620
   ctgctatgtg gcgcggtatt atcccgtgtt gacgccgggc aagagcaact cggtcgccgc    7680
   atacactatt ctcagaatga cttggttgag tactcaccag tcacagaaaa gcatcttacg    7740
   gatggcatga cagtaagaga attatgcagt gctgccataa ccatgagtga taacactgcg    7800
   gccaacttac ttctgacaac gatcggagga ccgaaggagc taaccgcttt tttgcacaac    7860
   atgggggatc atgtaactcg ccttgatcgt tgggaaccgg agctgaatga agccatacca    7920
   aacgacgagc gtgacaccac gatgccgggg gggggggggg gggacatgag gttgccccgt 7980
   attcagtgtc gctgatttgt attgtctgaa gttgttttta cgttaagttg atgcagatca 8040
   attaatacga tacctgcgtc ataattgatt atttgacgtg gtttgatggc ctccacgcac 8100
   gttgtgatat gtagatgata atcattatca ctttacgggt cctttccggt gatccgacag 8160
   gttacggggc ggcgacctcg cgggttttcg ctatttatga aaattttccg gtttaaggcg 8220
   tttccgttct tcttcgtcat aacttaatgt ttttatttaa aataccctct gaaaagaaag 8280
   gaaacgacag gtgctgaaag cgagcttttt ggcctctgtc gtttcctttc tctgtttttg 8340
   tccgtggaat gaacaatgga accccccccc cccccccctg cagcaatggc aacaacgttg 8400
   cgcaaactat taactggcga actacttact ctagcttccc ggcaacaatt aatagactgg 8460
   atggaggcgg ataaagttgc aggaccactt ctgcgctcgg cccttccggc tggctggttt 8520
   attgctgata aatctggagc cggtgagcgt gggtctcgcg gtatcattgc agcactgggg 8580
   ccagatggta agccctcccg tatcgtagtt atctacacga cggggagtca ggcaactatg 8640
   gatgaacgaa atagacagat cgctgagata ggtgcctcac tgattaagca ttggtaactg 8700
   tcagaccaag tttactcata tatactttag attgatttaa aacttcattt ttaatttaaa 8760
   aggatctagg tgaagatcct ttttgataat ctcatgacca aaatccctta acgtgagttt 8820
   tcgttccact gagcgtcaga ccccgtagaa aagatcaaag gatcttcttg agatcctttt 8880
   tttctgcgcg taatctgctg cttgcaaaca aaaaaaccac cgctaccagc ggtggtttgt 8940
   ttgccggatc aagagctacc aactcttttt ccgaaggtaa ctggcttcag cagagcgcag 9000
   ataccaaata ctgtccttct agtgtagccg tagttaggcc accacttcaa gaactctgta 9060
   gcaccgccta catacctcgc tctgctaatc ctgttaccag tggctgctgc cagtggcgat 9120
   aagtcgtgtc ttaccgggtt ggactcaaga cgatagttac cggataaggc gcagcggtcg 9180
   ggctgaacgg ggggttcgtg cacacagccc agcttggagc gaacgaccta caccgaactg 9240
   agatacctac agcgtgagct atgagaaagc gccacgcttc ccgaagggag aaaggcggac 9300
   aggtatccgg taagcggcag ggtcggaaca ggagagcgca cgagggagct tccaggggga 9360
   aacgcctggt atctttatag tcctgtcggg tttcgccacc tctgacttga gcgtcgattt 9420
   ttgtgatgct cgtcaggggg gcggagccta tggaaaaacg ccagcaacgc ggccttttta 9480
   cggttcctgg ccttttgctg gccttttgct cacatgttct ttcctgcgtt atcccctgat 9540
   tctgtggata accgtattac cgcctttgag tgagctgata ccgctcgccg cagccgaacg 9600
   accgagcgca gcgagtcagt gagcgaggaa gcggaagagc gcctgatgcg gtattttctc 9660
   cttacgcatc tgtgcggtat ttcacaccgc atatggtgca ctctcagtac aatctgctct 9720
   gatgccgcat agttaagcca gtatacactc cgctatcgct acgtgactgg gtcatggctg 9780
   cgccccgaca cccgccaaca cccgctgacg cgccctgacg ggcttgtctg ctcccggcat 9840
   ccgcttacag acaagctgtg accgtctccg ggagctgcat gtgtcagagg ttttcaccgt 9900
   catcaccgaa acgcgcgagg cagcagatcc cccgatcaag tagatacact acatatatct 9960
   acaatagaca tcgagccgga aggtgatgtt tactttcctg aaatccccag caattttagg 10020
   ccagttttta cccaagactt cgcctctaac ataaattata gttaccaaat ctggcaaaag 10080
   ggttgaccgg gggggggggg aaagccacgt tgtgtctcaa aatctctgat gttacattgc 10140
   acaagataaa aatatatcat catgaacaat aaaactgtct gcttacataa acagtaatac 10200
   aaggggtgtt atgagccata ttcaacggga aacgtcttgc tcgaggccgc gattaaattc 10260
   caacatggat gctgatttat atgggtataa atgggctcgc gataatgtcg ggcaatcagg 10320
   tgcgacaatc tatcgattgt atgggaagcc cgatgcgcca gagttgtttc tgaaacatgg 10380
   caaaggtagc gttgccaatg atgttacaga tgagatggtc agactaaact ggctgacgga 10440
   atttatgcct cttccgacca tcaagcattt tatccgtact cctgatgatg catggttact 10500
   caccactgcg atccccggga aaacagcatt ccaggtatta gaagaatatc ctgattcagg 10560
   tgaaaatatt gttgatgcgc tggcagtgtt cctgcgccgg ttgcattcga ttcctgtttg 10620
   taattgtcct tttaacagcg atcgcgtatt tcgtctcgct caggcgcaat cacgaatgaa 10680
   taacggtttg gttgatgcga gtgattttga tgacgagcgt aatggctggc ctgttgaaca 10740
   agtctggaaa gaaatgcata agcttttgcc attctcaccg gattcagtcg tcactcatgg 10800
   tgatttctca cttgataacc ttatttttga cgaggggaaa ttaataggtt gtattgatgt 10860
   tggacgagtc ggaatcgcag accgatacca ggatcttgcc atcctatgga actgcctcgg 10920
   tgagttttct ccttcattac agaaacggct ttttcaaaaa tatggtattg ataatcctga 10980
   tatgaataaa ttgcagtttc atttgatgct cgatgagttt ttctaatcag aattggttaa 11040
   ttggttgtaa cactggcaga gcattacgct gacttgacgg gacggcggct ttgttgaata 11100
   aatcgaactt ttgctgagtt gaaggatcag atcacgcatc ttcccgacaa cgcagaccgt 11160
   tccgtggcaa agcaaaagtt caaaatcacc aactggtcca cctacaacaa agctctcatc 11220
   aaccgtggct ccctcacttt ctggctggat gatggggcga ttcagggatc acaggcagca 11280
   acgctctgtc atcgttacaa tcaacatgct accctccgcg agatcatccg tgtttcaaac 11340
   ccggcagctt agttgccgtt cttccgaata gcatcggtaa catgagcaaa gtctgccgcc 11400
   ttacaacggc tctcccgctg acgccgtccc ggactgatgg gctgcctgta tcgagtg 11457
<210> 6
   <211> 3164
   <212> DNA
   <213> Artificial
<220>
   <223> SuperPromoter::Ubiquitin intron::I-SceI::Nos terminator cassette that replaces the reverse orientation [Super promoter::I-SceI::Nos] cassette in pLM 319
<220>
   <221> promoter
   <222> (1)..(1112)
   <223> Super promoter
<220>
   <221> Intron
   <222> (1123)..(2167)
   <223> z mays ubiquitin intron
<220>
   <221> misc_signal
   <222> (2181)..(2888)
   <223> I-SceI CDS
<220>
   <221> terminator
   <222> (2912)..(3164)
   <223> I-SceI CDS
<400> 6
   ggatccctga aagcgacgtt ggatgttaac atctacaaat tgccttttct tatcgaccat      60
   gtacgtaagc gcttacgttt ttggtggacc cttgaggaaa ctggtagctg ttgtgggcct     120
   gtggtctcaa gatggatcat taatttccac cttcacctac gatggggggc atcgcaccgg     180
   tgagtaatat tgtacggcta agagcgaatt tggcctgtag gatccctgaa agcgacgttg     240
   gatgttaaca tctacaaatt gccttttctt atcgaccatg tacgtaagcg cttacgtttt     300
   tggtggaccc ttgaggaaac tggtagctgt tgtgggcctg tggtctcaag atggatcatt     360
   aatttccacc ttcacctacg atggggggca tcgcaccggt gagtaatatt gtacggctaa     420
   gagcgaattt ggcctgtagg atccctgaaa gcgacgttgg atgttaacat ctacaaattg     480
   ccttttctta tcgaccatgt acgtaagcgc ttacgttttt ggtggaccct tgaggaaact     540
   ggtagctgtt gtgggcctgt ggtctcaaga tggatcatta atttccacct tcacctacga     600
   tggggggcat cgcaccggtg agtaatattg tacggctaag agcgaatttg gcctgtagga     660
   tccgcgagct ggtcaatccc attgcttttg aagcagctca acattgatct ctttctcgat     720
   cgagggagat ttttcaaatc agtgcgcaag acgtgacgta agtatccgag tcagttttta     780
   tttttctact aatttggtcg tttatttcgg cgtgtaggac atggcaaccg ggcctgaatt     840
   tcgcgggtat tctgtttcta ttccaacttt ttcttgatcc gcagccatta acgacttttg     900
   aatagatacg ctgacacgcc aagcctcgct agtcaaaagt gtaccaaaca acgctttaca     960
   gcaagaacgg aatgcgcgtg acgctcgcgg tgacgccatt tcgccttttc agaaatggat    1020
   aaatagcctt gcttcctatt atatcttccc aaattaccaa tacattacac tagcatctga    1080
   atttcataac caatctcgat acaccaaatc gaagatcttc tcccccaaat ccacccgtcg    1140
   gcacctccgc ttcaaggtac gccgctcgtc ctcccccccc ccccctctct accttctcta    1200
   gatcggcgtt ccggtccatg gttagggccc ggtagttcta cttctgttca tgtttgtgtt    1260
   agatccgtgt ttgtgttaga tccgtgctgc tagcgttcgt acacggatgc gacctgtacg    1320
   tcagacacgt tctgattgct aacttgccag tgtttctctt tggggaatcc tgggatggct    1380
   ctagccgttc cgcagacggg atcgatttca tgattttttt tgtttcgttg catagggttt    1440
   ggtttgccct tttcctttat ttcaatatat gccgtgcact tgtttgtcgg gtcatctttt    1500
   catgcttttt tttgtcttgg ttgtgatgat gtggtctggt tgggcggtcg ttctagatcg    1560
   gagtagaatt ctgtttcaaa ctacctggtg gatttattaa ttttggatct gtatgtgtgt 1620
   gccatacata ttcatagtta cgaattgaag atgatggatg gaaatatcga tctaggatag 1680
   gtatacatgt tgatgcgggt tttactgatg catatacaga gatgcttttt gttcgcttgg 1740
   ttgtgatgat gtggtgtggt tgggcggtcg ttcattcgtt ctagatcgga gtagaatact 1800
   gtttcaaact acctggtgta tttattaatt ttggaactgt atgtgtgtgt catacatctt 1860
   catagttacg agtttaagat ggatggaaat atcgatctag gataggtata catgttgatg 1920
   tgggttttac tgatgcatat acatgatggc atatgcagca tctattcata tgctctaacc 1980
   ttgagtacct atctattata ataaacaagt atgttttata attattttga tcttgatata 2040
   cttggatgat ggcatatgca gcagctatat gtggattttt ttagccctgc cttcatacgc 2100
   tatttatttg cttggtactg tttcttttgt cgatgctcac cctgttgttt ggtgttactt 2160
   ctgcagcccg ggggcgcgcc atgaaaaaca tcaaaaaaaa ccaggtaatg aacctgggtc 2220
   cgaactctaa actgctgaaa gaatacaaat cccagctgat cgaactgaac atcgaacagt 2280
   tcgaagcagg tatcggtctg atcctgggtg atgcttacat ccgttctcgt gatgaaggta 2340
   aaacctactg tatgcagttc gagtggaaaa acaaagcata catggaccac gtatgtctgc 2400
   tgtacgatca gtgggtactg tccccgccgc acaaaaaaga acgtgttaac cacctgggta 2460
   acctggtaat cacctggggc gcccagacct tcaaacacca agctttcaac aaactggcta 2520
   gcctgttcat cgttaacaac aaaaaaacca tcccgaacaa cctggttgaa aactacctga 2580
   ccccgatgtc tctggcatac tggttcatgg atgatggtgg taaatgggat tacaacaaaa 2640
   actctaccaa caaatcgatc gtactgaaca cccagtcttt cactttcgaa gaagtagaat 2700
   acctggttaa gggtctgcgt aacaaattcc aactgaactg ttacgtaaaa atcaacaaaa 2760
   acaaaccgat catctacatc gattctatgt cttacctgat cttctacaac ctgatcaaac 2820
   cgtacctgat cccgcagatg atgtacaaac tgccgaacac tatctcctcc gaaactttcc 2880
   tgaaataacc tgcaggagct cgaatttccc cgatcgttca aacatttggc aataaagttt 2940
   cttaagattg aatcctgttg ccggtcttgc gatgattatc atataatttc tgttgaatta 3000
   cgttaagcat gtaataatta acatgtaatg catgacgtta tttatgagat gggtttttat 3060
   gattagagtc ccgcaattat acatttaata cgcgatagaa aacaaaatat agcgcgcaaa 3120
   ctaggataaa ttatcgcgcg cggtgtcatc tatgttacta gatc 3164
<210> 7
   <211> 32
   <212> DNA
   <213> Artificial
<220>
   <223> Fwd PCR primer for amplification of I-SceI with AscI restriction site upstream of initiating ATG
<400> 7
   aggcgcgcca tgaaaaacat caaaaaaaac ca 32
<210> 8
   <211> 29
   <212> DNA
   <213> Artificial
<220>
   <223> Rev PCR primer for amplification of I-SceI with SbfI site downstream of TAA termination codon
<400> 8
   gctcctgcag gttatttcag gaaagtttc 29
<210> 9
   <211> 22
   <212> DNA
   <213> Artificial
<220>
   <223> Oligonucleotide used with SeqID #10 to generate an I-SceI recognition site flanked by SalI and XbaI sites
<400> 9
   tcgataggga taacagggta at 22
<210> 10
   <211> 22
   <212> DNA
   <213> Artificial
<220>
   <223> oligonucleotide used with SeqID #9 to generate an I-SceI recognition site flanked by SalI and XbaI sites
<400> 10
   ctagattacc ctgttatccc ta 22
<210> 11
   <211> 18
   <212> DNA
   <213> Artificial
<220>
   <223> oligonucleotide used with seqID #12 to generate an I-SceI recognition site flanked by PacI sites
<400> 11
   tagggataac agggtaat 18
<210> 12
   <211> 18
   <212> DNA
   <213> Artificial
<220>
   <223> Oligonucleotide used with SeqID #11 to generate an I-SceI recognition site flanked by PacI sites
<400> 12
   taccctgtta tccctaat 18
<210> 13
   <211> 18
   <212> DNA
   <213> Artificial
<220>
   <223> Fwd PCR primer used for RT-PCR analysis of maize endogenous constitutively expressed gene used as internal control for transgene expression level assays
<400> 13
   tctgccttgc ccttgctt 18
<210> 14
   <211> 23
   <212> DNA
   <213> Artificial
<220>
   <223> Rev PCR primer used for RT-PCR analysis of maize endogenous constitutively expressed gene used as internal control for transgene expression level assays
<400> 14
   caattgcttg gcaggtctta ttt 23
<210> 15
   <211> 20
   <212> DNA
   <213> Artificial
<220>
   <223> Fwd PCR primer used for RT-PCR analysis of transgene expression levels via amplification of 3' UTR region encoded by NOS terminator
<400> 15
   tccccgatcg ttcaaacatt 20
<210> 16
   <211> 25
   <212> DNA
   <213> Artificial
<220>
   <223> Rev PCR primer used for RT-PCR analysis of transgene expression levels via amplification of 3' UTR region encoded by NOS terminator
<400> 16
   ccatctcata aataacgtca tgcat 25
<210> 17
   <211> 21
   <212> DNA
   <213> Artificial
<220>
   <223> Fwd PCR primer used for RT-PCR analysis of GUS expression levels
<400> 17
   ttacgtggca aaggattcga t 21
<210> 18
   <211> 20
   <212> DNA
   <213> Artificial
<220>
   <223> Rev PCR primer used for RT-PCR analysis of GUS expression levels
<400> 18
   gccccaatcc agtccattaa 20
<210> 19
   <211> 23
   <212> DNA
   <213> Artificial
<220>
   <223> Fwd primer for RT-PCR analysis of expression of I-SceI
<400> 19
   gaccaggtat gtctgctgta cga 23
<210> 20
   <211> 24
   <212> DNA
   <213> Artificial
<220>
   <223> Rev primer for RT-PCR analysis of expression of I-SceI
<400> 20
   caggtggtta acacgttctt tttt 24
<210> 21
   <211> 23
   <212> DNA
   <213> Artificial
<220>
   <223> Fwd PCR primer used for detection of homologous recombination of JB034 locus
<400> 21
   gatcgcagtg cgtgtgtgac acc 23
<210> 22
   <211> 21
   <212> DNA
   <213> Artificial
<220>
   <223> Rev PCR primer used for detection of homologous recombination of JB034 locus
<400> 22
   gtccgcatct tcatgacgac c 21
<210> 23
   <211> 21
   <212> DNA
   <213> Artificial
<220>
   <223> Fwd PCR primer used for detection of homologous recombination of JB039 locus
<400> 23
   ctaatggtgg ggctttcaag g 21
<210> 24
   <211> 23
   <212> DNA
   <213> Artificial
<220>
   <223> Rev PCR primer used for detection of homologous recombination of JB039 locus. when used with SeqID #23, amplification of a recombined locus should yeild a 0.9Kb product, while amplification of a native locus should amplify a 6.7Kb p
<400> 24
   ccttaaggcg atcgcgctga ggc 23
<210> 25
   <211> 20
   <212> DNA
   <213> Artificial
<220>
   <223> Rev PCR primer used for confirming the presence of the JB039 locus. when used with SeqID #23, amplification of an unrecombined locus should yeild a 1.2Kb product, while the recombined locus should fail to produce a PCR product in this
<400> 25
   agtgtacgga ataaaagtcc 20
<210> 26
   <211> 708
   <212> DNA
   <213> Artificial
<220>
   <223> I-SceI gene codon optimized for expression in maize and soybean using the Leto program from Entelechon GmbH, Regensburg, Germany.
<400> 26
   atgaagaaca ttaagaagaa ccaggtgatg aacctgggcc ctaactctaa gctgcttaag     60
   gaatacaagt ctcagctgat tgagctgaac attgagcagt tcgaggctgg cataggcctg    120
   attctgggcg atgcttacat taggtctagg gatgagggca agacctactg catgcagttc    180
   gagtggaaga acaaggctta catggatcac gtgtgcctgc tgtacgatca gtgggtgctg    240
   tctcctcctc acaagaagga gagggtgaac cacttgggaa acctggtgat tacctggggc    300
   gctcaaacct tcaagcacca ggctttcaac aagctggctt ctctgttcat tgtgaacaac    360
   aagaagacca ttcctaacaa cctggtggag aactacctga cccctatgtc tctggcttac    420
   tggttcatgg atgatggcgg caagtgggat tacaacaaga actctaccaa caagtctatt    480
   gtgctgaaca cccagtcttt caccttcgag gaggtggaat acctggtgaa gggcctgagg    540
   aacaagttcc agctgaactg ctacgtgaag attaacaaga acaagcctat tatttacatt    600
   gattctatgt cttacctgat tttctacaac ctgattaagc cttacctgat tcctcagatg    660
   atgtacaagc tgcctaacac catctcttct gagaccttcc tgaagtga                  708
<210> 27
   <211> 923
   <212> DNA
   <213> Artificial
<220>
   <223> Optimized Coding region of the Enzyme with Attachment regions
<400> 27
   caaataatga ttttattttg actgatagtg acctgttcgt tgcaacaaat tgatgagcaa     60
   tgctttttta taatgccaac tttgtacaaa aaagcagggg cgcgccacca tgaagaacat    120
   taagaagaac caggtgatga acctgggccc taactctaag ctgcttaagg aatacaagtc    180
   tcagctgatt gagctgaaca ttgagcagtt cgaggctggc ataggcctga ttctgggcga    240
   tgcttacatt aggtctaggg atgagggcaa gacctactgc atgcagttcg agtggaagaa    300
   caaggcttac atggatcacg tgtgcctgct gtacgatcag tgggtgctgt ctcctcctca    360
   caagaaggag agggtgaacc acttgggaaa cctggtgatt acctggggcg ctcaaacctt    420
   caagcaccag gctttcaaca agctggcttc tctgttcatt gtgaacaaca agaagaccat    480
   tcctaacaac ctggtggaga actacctgac ccctatgtct ctggcttact ggttcatgga    540
   tgatggcggc aagtgggatt acaacaagaa ctctaccaac aagtctattg tgctgaacac    600
   ccagtctttc accttcgagg aggtggaata cctggtgaag ggcctgagga acaagttcca    660
   gctgaactgc tacgtgaaga ttaacaagaa caagcctatt atttacattg attctatgtc    720
   ttacctgatt ttctacaacc tgattaagcc ttacctgatt cctcagatga tgtacaagct    780
   gcctaacacc atctcttctg agaccttcct gaagtgacct gcaggccagc tttcttgtac    840
   aaagttggca ttataagaaa gcattgctta tcaatttgtt gcaacgaaca ggtcactatc    900
   agtcaaaata aaatcattat ttg                                            923
<210> 28
   <211> 30
   <212> DNA
   <213> Artificial
<220>
   <223> Recognition sequence of I-SceI
<400> 28
   agttacgcta gggataacag ggtaatatag 30
<210> 29
   <211> 18
   <212> DNA
   <213> Artificial
<220>
   <223> Core sequence of I-SceI
<400> 29
   tagggataac agggtaat 18

## Description

### SUMMARY OF THE INVENTION

The present invention relates to a method for excising a nucleic acid sequence from the genome of a plant or a plant cell. This method is based on the steps of transforming a plant cell with a construct encoding a DNA double strand break inducing enzyme (DSBI), generating a transgenic plant line, performing a transient assay to analyze the functionality of the transgenic enzyme, crossing the plant line with a line containing a nucleic acid sequence to be excised and performing an immature embryo conversion or a tissue culture regeneration through callus formation. The method can also be reversed, which means that a plant cell is transformed with a construct encoding a nucleic acid sequence to be excised, and the crossing is performed with a plant line containing a DSBI. As an alternative to the crossing step, a re-transformation of a transgenic plant line with a second construct can also be performed.

### BACKGROUND ART

An aim of plant biotechnology is the generation of plants with advantageous novel characteristics, for example for increasing agricultural productivity, improving the quality in foodstuffs or for the production of certain chemicals or pharmaceuticals (Dunwell J.M.(2000) J. Exp. Bot. 51: 487-96).

Transgenic plants can be generated by a variety of techniques (Review: Potrykus I. and Spangenberg G. ed. (1995) Gene transfer to plants. Springer, Berlin) that typically involve the introduction of separate trait and selectable marker genes. The trait gene, or gene of interest, provides the desired trait, while the selectable marker gene (such as a herbicide resistance gene) provides a means during the transformation process of selecting plants that contain the introduced DNA. The selectable marker gene typically provides no useful function once the transformed plant has been identified. The persistence of the selectable marker gene contributes substantially to the lack of acceptance of these "gene food" products among consumers. Thus, there is a demand to develop techniques by means of which marker DNA can be excised from the plant genome in a time-saving and efficient way.

In addition to improving public acceptance, removal of selectable markers can increase the ease in which multiple traits are combined into a single plant (trait stacking) by facilitating retransformation with the same selectable marker or allowing multiple traits to be crossed into a single line without resulting in multiple copies of the selectable marker.

The skilled worker is familiar with a variety of systems for the site-directed removal of recombinantly introduced nucleic acid sequences. One such system is based on the use of sequence specific recombinases (double strand break inducing enzymes, or DSBI) and two recognition sequences of said recombinase which flank the region to be removed. The effect of the DSBI on this construct brings about the excision of the flanked sequence with one of the recognition sequences remaining in the genome. Various sequence-specific recombination systems are described, such as the Cre/lox system of the bacteriophage P1 (Dale EC and Ow DW (1991) Proc Natl Acad Sci USA 88:10558-10562; Russell SH et al. (1992) Mol Gen Genet 234: 49-59; Osborne BI et al. (1995) Plant J. 7, 687-701), the yeast FLP/FRT system (Kilby NJ et al. (1995) Plant J 8:637-652; Lyznik LA et al. (1996) Nucleic Acids Res 24:3784-3789), the Mu phage Gin recombinase, the E. coli Pin recombinase or the R/RS system of the plasmid pSR1 (Onouchi H et al.(1995) Mol Gen Genet 247:653-660; Sugita K et al. (2000) Plant J. 22:461-469).

A disadvantage of the sequence-specific recombination systems is the reversibility of the reaction, that is to say an equilibrium exists between excision and integration of the marker sequence in question. This frequently brings about unwanted mutations by multiple consequtive insertions and excisions. This not only applies to the Cre-lox system, but also to the other sequence-specific recombinases (see above). A further disadvantage is the fact that one of the recognition sequences of the recombinase remains in the genome, which is thus modified: The remaining recognition sequence excludes a further use of the recombination system, for example for a second genetic modification, since interactions with the subsequently introduced recognition sequences cannot be ruled out. Substantial chromosomal rearrangements or deletions may result.

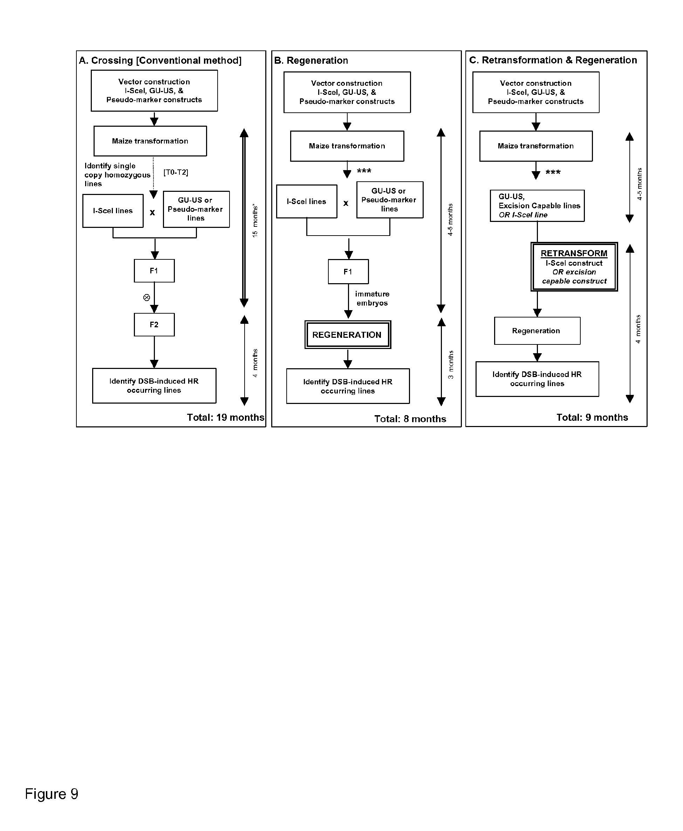

The conventional approach for identifying double strand break (DSB) induced homologous recombination (HR) in transgenic plant lines requires a minimum time of about 19 months. After the transformation of i) a plant line with a vector encoding a DSBI and ii) a plant line with a vector encoding the sequence to be excised, single copy homozygous lines are identified in the To to T₂ generations of both plant lines. Then the two lines are crossed to create an F₁ line, and it is only the F₂ line which is analyzed for DSB induced HR (see Figure 9).

WO 2006/105946 A2 discloses a method for removing a sub-sequence of a DNA molecule which sequence is flanked by two DNA sequences arranged in direct repeat and which further comprises a recognition site for a double strand break inducing enzyme. This DNA molecule is introduced into plants and the resulting plants are crossed with plants comprising a gene coding for a double strand break inducing enzyme.

Puchta (2005) J. Exp. Botany 56(409): 1-14) discusses the repair of double strand breaks in plants and biotechnological applications of this mechanism and mentions that the double strand break repair may be used for marker gene excision.

Siebert and Puchta (2002) The Plant Cell 14: 1121-1131) discloses that a negative selection marker gene such as *codA* which is flanked by I-SceI sites may be excised by I-SceI and that the homologous recombination between the cutting sites leads to the restoration of a GUS gene, when two halves of a GUS gene having an overlap are positioned next to the I-SceI sites.

### DETAILED DESCRIPTION OF THE INVENTION

The inventors demonstrate the development of a novel plant regeneration strategy that may allow for shortening the process of getting DSB-mediated repair plant lines, collecting more data than crossing *(in vitro* tissue culture materials versus conventional crossing approach), and reducing the greenhouse space and labor (see Figure 9).

In this context the inventors developed an improved method for excising a nucleic acid sequence from the genome of a plant or a plant cell. The sequence to be excised can be, for example, a marker gene, and the marker gene can be e.g. a selectable marker gene cassette for selection of transgenic plants or the entire T-DNA region in a transgenic plant for trait containment purpose. The invention is directed to data on establishment of DSB mediated repair for marker excision in plants using DSBI enzymes, especially homing endonucleases (HENs). The introduced DSB can preferably be repaired by homologous recombination (HR), nonhomologous end joining (NHEJ), precise ligation (PL), or other mechanism so that the sequence of interest is fully excised.

The invention is therefore directed to a method for excising a nucleic acid sequence from the genome of a plant or of a plant cell, comprising:
a) transforming a plant cell with a construct encoding a DNA double strand break inducing enzyme,
b) generating a transgenic plant line from the cell of step a),
c) performing a transient assay with the plant line of step b) or cells or parts thereof to analyze the functionality of the transgenic DNA double strand break inducing enzyme, wherein the transient assay is an intrachromosomal homologous recombination assay and wherein the method further comprises the identification of a single copy transgenic line following step b) or c);
d) crossing the plant line of step b) with a plant line containing a nucleic acid sequence to be excised, wherein the nucleic acid sequence to be excised comprises at least one recognition sequence which is specific for the enzyme of step a) for the site-directed induction of DNA double strand breaks, and wherein the nucleic acid sequence to be excised is bordered at both sides by a repeated sequence which allows for a DNA repair mechanism, and
e) performing a tissue culture regeneration through callus formation of F1 immature embryos, wherein the seeds and/or seedlings obtained by step e) are analyzed for DNA double strand break mediated repair mechanisms including homologous recombination and lines in which double strand break-induced homologous recombination has occurred are identified.

Preferably the DNA repair mechanism of step d) is homologous recombination.

The term "DNA double strand break inducing enzyme" (DSBI) generally refers to all those enzymes which are capable of generating double strand breaks in double stranded DNA in a sequence specific manner at one or more recognition sequences or recognition sites. The DNA break or cleavage may result in blunt ends or so called "sticky" ends of the DNA (having a 5'- or 3'-overhang). The cleavage site may be localized within or outside the recognition sequence of the enzyme. The subsequent excision of the nucleic acid sequence from the genome of a plant or plant cell is preferably realized by homologous recombination between the homologous or "repeated" sequences that should be induced by the double strand break. General methods are disclosed for example in WO 03/004659. Various enzymes suitable for the induction of double strand breaks are known in the art. The following DSBIs are mentioned by way of example, but not by limitation:
1. Restriction endonucleases (e.g. type II), preferably homing endonucleases as described in detail herein.
2. Recombinases (such as, for example, Cre/lox; R-RS; FLP/FTR).
3. Transposases, for example the P-element transposase (Kaufman P D and Rio D C (1992) Cell 69(1):27-39) or AcDs (Xiao Y L and Peterson T (2000) Mol Gen Genet 263(1):22-29). In principle, all transposases or integrases are suitable as long as they have sequence specificity (Haren L et al. (1999) Annu Rev Microbiol. 1999; 53:245-281; Beall E L, Rio D C (1997) Genes Dev. 11(16):2137-2151).
4. Chimeric nucleases as described herein.
5. Enzymes which induce double-strand breaks in the immune system, such as the RAG1/RAG2 system (Agrawal A et al. (1998) Nature 394(6695):744-451).
6. Group II endonucleases or group II intron endonucleases. Modifications of the intron sequence allows group II introns to be directed to virtually any sequence in a double-stranded DNA, where group II introns can subsequently insert by means of a reverse splice mechanism (Mohr et al. (2000) Genes & Development 14:559-573; Guo et al. (2000) Science 289:452-457). During this reverse splice mechanism, a double-strand break is introduced into the target DNA, the excised intron RNA cleaving the sense strand while the protein portion of the group II intron endonuclease hydrolyses the antisense strand (Guo et al. (1997) EMBO J 16: 6835-6848). If it is only desired to induce the double-strand break without achieving complete reverse splicing, as is preferably the case in the present invention, it is possible to resort to, for example, group II intron endonucleases which lack the reverse transcriptase activity. While this does not prevent the generation of the double-strand break, the reverse splicing mechanism cannot proceed to completion.

Preferably, the DSBI is chosen in a way that its corresponding recognition sequences are rarely, if ever, found in the unmodified genome of the target plant organism. Ideally, the only copy (or copies) of the recognition sequence in the genome is (or are) the one(s) comprised within the nucleic acid to be excised, thereby eliminating the chance that other DNA in the genome is excised or rearranged when the DSBI is expressed.

The term "expression" refers to the biosynthesis of a gene product. For example, in the case of a structural gene, expression involves transcription of the structural gene into mRNA and - optionally - the subsequent translation of mRNA into one or more polypetides.

The term "genome" or "genomic DNA" is referring to the heritable genetic information of a host organism. Said genomic DNA comprises the entire genetic material of a cell or an organism, including the DNA of the nucleus (chromosomal DNA), extrachromosomal DNA, and organellar DNA (e.g. of mitochondria and plastids like chloroplasts). Preferably the terms genome or genomic DNA is referring to the chromosomal DNA of the nucleus.

The term "chromosomal DNA" or "chromosomal DNA sequence" is to be understood as the genomic DNA of the cellular nucleus independent from the cell cycle status. Chromosomal DNA might therefore be organized in chromosomes or chromatids, they might be condensed or uncoiled. An insertion into the chromosomal DNA can be demonstrated and analyzed by various methods known in the art like e.g., polymerase chain reaction (PCR) analysis, Southern blot analysis, fluorescence in situ hybridization (FISH), and in situ PCR.

One criterion for selecting a suitable DSBI is the length of its corresponding recognition sequence. Said recognition sequence has an appropriate length to allow for rare cleavage (or DSB), more preferably cleavage only at the recognition sequence(s) comprised in the DNA construct of the invention. One factor determining the minimum length of said recognition sequence is - from a statistical point of view - the size of the genome of the host plant. In a preferred embodiment the recognition sequence has a length of at least 10 base pairs, preferably at least 14 base pairs, more preferably at least 16 base pairs, especially preferably at least 18 base pairs, most preferably at least 20 base pairs. A DSBI enzyme that cleaves a 10 base pair recognition sequence is described in Huang B. et al. (1996) J Protein Chem 15 (5): 481-9.

Suitable enzymes are not only natural enzymes, but also synthetic enzymes. Preferred enzymes are all those DSBI enzymes whose recognition sequence is known and which can either be obtained in the form of their proteins (for example by purification) or expressed using their nucleic acid sequence. Especially preferred are those enzymes which have no or only a few recognition sequences - besides the recognition sequences present in the nucleic acid to be excised - in the genomic sequence of a particular plant. This avoids further double strand breaks at undesired loci in the genome.

This is why homing endonucleases are very especially preferred (Review: Belfort M. and Roberts R.J. (1997) Nucleic Acids Res 25: 3379-3388; Jasin M. (1996) Trends Genet. 12:224-228; Internet: http://rebase.neb.com/rebase/rebase.homing.html). Owing to their long recognition sequences, they have no, or only a few, further recognition sequences in the genomic DNA of eukaryotic organisms in most cases.

The sequences encoding for homing endonucleases can be isolated for example from the chloroplast genome of Chlamydomonas (Turmel M et al. (1993) J Mol Biol 232: 446-467). They are small (usually 18 to 26 kD) and their open reading frame (ORF) has a "codon usage" which is suitable directly for nuclear expression in eukaryotes (Monnat R.J. Jr et al. (1999) Biochem Biophys Res Com 255:88-93). Homing endonucleases which are especially preferably isolated are the homing endonucleases I-SceI (WO96/14408), I-SceII (Sarguiel B et al. (1990) Nucleic Acids Res 18:5659-5665), I-SceIII (Sarguiel B et al. (1991) Mol Gen Genet. 255:340-341), I-CeuI (Marshall (1991) Gene 104:241-245), 1-CreI (Wang J et al. (1997) Nucleic Acids Res 25: 3767-3776), I-ChuI (Cote V et al.(1993) Gen 129:69-76), I-TevI (Chu et al. (1990) Proc Natl Acad Sci USA 87:3574-3578; Bell-Pedersen et al. (1990) Nucleic Acids Res18:3763-3770), I-TevII (Bell-Pedersen et al. (1990) Nucleic Acids Res18:3763-3770), I-TevIII (Eddy et al. (1991) Genes Dev. 5:1032-1041), Endo SceI (Kawasaki et al. (1991) J Biol Chem 266:5342-5347), I-CpaI (Turmel M et al. (1995a) Nucleic Acids Res 23:2519-2525) and I-CpaII (Turmel M et al. (1995b) Mol. Biol. Evol. 12, 533-545).

Further examples which may be mentioned are homing endonucleases such as F-SceI, F-SceII, F-SuvI, F-TevI, F-TevII, I-AmaI, I-AniI, I-CeuI, I-CeuAIIP, I-ChuI, I-CmoeI, I-CpaI, I-CpaII, I-CreI, I-CrepsbIP, I-CrepsbIIP, I-CrepsbIIIP, I-CrepsbIVP, I-CsmI, I-CvuI, I-CvuAIP, I-DdiI, I-DdiII, I-DirI, I-DmoI, I-HmuI, I-HmuII, I-HspNIP, I-LlaI, I-MsoI, I-NaaI, I-NanI, I-NclIP, I-NgrIP, I-NitI, I-NjaI, I-Nsp236IP, I-PakI, I-PboIP, I-PcuIP, I-PcuAI, I-PcuVI, I-PgrIP, I-PobIP, I-PorI, I-PorIIP, I-PpbIP, I-PpoI, I-SPBetaIP, I-ScaI, I-SceI, I-SceII, I-SceIII, I-SceIV, I-SceV, I-SceVI, I-SceVII, I-SexIP, I-SneIP, I-SpomCP, I-SpomIP, I-SpomIIP, I-SquIP, I-Ssp6803I, I-SthPhiJP, I-SthPhiST3P, I-SthPhiS3bP, I-TdeIP, I-TevI, I-TevII, I-TevIII, I-UarAP, I-UarHGPA1P, I-UarHGPA13P, I-VinIP, I-ZbiIP, PI-MtuI, PI-MtuHIP, PI-MtuHIP, PI-PfuI, PI-PfuII, PI-PkoI, PI-PkoII, PI-PspI, PI-Rma43812IP, PI-SPBetaIP, PI-SceI, PI-TfuI, PI-TfuII, PI-ThyI, PI-TliI, PI-TliII, and combinations thereof.

The enzymes can be isolated from their organisms of origin in the manner with which the skilled worker is familiar, and/or their coding nucleic acid sequence can be cloned. The sequences of various enzymes are deposited in GenBank.

Other suitable DSBI enzymes that may be mentioned by way of example are chimeric nucleases which are composed of an unspecific catalytic nuclease domain and a sequence specific DNA binding domaine consisting of zinc fingers (Bibikova M et al. (2001) Mol Cell Biol. 21:289-297). These DNA-binding zinc finger domains can be adapted to suit any DNA sequence. Suitable methods for preparing suitable zinc finger domains are described and known to the skilled worker (Beerli R.R. et al., Proc. Natl. Acad. Sci. USA. 2000; 97 (4):1495-1500; Beerli R.R. et al., J. Biol. Chem 2000; 275(42):32617-32627; Segal D.J. and Barbas C.F. 3rd., Curr. Opin. Chem. Biol. 2000; 4(1):34-39; Kang J S and Kim J S, J Biol Chem 2000; 275(12):8742-8748; Beerli R R et al., Proc Natl Acad Sci USA 1998; 95(25):14628-14633; Kim J S et al., Proc Natl Acad Sci USA 1997; 94(8):3616-3620; Klug A, J Mol Biol 1999; 293(2):215-218; Tsai S Y et al., Adv Drug Deliv Rev 1998; 30(1-3):23-31; Mapp A K et al., Proc Natl Acad Sci USA 2000; 97(8):3930-3935; Sharrocks A D et al., Int J Biochem Cell Biol 1997; 29(12):1371-1387; Zhang L et al., J Biol Chem 2000; 275(43):33850-33860).

Molecular evolution can be employed to create an improved DSBI. Polynucleotides encoding a candidate DSBI enzyme can, for example, be modulated with DNA shuffling protocols. DNA shuffling is a process of recursive recombination and mutation, performed by random fragmentation of a pool of related genes, followed by reassembly of the fragments by a polymerase chain reaction-like process. See, e.g., Stemmer (1994) Proc Natl Acad Sci USA 91: 10747-10751; Stemmer (1994) Nature 370: 389-391; and US 5,605,793, US 5,837,458, US 5,830,721 and US 5,811,238. An alternative to DNA shuffling for the modification of DSBI is rational design. Rational design involves the directed mutation of a gene based on an existing understanding of DNA and/or protein interactions so that the outcome of the mutation is anticipated.

The DSBI enzyme can also be expressed as a fusion protein with a nuclear localization sequence (NLS). This NLS sequence enables facilitated transport into the nucleus and increases the efficacy of the recombination system. A variety of NLS sequences are known to the skilled worker and described, inter alia, by Jicks G R and Raikhel N V (1995) Annu. Rev. Cell Biol. 11:155-188. Preferred for plant organisms is, for example, the NLS sequence of the SV40 large antigen. Owing to the small size of many DSBI enzymes (such as, for example, the homing endonucleases), an NLS sequence is however not necessarily required. These enzymes can be capable of passing through the nuclear pores without the need for transport processes mediated by an NLS.

For the present invention, the DNA double strand break inducing enzyme is preferably selected from the group consisting of homing endonucleases, restriction endonucleases, group II endonucleases, recombinases, transposases and chimeric endonucleases.

More preferably, the DNA double strand break inducing enzyme is selected from the group consisting of I-SceI, F-SceI, F-SceII, F-SuvI, F-TevI, F-TevII, I-AmaI, I-AniI, I-CeuI, I-CeuAIIP, I-ChuI, I-CmoeI, I-CpaI, I-CpaII, I-CreI, I-CrepsbIP, I-CrepsbIIP, I-CrepsbIIIP, I-CrepsbIVP, I-CsmI, I-CvuI, I-CvuAIP,I-DdiI, I-DdiII, I-DirI, I-DmoI, I-HmuI, I-HmuII, I-HspNIP,I-LlaI, I-MsoI, I-NaaI, I-NanI, I-NclIP, I-NgrIP, I-NitI, I-NjaI, I-Nsp236IP, I-PakI, I-PboIP, I-PcuIP, I-PcuAI, I-PcuVI,I-PgrIP, I-PobIP, I-PorI, I-PorIIP, I-PpbIP,I-PpoI, I-SPBetaIP, I-ScaI, I-SceII, I-SceIII, I-SceIV, I-SceV, I-SceVI, I-SceVII, I-SexIP, I-SneIP, I-SpomCP, I-SpomIP, I-SpomIIP, I-SquIP, I-Ssp6803I, I-SthPhiJP, I-SthPhiST3P, I-SthPhiS3bP, I-TdeIP, I-TevI, RTI I-TevII, I-TevIII, I-UarAP, I-UarHGPAIP, I-UarHGPA13P, I-VinIP, I-ZbiIP, PI-MtuI, PI-MtuHIP, PI-MtuHIIP, PI-PfuI, PI-PfuII, PI-PkoI, PI-PkoII, PI-PspI, PI-Rma43812IP, PI-SPBetaIP, PI-SceI, PI-TfuI, PI-TfuII, PI-ThyI, PI-TliI and PI-TliII.

Most preferably the DNA double strand break inducing enzyme is selected from the group consisting of enzymes having a nucleotide sequence as depicted in SEQ ID NOs: 26 or 27 or a substantial homologue thereof.

As used herein, the term "amino acid sequence" refers to a list of abbreviations, letters, characters or words representing amino acid residues. Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

The terms "polypeptide", "peptide", "oligopeptide", "polypeptide", " gene product", "expression product" and "protein" are used interchangeably herein to refer to a polymer or oligomer of consecutive amino acid residues.

The term "nucleic acid" refers to deoxyribonucleotides or ribonucleotides and polymers or hybrids thereof in either single- or double-stranded, sense or antisense form. Unless otherwise indicated, a particular nucleic acid sequence also implicitly encompasses conservatively modified variants thereof (e.g., degenerate codon substitutions) and complementary sequences, as well as the sequence explicitly indicated. The term "nucleic acid" can represent for example a gene, a cDNA, an mRNA, an oligonucleotide and a polynucleotide.

The phrase "nucleic acid sequence" refers to a single or double-stranded polymer of deoxyribonucleotide or ribonucleotide bases usually read from the 5'- to the 3'-end. It can have any length from only a few nucleotides to many kilo bases and includes chromosomal DNA, self-replicating plasmids, infectious polymers of DNA or RNA and DNA or RNA that performs a primarily structural role.

A "coding region" is the portion of the nucleic acid that is transcribed to an mRNA and directs the translation of the specified protein sequence. Eventually the mRNA is translated in a sequence-specific manner to produce into a particular polypeptide or protein. The coding region is said to encode such a polypeptide or protein. The coding region is bounded, in eukaryotes, on the 5'-side by the nucleotide triplet "ATG" which encodes the initiator methionine and on the 3'-side by one of the three triplets that specify stop codons (i.e., TAA, TAG, TGA). In addition to containing introns, genomic forms of a gene may also include sequences located on both the 5'-and 3'-end of the sequences that are present on the RNA transcript. These sequences are referred to as untranslated regions or UTRs; these UTRs are located 5' or 3' to the coding region of the mRNA. The 5'-UTR may contain regulatory sequences such as enhancers that can control or influence the transcription of the gene. The 3'-flanking region may contain sequences that can provide information relevant for mRNA processing, stability, and/or expression, as well as direct the termination of transcription and subsequent functions involved in proper mRNA processing, including posttranscriptional cleavage and polyadenylation.

The term "gene" refers to a coding region operably joined to appropriate regulatory sequences capable of regulating the expression of the polypeptide in some manner. A gene includes untranscribed and/or untranslated regulatory regions of DNA (e.g. , promoters, enhancers, repressors, etc.) preceding (upstream) and following (downstream) the coding region (open reading frame, ORF) as well as, where applicable, intervening sequences (i.e., introns) between individual coding regions (i.e., exons). The term "structural gene" as used herein is intended to mean a DNA sequence that is transcribed into mRNA which is then translated into a sequence of amino acids characteristic of a specific polypetide.

A (polynucleotide) "construct" refers to a nucleic acid at least partly created by recombinant methods. The term "DNA construct" is referring to a polynucleotide construct consisting of deoxyribonucleotides. The construct may be single-stranded or preferably double-stranded. The construct may be circular or linear. The skilled worker is familiar with a variety of ways to obtain and generate a DNA construct.

Constructs can be prepared by means of customary recombination and cloning techniques as are described, for example, in T. Maniatis, E. F. Fritsch and J. Sambrook, Molecular Cloning: A Laboratory Manual, Cold Spring Harbor Laboratory, Cold Spring Harbor, NY (1989), in T. J.Silhavy, M. L. Berman and L. W. Enquist, Experiments with Gene Fusions, Cold Spring Harbor Laboratory, Cold Spring Harbor, NY (1984) andin Ausubel, F. M. etal., Current Protocols in Molecular. Biology, Greene Publishing Assoc. and Wiley Interscience (1987).

When used in reference to a double-stranded nucleic acid sequence such as a cDNA or genomic clone, the term "substantially homologous" or "substantial homologue" refers to any probe which can hybridize to either or both strands of the double-stranded nucleic acid sequence under conditions of low stringency as described infra. When used in reference to a single stranded nucleic acid sequence, the term "substantially homologous" refers to any probe which can hybridize to the single-stranded nucleic acid sequence under conditions of low stringency as described infra.

The term "hybridization" as used herein includes any process by which a strand of nucleic acid joins with a complementary strand through base pairing. (Coombs 1994). Hybridization and the strength of hybridization (i.e., the strength of the association between the nucleic acids) is impacted by such factors as the degree of complementarity between the nucleic acids, stringency of the conditions involved, the Tm of the formed hybrid, and the G: C ratio within the nucleic acids.

As used herein, the term "Tm" is used in reference to the "melting temperature". The melting temperature is the temperature at which a population of double-stranded nucleic acid molecules becomes half dissociated into single strands. The equation for calculating the Tm of nucleic acids is well known in the art. As indicated by standard references, a simple estimate of the Tm value may be calculated by the equation: Tm=81.5+0. 41 (% G+C), when a nucleic acid is in aqueous solution at 1 M NaCI [see, e.g., Anderson and Young, Quantitative Filter Hybridization, in Nucleic Acid Hybridization (1985)]. Other references include more sophisticated computations which take structural as well as sequence characteristics into account for the calculation of Tm.

Low stringency conditions when used in reference to nucleic acid hybridization comprise conditions equivalent to binding or hybridization at 68°C in a solution consisting of 5x SSPE (43.8 g/L NaCI, 6.9g/L NaH₂PO₄. H₂0 and 1.85 g/L EDTA, pH adjusted to 7.4 with NaOH),1 % SDS, 5x Denhardt's reagent [50x Denhardt's contains the following per 500 mL: 5 gFicoll (Type 400, Pharmacia), 5 g BSA (Fraction V; Sigma)] and 100 ug/mL denatured salmon sperm DNA followed by washing in a solution comprising 0.2x SSPE, and 0.1% SDS at room temperature when a DNA probe of about 100 to about 1000 nucleotides in length is employed.

High stringency conditions when used in reference to nucleic acid hybridization comprise conditions equivalent to binding or hybridization at 68°C in a solution consisting of 5xSSPE, 1% SDS, 5x Denhardt's reagent and 100 µg/mL denatured salmon sperm DNA followed by washing in a solution comprising 0. 1x SSPE, and 0.1 % SDS at 68°C when a probe of about 100 to about 1000 nucleotides in length is employed.

The term "equivalent" when made in reference to a hybridization condition as it relates to a hybridization condition of interest means that the hybridization condition and the hybridization condition of interest result in hybridization of nucleic acid sequences which have the same range of percent (%) homology. For example, if a hybridization condition of interest results in hybridization of a first nucleic acid sequence with other nucleic acid sequences that have from 80% to 90% homology to the first nucleic acid sequence, then another hybridization condition is said to be equivalent to the hybridization condition of interest if this other hybridization condition also results in hybridization of the first nucleic acid sequence with the other nucleic acid sequences that have from 80% to 90% homology to the first nucleic acid sequence.

When used in reference to nucleic acid hybridization the art knows well that numerous equivalent conditions may be employed to comprise either low or high stringency conditions; factors such as the length and nature (DNA, RNA, base composition) of the probe and nature of the target (DNA, RNA, base composition, present in solution or immobilized, etc.) and the concentration of the salts and other components (e.g., the presence or absence of formamide, dextran sulfate, polyethylene glycol) are considered and the hybridization solution may be varied to generate conditions of either low or high stringency hybridization different from, but equivalent to, the above-listed conditions. Those skilled in the art know that whereas higher stringencies may be preferred to reduce or eliminate non-specific binding, lower stringencies may be preferred to detect a larger number of nucleic acid sequences having different homologies.

The DSBI is encoded by a construct that may preferably be a nucleic acid construct. The DSBI enzyme is generated using an expression cassette that comprises the nucleic acid encoding a DSBI enzyme. The cassette is introduced into a plant cell or a plant. The term "expression cassette" - for example when referring to the expression cassette for the DSBI enzyme, but also with respect to any other sequence to be expressed in accordance with the present invention - means those constructs in which the "coding sequence" DNA to be expressed is linked operably to at least one genetic control element which enables or regulates its expression (i.e. transcription and/or translation). Here, expression may be for example stable or transient, constitutive or inducible. For introducing it, the skilled worker may resort to various direct methods (for example transfection, particle bombardment, microinjection) or indirect methods (for example infection with agrobacteria or viruses), all of which are detailed further below.

The following specifications about the expression cassettes, genetic control elements, promoters, enhancers etc. refer to the constructs encoding the DSBI as well as any other nucleic acid sequence which may possibly be expressed in the scope of this invention, such as the sequence which is to be excised, the marker gene sequence, the gene for resistance to antibiotics or herbicides etc.

A construct which is used for the transformation according to method step a) of the present invention may be any nucleic acid molecule which encodes a DNA double strand break inducing enzyme operably linked to at least one genetic control element. Preferably the construct encoding a DNA double strand break-inducing enzyme is selected from the group consisting of a vector, a plasmid, a cosmid, a bacterial construct or a viral construct.

A vector is a genetic construct that can be introduced into a cell. There are for example cloning vectors, expression vectors, gene fusion vectors, shuttle vectors, targeting vectors etc. If the construct is a vector, the vector is preferably selected from the group consisting of pCB series, pLM series, pJB series, pCER series, pEG series, pBR series, pUC series, M13mp series and pACYC series.

A plasmid is a circular DNA double strand molecule. It may be designed to allow the cloning and/or expression of DNA with recombinant DNA techniques. A cosmid (first described by Collins J. and Hohn B. in Proc. Natl. Acad. Sci. USA 1978 Sep;75(9):4242-6) is a vector derived from the bacterial λ virus (phage). It usually contains at least one or two cohesive ("cos") sites. The cloning capacity of a cosmid is up to about 47 kb.

The term "about" is used herein to mean approximately, roughly, around, or in the region of. When the term "about" is used in conjunction with a numerical range, it modifies that range by extending the boundaries above and below the numerical values-set forth. In general, the term "about" is used herein to modify a numerical value above and below the stated value by a variance of 20 percent up or down (higher or lower), preferably 15 percent, more preferably 10 percent and most preferably 5 percent.

"Operable linkage" is generally understood as meaning an arrangement in which a genetic control sequence is capable of exerting its function with regard to a nucleic acid sequence to be expressed, for example while encoding a DSBI enzyme. Function, in this context, may mean for example control of the expression, i.e. transcription and/or translation, of the nucleic acid sequence, for example one encoding a DSBI enzyme. Control, in this context, encompasses for example initiating, increasing, governing or suppressing the expression, i.e. transcription and, if appropriate, translation. Controlling, in turn, may be, for example, tissue- and/or time-specific. It may also be inducible, for example by certain chemicals, stress, pathogens and the like.

Operable linkage is understood as meaning for example the sequential arrangement of a promotor, of the nucleic acid sequence to be expressed - in the present case e.g. one encoding a DSBI enzyme - and, if appropriate, further regulatory elements such as, for example, a terminator, in such a way that each of the regulatory elements can fulfill its function when the nucleic acid sequence - for example one encoding a DSBI enzyme - is expressed.

This does not necessarily require a direct linkage in the chemical sense. Genetic control sequences such as, for example, enhancer sequences are also capable of exerting their function on the target sequence from positions located at a distance or indeed other DNA molecules. Preferred arrangements are those in which the nucleic acid sequence to be expressed - for example one encoding a DSBI enzyme - is positioned after a sequence acting as promotor so that the two sequences are linked covalently to one another. The distance between the promotor sequence and the nucleic acid sequence - for example one encoding a DSBI enzyme - is preferably less than 200 base pairs, especially preferably less than 100 base pairs, very especially preferably less than 50 base pairs.

The skilled worker is familiar with a variety of ways in order to obtain such an expression cassette. For example, it is preferably prepared by directly fusing a nucleic acid sequence which acts as promotor with a nucleotide sequence to be expressed - for example one encoding a DSBI enzyme. Operable linkage can be achieved by means of customary recombination and cloning techniques as are described, for example, in T. Maniatis, E. F. Fritsch and J. Sambrook, Molecular Cloning: A Laboratory Manual, Cold Spring Harbor Laboratory, Cold Spring Harbor, N.Y. (1989), in T. J. Silhavy, M. L. Berman and L. W. Enquist, Experiments with Gene Fusions, Cold Spring Harbor Laboratory, Cold Spring Harbor, N.Y. (1984) and in Ausubel, F. M. et al., Current Protocols in Molecular Biology, Greene Publishing Assoc. and Wiley Interscience (1987).

However, an expression cassette may also be constructed in such a way that the nucleic acid sequence to be expressed (for example one encoding a DSBI enzyme) is brought under the control of an endogenous genetic control element, for example a promotor, for example by means of homologous recombination or else by random insertion. Such constructs are likewise understood as being expression cassettes for the purposes of the invention.

The skilled worker furthermore knows that nucleic acid molecules may also be expressed using artificial transcription factors of the zinc finger protein type (Beerli R R et al. (2000) Proc Natl Acad Sci USA 97(4):1495-500). These factors can be adapted to suit any sequence region and enable expression independently of certain promotor sequences.

The term "genetic control sequences" is to be understood in the broad sense and refers to all those sequences that affect the coming into existence, or the function, of the expression cassette according to the invention. For example, genetic control sequences ensure transcription and, if appropriate, translation in the organism. Preferably, the expression cassettes according to the invention encompass 5'-upstream of the respective nucleic acid sequence to be expressed a promotor and 3'-downstream a terminator sequence as additional genetic control sequence, and, if appropriate, further customary regulatory elements, in each case in operable linkage with the nucleic acid sequence to be expressed. Genetic control sequences are described, for example, in "Goeddel; Gene Expression Technology: Methods in Enzymology 185, Academic Press, San Diego, Calif. (1990)" or "Gruber and Crosby, in: Methods in Plant Molecular Biology and Biotechnology, CRC Press, Boca Raton, Fla., eds.: Glick and Thompson, Chapter 7, 89-108" and the references cited therein.

Examples of such control sequences are sequences to which inductors or repressors bind and thus regulate the expression of the nucleic acid. The natural regulation of the sequences before the actual structural genes may still be present in addition to these novel control sequences or instead of these sequences and, if appropriate, may have been genetically modified in such a way that the natural regulation has been switched off and gene expression increased. However, the expression cassette may also be simpler in construction, that is to say no additional regulatory signals are inserted before the abovementioned genes, and the natural promotor together with its regulation is not removed. Instead, the natural control sequence is mutated in such a way that regulation no longer takes place and gene expression is increased. These modified promotors may also be placed on their own before the natural genes for increasing the activity.

A variety of control sequences are suitable, depending on the host organism or starting organism described in greater detail herein, which, owing to the introduction of the expression cassettes or vectors, becomes a genetically modified, or transgenic, organism.

"Transgene", "transgenic" or "recombinant" refers to a polynucleotide manipulated by man or a copy or complement of a polynucleotide manipulated by man. For instance, a transgenic expression cassette comprising a promoter operably linked to a second polynucleotide may include a promoter that is heterologous to the second polynucleotide as the result of manipulation by man (e.g., by methods described in Sambrook et al., Molecular Cloning-A Laboratory Manual, Cold Spring Harbor Laboratory, Cold Spring Harbor, New York, (1989) or Current Protocols in Molecular Biology Volumes 1- 3, John Wiley & Sons, Inc. (1994-1998)) of an isolated nucleic acid comprising the expression cassette. In another example, a recombinant expression cassette may comprise polynucleotides combined in such a way that the polynucleotides are extremely unlikely to be found in nature. For instance, restriction sites or plasmid vector sequences manipulated by man may flank or separate the promoter from the second polynucleotide. One of skill will recognize that polynucleotides can be manipulated in many ways and are not limited to the examples as described herein.

The term "transgenic" or "recombinant" when used in reference to a cell refers to a cell which contains a transgene, or whose genome has been altered by the introduction of a transgene. The term "transgenic" when used in reference to a tissue or to a plant refers to a tissue or plant, respectively, which comprises one or more cells that contain a transgene, or whose genome has been altered by the introduction of a transgene. Transgenic cells, tissues and plants may be produced by several methods including the introduction of a "transgene" comprising nucleic acid (usually DNA) into a target cell or integration of the transgene into a chromosome of a target cell by way of human intervention, such as by the methods described herein.

A preferred promotor is, in principle, any promotor that is capable of controlling the expression of genes, in particular foreign genes, in plants. Preferred promotors are those that enable constitutive expression in plants (Benfey et al. (1989) EMBO J. 8:2195-2202). A promotor that is preferably used is, in particular, a plant promotor or a promotor derived from a plant virus. Especially preferred is the promotor of the cauliflower mosaic virus 35S transcript (Franck et al. (1980) Cell 21:285-294; Odell et al. (1985) Nature 313:810-812; Shewmaker et al. (1985) Virology 140:281-288; Gardner et al. 1986, Plant Mol. Biol. 6, 221-228) or the 19S CaMV promotor (U.S. Pat. No. 5,352,605 and WO 84/02913). It is known that this promotor comprises a variety of recognition sequences for transcriptional effectors that, in their totality, bring about permanent and constitutive expression of the gene introduced (Benfey et al. (1989) EMBO J 8:2195-2202). A further suitable constitutive promotor is the Rubisco small subunit (SSU) promotor (U.S. Pat. No. 4,962,028). A further example of a suitable promotor is the leguminB promotor (GenBank Acc.-No.: X03677). Further preferred constitutive promotors are, for example, the Agrobacterium nopaline synthase promotor, the TR dual promotor, the Agrobacterium OCS (octopine synthase) promotor, the ubiquitin promotor (Holtorf S et al. (1995) Plant Mol Biol 29:637-649), the promoters of the vacuolar ATPase subunits, or the promotor of a wheat proline-rich protein (WO 91/13991).

The expression cassettes may also comprise an inducible, preferably a chemically inducible, promotor (Aoyama T and Chua N H (1997) Plant J 11:605-612; Caddick M X et al. (1998) Nat. Biotechnol 16:177-180; Review: Gatz, Annu Rev Plant Physiol Plant Mol Biol 1997, 48:89-108), by means of which the expression of the exogenous gene in the plant can be controlled at a specific point in time. Such promotors, such as, for example, the PRP1 promotor (Ward et al., Plant. Mol. Biol. 22 (1993), 361-366), a salicylic acid inducible promotor (WO 95/19443), a benzenesulfonamide inducible promotor (EP-A-0388186), a tetracycline inducible promotor (Gatz et al., (1992) Plant J. 2, 397-404), an abscisic acid inducible promotor (EP-A 335528), a salicylic acid inducible promotor (WO 95/19443) or an ethanol-(Salter M G et al. (1998) Plant J. 16:127-132) or cyclohexanone inducible (WO 93/21334) promotor may likewise be used.

In an especially preferred embodiment, nucleic acid encoding the DSBI enzyme, in particular, is expressed under the control of an inducible promotor. This leads to a controlled, governable expression and deletion - for example in plants -, and any potential deleterious effects caused by a constitutive expression of a DSBI enzyme are avoided.

Other preferred promotors are promoters induced by biotic or abiotic stress, such as, for example, the pathogen-inducible promotor of the PRP1 gene (Ward et al., Plant Mol Biol 1993, 22: 361-366), the tomato heat-inducible hsp80 promotor (U.S. Pat. No. 5,187,267), the potato chill-inducible alpha-amylase promotor (WO 96/12814) or the wound-induced pinII promotor (EP375091). Other preferred promoters are promoters with specificity for the anthers, ovaries, pollen, the meristem, flowers, leaves, stems, roots and seeds. A development-regulated promotor is, inter alia, described by Baerson et al. (Baerson S R, Lamppa G K (1993) Plant Mol Biol 22(2):255-67).

Especially preferred promoters are those that ensure expression in tissues or plant parts in which the biosynthesis of starch and/or oils or their precursors takes place or in which the products are advantageously accumulated. The cellular locations for starch biosynthesis are the chloroplasts of the leaves or the amyloplasts of the storage organs such as seeds, fruits or tubers. Within these organs, it is predominantly the cells of the endosperm or the cotyledons of the embryo in which synthesis takes place. Preferred promotors are thus in addition to the above-mentioned constitutive promotors in particular seed-specific promotors such as, for example, the phaseolin promotor (U.S. Pat. No. 5,504,200, Bustos M M et al., Plant Cell. 1989; 1(9):839-53), the promotor of the 2S albumin gene (Joseffson L G et al. (1987) J Biol Chem 262: 12196-12201), the legumin promotor (Shirsat A et al. (1989) Mol Gen Genet. 215(2):326-331), the USP (unknown seed protein) promotor (Baeumlein H et al. (1991) Molecular & General Genetics 225(3):459-67), the napin gene promotor (U.S. Pat. No. 5,608,152; Stalberg K, et al. (1996) L. Planta 199: 515-519), the sucrose binding protein promotor (WO 00/26388) or the legumin B4 promotor (LeB4; Bumlein H et al. (1991) Mol Gen Genet 225:121-128; Baeumlein et al. (1992) Plant Journal 2(2):233-239; Fiedler U et al. (1995) Biotechnology (NY) 13(10):1090-1093), the Ins Arabidopsis oleosin promotor (WO9845461), the Brassica Bce4 promotor (WO 91/13980). Further suitable seed-specific promoters are those of the genes encoding the "high-molecular-weight glutenin" (HMWG), gliadin, branching enzyme, ADP-glucose pyrophosphatase (AGPase) or starch synthase. Furthermore preferred promoters are those which enable seed-specific expression in monocots such as maize, barley, wheat, rye, rice and the like. Promotors that may advantageously be employed are the promotor of the lpt2 or lpt1 gene (WO 95/15389, WO 95/23230) or the promotors described in WO 99/16890 (promoters of the hordein gene, the glutelin gene, the oryzin gene, the prolamine gene, the gliadin gene, the glutelin gene, the zein gene, the kasirin gene or the secalin gene).

Promotors which are preferred as genetic control elements are, furthermore, pollen specific promoters such as, for example, the promotor of the B. campestris bgp1 gene (GenBank Acc.-No: X68210; Xu H et al. (1993) Mol Gen Genet 239(1-2):58-65; WO 94/13809), of the Oryza sativa ory s1 gene (GenBank Acc.-No.: AJ012760; Xu H et al. (1995) Gene 164 (2):255-259), of the pollen-specific maize gene ZM13 (Hamilton D A et al. (1998) Plant Mol Biol 38(4):663-669; U.S. Pat. No. 5,086,169), of the B.napus gene Bp10 (GenBank Acc.-No.: X64257; Albani D (1992) Plant J 2(3):331-342; U.S. Pat. No. 6,013,859), and functional combinations of such promoters. Other preferred promoters are the Lcg1 promotor for cell-specific expression in the male gametes (WO 99/05281; XU H et al. (1999) Proc. Natl. Acad. Sci. USA Vol. 96:2554-2558) and the promotor of the AtDMC1 gene (Klimyuk V I et al.(1997) Plant J. 11(1):1-14). Further suitable promotors are, for example, specific promotors for tubers, storage roots or roots such as, for example, the class I patatin promotor (B33), the potato cathepsin D inhibitor promotor, the starch synthase (GBSS1) promotor or the sporamin promotor, and fruit-specific promoters such as, for example, the tomato fruit-specific promotor (EP-A 409625).

Promotors that are furthermore suitable are those which ensure leaf specific expression. Promotors which may be mentioned are the potato cytosolic FBPase promotor (WO 98/18940), the Rubisco (ribulose-1,5-bisphosphate carboxylase) SSU (small subunit) promoter, the potato ST-LSI promotor (Stockhaus et al. (1989) EMBO J 8(9):2445-2451) or functional combinations of such promoters. Other preferred promotors are those that govern expression in seeds and plant embryos. Further suitable promoters are, for example, fruit-maturation-specific promotors such as, for example, the tomato fruit-maturation-specific promotor (WO 94/21794), flower-specific promotors such as, for example, the phytoene synthase promotor (WO 92/16635) or the promotor of the P-rr gene (WO 98/22593) or another node-specific promotor as described in EP-A 249676 may be used advantageously.

In principle, all natural promotors together with their regulatory sequences, such as those mentioned above, may be used for the method according to the invention. In addition, synthetic promotors may also be used advantageously. Genetic control sequences also encompass further promoters, promotor elements or minimal promotors capable of modifying the expression-specific characteristics. Thus, for example, the tissue-specific expression may take place in addition as a function of certain stress factors, owing to genetic control sequences. Such elements are, for example, described for water stress, abscisic acid (Lam E and Chua N H (1991) J Biol Chem 266(26):17131-17135) and heat stress (Schoffl F et al. (1989) Molecular & General Genetics 217(2-3):246-53). Furthermore, other promotors that enable expression in further plant tissues or other organisms, such as, for example, E.coli bacteria, may be linked operably with the nucleic acid sequence to be expressed. Plant promotors that are suitable are, in principle, all of the above-described promotors.

Preferably the promoter of the present invention is selected from the group consisting of constitutive promoters, development-dependent promoters, plant virus derived promoters, inducible promoters, chemically inducible promoters, biotic or abiotic stress inducible promoters, pathogen inducible promoters, tissue specific promoters, promoters with specificity for the embryo, scutellum, endosperm, embryo axis, anthers, ovaries, pollen, meristem, flowers, leaves, stems, roots, seeds, fruits and/or tubers, promoters which enable seed specific expression in monocotyledons including maize, barley, wheat, rye and rice, super promoters, and functional combinations of such promoters.

More preferably the promoter is selected from the group consisting of a ubiquitin promoter, sugarcane bacilliform virus promoter, phaseolin promoter, 35S CaMV promoter, 19S CaMV promoter, short or long USB promoter, Rubisco small subunit promoter, legumin B promoter, nopaline synthase promoter, TR dual promoter, octopine synthase promoter, vacuolar ATPase subunit promoter, proline-rich protein promoter, PRP1 promoter, benzenesulfonamide-inducible promoter, tetracycline-inducible promoter, abscisic acid-inducible promoter, salicylic acid-inducible promoter, ethanol inducible promoter, cyclohexanone inducible promoter, heat-inducible hsp80 promoter, chill-inducible alpha-amylase promoter, wound-induced pinII promoter, 2S albumin promoter, legumin promoter, unknown seed protein promoter, napin promoter, sucrose binding protein promoter, legumin B4 promoter, oleosin promoter, Bce4 promoter, high-molecular-weight glutenin promoter, gliadin promoter, branching enzyme promoter, ADP-glucose pyrophosphatase promoter, synthase promoter, bgpl promoter, lpt2 or lpt1 promoter, hordein promoter, glutelin promoter, oryzin promoter, prolamine promoter, gliadin promoter, glutelin promoter, zein promoter, kasirin promoter, secalin promoter, ory s1 promoter, ZM 13 promoter, Bp10 promoter, Lcg1 promoter, AtDMC1 promoter, class I patatin promoter, B33 promoter, cathepsin D inhibitor promoter, starch synthase promoter, GBSS1 promoter, sporamin promoter, tomato fruit-specific promoter, cytosolic FBPase promoter, ST-LSI promoter, CP12 promoter, CcoMT1 promoter, HRGP promoter, super promoter, promoters in combination with an intron-mediated enhancement (IME) conferring intron (preferably located between the promoter and the "structural" gene, i.e. the sequence to be expressed), and functional combinations of such promoters.

Most preferably the promoter comprises a nucleic acid sequence as depicted in nucleotides 1 to 1112 of SEQ ID NO: 6.

Genetic control sequences furthermore also encompass the 5'-untranslated region, introns or the noncoding 3'-region of genes. It has been demonstrated that they may play a significant role in the regulation of gene expression. Thus, it has been demonstrated that 5'-untranslated sequences are capable of enhancing the transient expression of heterologous genes. Furthermore, they may promote tissue specificity (Rouster J et al., Plant J. 1998, 15: 435-440.). Conversely, the 5'-untranslated region of the opaque-2 gene suppresses expression. Deletion of the region in question leads to an increased gene activity (Lohmer S et al., Plant Cell 1993, 5:65-73).

Genetic control sequences may also encompass ribosome-binding sequences for initiating translation. This is preferred in particular when the nucleic acid sequence to be expressed does not provide suitable sequences or when they are not compatible with the expression system. Genetic control sequences are furthermore understood as also encompassing sequences that create fusion proteins comprising a signal peptide sequence directing subcellular localization of a protein.

The expression cassette can advantageously comprise one or more of what are known as enhancer sequences in operable linkage with the promotor, which enable the increased transgenic expression of the nucleic acid sequence. Additional advantageous sequences, such as further regulatory elements or terminators, may also be inserted at the 3'-end of the nucleic acid sequences to be expressed recombinantly. One or more copies of the nucleic acid sequences to be expressed recombinantly may be present in the gene construct.

Polyadenylation signals which are suitable as genetic control sequences are plant polyadenylation signals, preferably those which correspond essentially to T-DNA polyadenylation signals from Agrobacterium tumefaciens, in particular of gene 3 of the T-DNA (octopine synthase) of the Ti plasmids pTiACHS (Gielen et al., EMBO J. 3 (1984), 835 et sec.) or functional equivalents thereof. Examples of particularly suitable terminator sequences are the OCS (octopine synthase) terminator and the NOS (nopaline synthase) terminator.

The term "transformation" or "transforming" as used herein refers to the introduction of a nucleic acid molecule (e.g. a transgene) into a plant cell. Preferably the transformation method is selected from the group consisting of *Agrobacterium* mediated transformation, biolistic transformation (gene gun), protoplast transformation, polyethylene glycol transformation, electroporation, sonication, microinjection, macroinjection, vacuum filtration, infection, incubation of dried embryos in DNA-containing solution, osmotic shock, silica/carbon fibers, laser mediated transformation, meristem transformation (floral dip, vaccum infiltration), and pollen transformation.

Methods for transforming plant cells / plants and for regenerating plants from plant tissues or plant cells with which the skilled worker is familiar are exploited for transient or stable transformation. Suitable direct methods of DNA delivery are especially those for either protoplast transformation or for the intact cells and tissues by means of polyethylene-glycol-induced DNA uptake, biolistic methods such as the gene gun ("particle bombardment" method), electroporation, the incubation of dry embryos in DNA-containing solution, sonication and microinjection, the micro- or macroinjection into tissues or embryos, tissue electroporation, incubation of dry embryos in DNA-containing solution or vacuum infiltration of seeds. In the case of injection or electroporation of DNA into plant cells, the plasmid used need not meet any particular requirement. Simple plasmids such as those of the pUC series may be used with or without linearization. If intact plants are to be regenerated from the transformed cells, the presence of an additional selectable marker gene on the plasmid is useful.

Any plant tissue may act as target material. Likewise, expression may take place in callus, embryogenic tissue or somatic embryos.

In addition to these "direct" transformation techniques, transformation can also be carried out by means of Agrobacterium tumefaciens or Agrobacterium rhizogenes. These strains contain a plasmid (Ti or Ri plasmid). Part of this plasmid, termed T-DNA (transferred DNA), is transferred to the plant following agrobacterial infection and integrated into the genome of the plant cell.

The term "Agrobacterium" refers to a soil-borne, Gram-negative, rod-shaped phytopathogenic bacterium that causes crown gall. The term "Agrobacterium" includes, but is not limited to, the strains Agrobacterium tumefaciens, (which typically causes crown gall in infected plants), and Agrobacterium rhizogenes (which causes hairy root disease in infected host plants). Infection of a plant cell with Agrobacterium generally results in the production of opines (e.g., opaline, agropine, octopine etc.) by the infected cell.

The terms "infecting" and "infection" with a bacterium refer to co-incubation of a target biological sample, (e.g., cell, tissue, etc.) with the bacterium under conditions such that nucleic acid sequences contained within the bacterium are introduced into one or more cells of the target biological sample.

In general, transformation of a cell may be stable or transient. The term "transient transformation" or "transiently transformed" refers to the introduction of one or more transgenes into a cell in the absence of integration of the transgene into the host cell's genome. Transient transformation may be detected by, for example, enzyme linked immunosorbent assay (ELISA), which detects the presence of a polypetide encoded by one or more of the transgenes. Alternatively, transient transformation may be detected by assessing the activity of the protein encoded by the transgene as demonstrated herein (e.g., histochemical assay of GUS enzyme activity by staining with X-glucoronidase which gives a blue precipitate in the presence of the GUS enzyme; or a chemiluminescent assay of GUS enzyme activity using the GUS-Light kit (Tropix)). The term "transient transformant" refers to a cell that has transiently contained one or more transgenes in the cell without incorporating the introduced DNA into its genome.

In contrast, the term "stable transformation" or "stably transformed" refers to the introduction and integration of one or more transgenes into the genome of a cell, preferably resulting in chromosomal integration and stable heritability through mitosis and meiosis. Stable transformation of a cell may be detected by Southern blot hybridization of genomic DNA of the cell with nucleic acid sequences that are capable of binding to one or more of the transgenes after a period of time when transgene integration into the plant genome occurs. Alternatively, stable transformation of a cell may also be detected by the polymerase chain reaction of genomic DNA of the cell to amplify transgene sequences. The term "stable transformant" refers to a cell that has stably integrated one or more transgenes into the genomic DNA. Thus, a stable transformant is distinguished from a transient transformant in that, whereas genomic DNA from the stable transformant contains one or more transgenes, genomic DNA from the transient transformant does not contain a transgene. Transformation also includes introduction of genetic material into plant cells in the form of plant viral vectors involving epichromosomal replication and gene expression that may exhibit variable properties with respect to meiotic stability.

The DNA constructs can be introduced into cells, either in culture or in the organs of a plant by a variety of conventional techniques. For example, the DNA constructs can be introduced directly to plant cells using ballistic methods such as DNA particle bombardment, or the DNA construct can be introduced using techniques such as electroporation and microinjection of a cell. Particle mediated transformation techniques (also known as "biolistics") are described in, e.g., Klein et al. (1987) Nature 327: 70-73; Vasil V et al. (1993) Bio/Technol 11: 1553-1558; and Becker D et al. (1994) Plant J 5: 299-307. These methods involve penetration of cells by small particles with the nucleic acid either within the matrix of small beads or particles, or on the surface.

The terms "bombarding", "bombardment", and "biolistic bombardment" refer to the process of accelerating particles towards a target biological sample (e.g., cell, tissue, etc.) to effect wounding of the cell membrane of a cell in the target biological sample and/or entry of the particles into the target biological sample. Methods for biolistic bombardment are known in the art (e.g., US 5,584, 807), and are commercially available (e.g., the helium gas-drivenmicroprojectile accelerator (PDS-1000/He) (BioRad).

The biolistic PDS-1000 Gene Gun (Biorad, Hercules, CA) uses helium pressure to accelerate DNA-coated gold or tungsten rnicrocarriers toward target cells. The process is applicable to a wide range of tissues and cells from organisms, including plants. The term "microwounding" when made in reference to plant tissue refers to the introduction of microscopic wounds in that tissue. Microwounding may be achieved by, for example, particle bombardment as described herein.

Microinjection techniques are known in the art and are well described in the scientific and patent literature. Also, the cell can be permeabilized chemically, for example using polyethylene glycol, so that the DNA can enter the cell by diffusion. The DNA can also be introduced by protoplast fusion with other DNA-containing units such as minicells, cells, lysosomes or liposoms. The introduction of DNA constructs using polyethylene glycol (PEG) precipitation is described in Paszkowski et al. (1984) EMBO J 3: 2717.

Liposome-based gene delivery is e.g., described in WO 93/24640; Mannino and Gould-Fogerite (1988) BioTechniques 6 (7): 682-691; US 5,279,833 ; WO 91/06309; and Felgner et al. (1987) Proc Natl Acad Sci USA 84:7413-7414).

Another suitable method of introducing DNA is electroporation, where an electrical pulse is used to reversibly permeabilize the cells. Electroporation techniques are described in Fromm et al. (1985) Proc Natl Acad Sci USA 82: 5824. PEG-mediated transformation and electroporation of plant protoplasts are also discussed in Lazzeri P (1995) Methods Mol. Biol. 49: 95-106. Preferred general methods that may be mentioned are the calcium-phosphate-mediated transfection, the DEAE-dextran-mediated transfection, the cationic lipid mediated transfection, electroporation, transduction and infection. Such methods are known to the skilled worker and described, for example, in Davis et al., Basic Methods In Molecular Biology (1986). For a review of gene transfer methods for plant and cell cultures, see, Fisk et al. (1993) Scientia Horticulturae 55: 5- 36 and Potrykus (1990) CIBA Found Symp 154: 198.

Methods are known for introduction and expression of heterologous genes in both monocot and dicot plants. See, e.g., US 5,633, 446, US 5,317, 096, US 5,689, 052, US 5,159, 135, and US 5,679, 558; Weising et al. (1988) Ann. Rev. Genet. 22: 421-477.

Transformation of monocots in particular can use various techniques including electroporation (e.g., Shimamoto et al. (1992) Nature 338: 274-276); biolistics (e.g., EP-A1270, 356); and Agrobacterium (e.g., Bytebier etal. (1987) Proc Natl Acad Sci USA . 84: 5345-5349). In particular, Agrobacterium mediated transformation is now a highly efficient transformation method in monocots (Hiei etal. (1994) Plant J 6: 271-282). A generation of fertile transgenic plants can be achieved using this approach in the cereals maize, rice, wheat, oat, and barley (reviewed in Shimamoto K (1994) Current Opinion in Biotechnology 5: 158-162; Vasil etal. (1992) Bio/Technology 10: 667-674; Vain et al. (1995) Biotechnology Advances 13(4) : 653-671 ; Vasil (1996) Nature Biotechnology 14: 702; Wan & Lemaux (1994) Plant Physio. 104: 37-48) Other methods, such as microprojectile or particle bombardment (US 5, 100, 792, EP-A- 444 882, EP-A-434 616), electroporation (EP-A 290 395, WO 87/06614), microinjection (WO 92/09696, WO 94/00583, EP-A 331 083, EP-A 175 966, Green et al. (1987) Plant Tissue and Cell Culture, Academic Press) direct DNA uptake (DE 4005152, WO 90/12096, US 4,684, 611), liposome mediated DNA uptake (e. g. Freeman et al. (1984) Plant Cell Physiol 2 9: 1353), or the vortexing method (e.g., Kindle (1990) Proc Natl Acad Sci USA 87: 1228) may be preferred where Agrobacterium transformation is inefficient or ineffective.

In particular, transformation of gymnosperms, such as conifers, may be performed using particle bombardment 20 techniques (Clapham D et al. (2000) Scan J For Res 15: 151-160). Physical methods for the transformation of plant cells are reviewed in Oard, (1991) Biotech. Adv. 9: 1-11. Alternatively, a combination of different techniques may be employed to enhance the efficiency of the transformation process, e.g. bombardment with Agrobacterium coated microparticles (EP-A-486234) or microprojectile bombardment to induce wounding followed byco-cultivation with Agrobacterium (EP-A- 486233).

The expression cassette for the DSBI enzyme is preferably integrated into specific plasmids, either into a shuttle, or intermediate, a vector or into a binary vector. If, for example, a Ti or Ri plasmid is to be used for the transformation, at least the right border, but in most cases the right and the left border, of the Ti or Ri plasmid T-DNA is linked with the expression cassette to be introduced as a flanking region. Binary vectors are preferably used. Binary vectors are capable of replication both in E. coli and in Agrobacterium. They contain a selection marker gene and a linker or polylinker flanked by the right or left T-DNA flanking sequence. They can be transformed directly into Agrobacterium (Holsters et al., Mol. Gen. Genet. 163 (1978), 181-187). The selection marker gene permits the selection of transformed agrobacteria and is, for example, the nptII gene, which imparts resistance to kanamycin. The agrobacterium, which acts as host organism in this case, should already contain a plasmid with the vir region. The latter is required for transferring the T-DNA to the plant cell. An agrobacterium thus transformed can be used for transforming plant cells.

The use of Agrobacterium tumefaciens for the transformation of plants using tissue culture explants has been described by Horsch et al. (Horsch RB (1986) Proc Natl Acad Sci USA 83(8):2571-2575), Fraley et al. (Fraley et al. 1983, Proc. Natl. Acad. Sci. USA 80, 4803-4807) and Bevans et al. (Bevans et al. 1983, Nature 304, 184-187).

Many strains of Agrobacterium tumefaciens are capable of transferring genetic material, such as, for example, the strains [pEHA101], EHA105[pEHA105], LBA4404[pAL4404], C58C1[pMP90]and C58C1[pGV2260]. The strain EHA101[pEHA101] has been described by Hood et al. (Hood E E et al. (1996) J Bacteriol 168(3):1291-1301), the strain EHA105[pEHA105] by Hood et al. (Hood et al. 1993, Transgenic Research 2, 208-218), the strain LBA4404[pAL4404] by Hoekema et al. (Hoekema et al. 1983, Nature 303, 179-181), the strain C58C1[pMP90] by Koncz and Schell (Koncz and Schell 1986, Mol. Gen. Genet. 204, 383-396), and the strain C58C1[pGV2260] by Deblaere et al. (Deblaere et al. 1985, Nucl. Acids Res. 13, 4777-4788).

For *Agrobacterium-mediated* transformation of plants, the DNA construct may be combined with suitable T-DNA flanking regions and introduced into a conventional Agrobacterium tumefaciens host vector. The virulence functions of the A. tumefaciens host will direct the insertion of a transgene and adjacent marker gene(s) (if present) into the plant cell DNA when the bacteria infect the cell. *Agrobacterium tumefaciens* mediated transformation techniques are well described in the scientific literature. See, for example, Horsch et al. (1984) Science 233: 496-498, Fraley et al. (1983) Proc Natl Acad Sci USA 80:4803-4807, Hooykaas (1989) Plant Mol Biol 13: 327-336, Horsch RB (1986) Proc Natl Acad Sci USA 83 (8):2571-2575), Bevans et al. (1983) Nature 304:184-187,Bechtold etal. (1993) Comptes Rendus De L'Academie Des Sciences Serie III-Sciences De La Vie-Life Sciences 316: 1194-1199, Valvekens et al. (1988) Proc Natl Acad Sci USA 85: 5536-5540.

The agrobacterial strain employed for the transformation comprises, in addition to its disarmed Ti plasmid, a binary plasmid with the T-DNA to be transferred, which usually comprises a gene for the selection of the transformed cells and the gene to be transferred. Both genes must be equipped with transcriptional and translational initiation and termination signals. The binary plasmid can be transferred into the agrobacterial strain for example by electroporation or other transformation methods (Mozo & Hooykaas 1991, Plant Mol. Biol. 16, 917-918). Coculture of the plant explants with the agrobacterial strain is usually performed for two to three days.

A variety of vectors could, or can, be used. In principle, one differentiates between those vectors which can be employed for the agrobacterium-mediated transformation or agroinfection, i.e. which comprise the the expression cassette, for the expression of the DSBI enzyme within a T-DNA, which indeed permits stable integration of the T-DNA into the plant genome. Moreover, border-sequence-free vectors may be employed, which can be transformed into the plant cells for example by particle bombardment, where they can lead both to transient and to stable expression.

The use of T-DNA for the transformation of plant cells has been studied and described intensively (EP 120516; Hoekema, In: The Binary Plant Vector System, Offsetdrukkerij Kanters B. V., Alblasserdam, Chapter V; Fraley et al., Crit. Rev. Plant. Sci., 4:1-46 and An et al., EMBO J. 4 (1985), 277-287). Various binary vectors are known, some of which are commercially available such as, for example, pBIN19 (Clontech Laboratories, Inc. USA).

To transfer the DNA to the plant cell, plant explants are cocultured with Agrobacterium tumefaciens or Agrobacterium rhizogenes. Starting from infected plant material (for example leaf, root or stalk sections, but also protoplasts or suspensions of plant cells), intact plants can be regenerated using a suitable medium that may contain, for example, antibiotics or biocides for selecting transformed cells. The plants obtained can then be screened for the presence of the DNA introduced, in this case the expression cassette for the DSBI enzyme. As soon as the DNA has integrated into the host genome, the genotype in question is, as a rule, stable and the insertion in question is also found in the subsequent generations. As a rule, the expression cassette integrated contains a selection marker that confers a resistance to a biocide (for example a herbicide) or an antibiotic such as kanamycin, G 418, bleomycin, hygromycin or phosphinotricin and the like to the transformed plant. The selection marker permits the selection of transformed cells (McCormick et al., Plant Cell Reports 5 (1986), 81-84). The plants obtained can be cultured and hybridized in the customary fashion. Two or more generations should be grown in order to ensure that the genomic integration is stable and hereditary.

The above-mentioned methods are described, for example, in B. Jenes et al., Techniques for Gene Transfer, in: Transgenic Plants, Vol. 1, Engineering and Utilization, edited by S. D. Kung and R. Wu, Academic Press (1993), 128-143 and in Potrykus, Annu. Rev. Plant Physiol. Plant Molec. Biol. 42 (1991), 205-225). The construct to be expressed is preferably cloned into a vector that is suitable for the transformation of Agrobacterium tumefaciens, for example pBin19 (Bevan et al., Nucl. Acids Res. 12 (1984), 8711).

Agrobacterium-mediated transformation is suited best to dicotyledonous plant cells, and has been succefully optimized for certain monocotyledonous plant cells, whereas the direct transformation techniques are suitable for any cell type.

Transformed cells, i.e. those that comprise the DNA integrated into the DNA of the host cell, can be selected from untransformed cells if a selectable marker is part of the DNA introduced. A marker can be, for example, any gene that is capable of conferring a resistance to antibiotics or herbicides. Transformed cells that express such a marker gene are capable of surviving in the presence of concentrations of a suitable antibiotic or herbicide that kill an untransformed wild type. Various positive and negative selection markers are described hereinabove. Examples are the ahas (acetohydroxy acid synthase) gene, which confers resistance to sulfonylurea and imidazolinone herbicides, the bar gene, which confers resistance to the herbicide phosphinothricin (Rathore K S et al., Plant Mol Biol. March 1993; 21(5):871-884), the nptII gene, which confers resistance to kanamycin, the hpt gene, which confers resistance to hygromycin, or the EPSP gene, which confers resistance to the herbicide Glyphosate.

As soon as a transformed plant cell has been generated, an intact plant can be obtained using methods known to the skilled worker. For example, callus cultures are used as starting material. The formation of shoot and root can be induced in this as yet undifferentiated cell biomass in the known fashion. The shoots obtained can be induced for root development under the suitable conditions. The recovered plants can then be cultured in vitro, and planted in soil.

After the transformation of a plant cell with a construct encoding a DSBI enzyme, a transgenic plant line is generated from the transformed plant cell. This generation occurs according to the methods known by the person skilled in the art. Usually, step b) of the present invention may be performed as follows.

For generating the intact transgenic plant containing the gene encoding a DSBI enzyme, the putative transgenic calli that are resistant to the chemical selection agent - either D-serine or Imazethapyr (Pursuit™ ) depending on the selection marker gene used, are cultured on the regeneration medium containing the shoot promoting phytohormone (e.g. cytokinine) and also the selection agent. Shoot formed are transferred to the rooting medium in the presence of selection agent, but in the absence of phytohormones. The integration of the transgene in the generated putative transgenic plant genome is confirmed by routine molecular techniques such as Southern blot analysis, or PCR analysis.

In the following will be described the characteristics of the nucleic acid sequence to be excised by the action of the DSBI enzyme.

The term "nucleic acid sequence to be excised" refers to any nucleotide sequence of any length, the excision or deletion of which may be deemed desirable for any reason (e.g., confer improved qualities), by one of ordinary skill in the art. Such nucleotide sequences include, but are not limited to, coding sequences of structural genes (e.g., reporter genes, selection marker genes, oncogenes, drug resistance genes, growth factors, etc.), and non-coding regulatory sequences which do not encode an mRNA or protein product, (e.g., promoter sequence, polyadenylation sequence, termination sequence, enhancer sequence, etc.).

Preferably, the length of the sequence to be excised is at least about 10 or at least about 50 base pairs, more preferably at least about 100 or at least about 500 base pairs, especially preferably at least about 1000 or at least about 5000 base or pairs, and most preferably at least about 10000 or at least about 50000 base pairs. Also, preferably, the length of the sequence to be excised is at most about 50000 or at most about 10000 base pairs, more preferably at most about 5000 or at most about 1000 base pairs, especially preferably at least about 500 or at least about 100 base pairs. Those lower and upper limits may be combined in any adequate way.

The nucleic acid to be excised may be initially part of a construct, a so-called "recombination construct", which serves e.g. for the transformation of a plant cell or a plant in order to result in a plant line containing the nucleic acid sequence to be excised (see step d) of the method according to the invention).

The nucleic acid sequence(s) to be excised (for example the T-DNA region or parts thereof, or selection markers such as genes for resistance to antibiotics or herbicides) are deleted or excised from the genome of a plant in a predictable manner. The sequence to be eliminated comprises at least one recognition sequence for the site directed induction of a DNA double strand break (for example recognition sequences of rare-cleaving restriction enzymes) and is bordered at both sides by a repeated sequence (or "homologous" sequence). A double strand break is induced by an enzyme suitable for inducing DNA double strand breaks at the recognition sequence (a DSBI enzyme), which, in consequence, triggers the homologous recombination of the homologous sequences, and thus the deletion of any nucleic acid sequence located between the homologous sequences. The recognition sequence for the site directed induction of DNA double strand breaks is likewise deleted.

The term "recognition sequence" refers to a DNA sequence that is recognized by a DSBI as described above. A recognition sequence for the site directed induction of DNA double strand breaks generally refers to those sequences that, under the conditions in the plant cell or plant used in each case, enable the recognition and cleavage by the DSBI enzyme. The recognition sequence will typically be at least 10 base pairs long, is more usually 10 to 30 base pairs long, and in most embodiments, is less than 50 base pairs long. Recognition sequences for sequence specific DSBIs (e.g., homing endonucleases) are described in the art. Recognition sequences and organisms of origin of the respective DSBI enzymes can be taken, e.g., from WO 03/004695.

Also encompassed are minor deviations (degenerations) of the recognition sequence that still enable recognition and cleavage by the DSBI enzyme in question. Such deviations - also in connection with different framework conditions such as, for example, calcium or magnesium concentration - have been described (Argast G M et al. (1998) J Mol Biol 280: 345-353). Also encompassed are core sequences of these recognition sequences. It is known that the inner portions of the recognition sequences suffice for an induced double-strand break and that the outer ones are not absolutely relevant, but can codetermine the cleavage efficacy. Thus, for example, an 18 bp core sequence can be defined for I-SceI:
Recognition sequence of I-SceI:
   5' - AGTTACGCTAGGGATAA^CAGGGTAATATAG (SEQ ID NO: 28)
   3'- TCAATGCGATCCC^TATTGTCCCATTATATC
Core sequence of I-SceI:
   5' - TAGGGATAA^CAGGGTAAT (SEQ ID NO: 29)
   3'- ATCCC^TATTGTCCCATTA

The sequences that are deleted or excised are those located between the two homology sequences (e.g. homology or repeated sequences called "A" and "B"). The skilled worker knows that he is not bound to specific sequences when performing recombination, but that any sequence can undergo homologous recombination with another sequence provided that sufficient length and homology exist.

"Homologous recombination" is a DNA recombination event occuring at and encouraged by the presence of two homologous ("repeated") DNA sites, it leads to a rearrangement or reunion of the DNA sequences by crossing over in the region of identical sequence.

Referring to the "homology" or "repeated" sequences A and B, "sufficient length" preferably refers to sequences with a length of at least 20 base pairs, preferably at least 50 base pairs, especially preferably at least 100 base pairs, very especially preferably at least 250 base pairs, most preferably at least 500 base pairs.

Referring to the homology sequences A and B, "sufficient homology" preferably refers to sequences with at least 70%, preferably 80%, by preference at least 90%, especially preferably at least 95%, very especially preferably at least 99%, most preferably 100%, homology within these homology sequences over a length of at least 20 base pairs, preferably at least 50 base pairs, especially preferably at least 100 base pairs, very especially preferably at least 250 base pairs, most preferably at least 500 base pairs.

"Homology" between two nucleic acid sequences is understood as meaning the identity of the nucleic acid sequence over in each case the entire sequence length which is calculated by alignment with the aid of the program algorithm GAP (Wisconsin Package Version 10.0, University of Wisconsin, Genetics Computer Group (GCG), Madison, USA), setting the following parameters: 1 Gap Weight: 12 Length Weight: 4 Average Match: 2,912 Average Mismatch: -2,003.

In one embodiment, only one recognition sequence for the site-directed induction of DNA double strand breaks is located between the homology sequences A and B, so that the nucleic acid sequence to be excised (or the recombination construct employed for the transformation of a target plant or plant cell for the generation of a plant line of step d) of the method according to the invention) is constructed in the 5'- to 3'-orientation as follows:
a1) a first homology sequence A,
b1) a recognition sequence for the site-directed induction of DNA double strand breaks, and
a2) a second homology sequence B, the homology sequences A and B having a sufficient length and sufficient homology in order to enable efficient homologous recombination.

In another embodiment, a further nucleic acid sequence is located between the homology sequences A and B, so that the nucleic acid sequence to be excised (or the recombination construct employed for the transformation of a target plant or plant cell for the generation of a plant line of step d) of the method according to the invention) is constructed as follows in the 5'/3'-direction of:
a1) a first homology sequence A,
b1) a recognition sequence for the site-directed induction of DNA double strand breaks,
c) a further nucleic acid sequence, and
a2) a second homology sequence B, the homology sequences A and B having a sufficient length and sufficient homology in order to enable efficient homologous recombination.

The recognition sequence for the site-directed induction of DNA double strand breaks may also be located after or within the further nucleic acid sequence.

In a further embodiment, a second recognition sequence for the site-directed induction of double strand breaks is present after the further nucleic acid sequence. This embodiment is advantageous in particular in the case of homology sequences A and B which are further apart, or in the case of longer further nucleic acid sequences, since recombination efficacy is increased. In this embodiment, the nucleic acid sequence to be excised (or the recombination construct employed for the transformation of a target plant or plant cell for the generation of a plant line of step d) of the method according to the invention) is constructed as follows in a 5'- to 3'-orientation of:
a1) a first homology sequence A,
b1) a first recognition sequence for the site-directed induction of DNA double strand breaks, and
c) a further nucleic acid sequence, and
b2) a second recognition sequence for the site-directed induction of DNA double strand breaks, and
a2) a second homology sequence B, the homology sequences A and B having a sufficient length and sufficient homology in order to enable efficient homologous recombination.

Furthermore, other recognition sequences may also be present between the homology sequences A and B, in addition to the second recognition sequences for the site-directed induction of DNA double strand breaks. The individual recognition sequences (for example b1 or b2) for the site-directed induction of DNA double strand breaks may be identical or different, i.e. they may act as recognition sequence for an individual enzyme for the site-directed induction of DNA double strand breaks or else for a variety of enzymes. The embodiment in which the recognition sequences for the site-directed induction of DNA double strand breaks act as recognition sequence for an individual enzyme for the site-directed induction of DNA double strand breaks is preferred in this context.

The skilled worker is familiar with a variety of ways to obtain a recombination construct comprising the nucleic acid sequence to be excised and to obtain a plant line containing the nucleic acid sequence to be excised. The construct can be prepared by means of customary recombination and cloning techniques as are described, for example, in T. Maniatis, E. F. Fritsch and J. Sambrook, Molecular Cloning: A Laboratory Manual, Cold Spring Harbor Laboratory, Cold Spring Harbor, N.Y. (1989), in T. J. Silhavy, M. L. Berman and L. W. Enquist, Experiments with Gene Fusions, Cold Spring Harbor Laboratory, Cold Spring Harbor, N.Y. (1984) and in Ausubel, F. M. et al., Current Protocols in Molecular Biology, Greene Publishing Assoc. and Wiley Interscience (1987). Preferably, the recombination construct is generated by joining the above-mentioned essential constituents of the recombination construct together in the above-mentioned sequence using the recombination and cloning techniques with which the skilled worker is familiar, and the result is then introduced into the genome of a host plant.

Furthermore, the skilled worker is familiar with various ways in which the recombination construct may be introduced into the genome of a plant cell or plant. In this context, the insertion may be directed (i.e. taking place at a defined insertion site) or undirected (i.e. taking place randomly). Suitable techniques are known to the skilled worker.

In addition to the elements described above with respect to the expression cassette of the DSBI enzyme (genetic control elements, promoter, enhancer etc.) the recombination construct (comprising the nucleic acid sequence to be excised) may encompass further nucleic acid sequences. Such nucleic acid sequences may preferably constitute expression cassettes. The following may be mentioned by way of example of the DNA sequences to be expressed in the expression constructs, but not by way of limitation:
i) Positive selection markers:
   Positive selection markers are genes whose presence conveys to a cell or plant the ability to persist or be identified in the presence of an otherwise harmful treatment. As pertaining to plant transformation, selection markers are required for selecting cells that have integrated and expressed any DNA of interest, e.g. the T-DNA. The selectable marker which has been introduced together with the expression construct can confer resistance to a biocide (for example a herbicide such as phosphinothricin, glyphosate or bromoxynil), a metabolism inhibitor such as 2-deoxyglucose-6-phosphate (WO 98/45456) or an antibiotic such as, for example, tetracyclines, ampicillin, kanamycin, G 418, neomycin, bleomycin or hygromycin to the cells which have successfully undergone recombination or transformation. The selection marker permits the selection of the transformed cells from untransformed cells (McCormick et al., Plant Cell Reports 5 (1986), 81-84). Especially preferred selection markers are those that confer resistance to herbicides. Examples of selection markers that may be mentioned are:
      - DNA sequences which encode phosphinothricin acetyltransferases (PAT), which acetylates the free amino group of the glutamine synthase inhibitor phosphinothricin (PPT) and thus brings about detoxification of the PPT (de Block et al. 1987, EMBO J. 6, 2513-2518) (also referred to as Bialophos.RTM. resistance gene (bar)),
      - 5-enolpyruvylshikimate-3-phosphate synthase genes (EPSP synthase genes), which confer resistance to Glyphosate.RTM. (N-(phosphonomethyl)glycine),
      - the gox gene, which encodes the Glyphosate.RTM.-degrading enzyme (Glyphosate oxidoreductase),
      - the deh gene (encoding a dehalogenase which inactivates Dalapon.RTM.),
      - the mutated acetolactate synthases which are insensitive to sulfonylurea and imidazolinone,
      - bxn genes which encode Bromoxynil.RTM.-degrading nitrilase enzymes,
      - the kanamycin, or G418, resistance gene (NPTII). The NPTII gene encodes a neomycin phosphotransferase which reduces the inhibitory effect of kanamycin, neomycin, G418 and paromomycin owing to a phosphorylation reaction,
      - the DOG.sup.R1 gene. The DOG.sup.Rl gene has been isolated from the yeast Saccharomyces cerevisiae (EP 0 807 836). It encodes a 2-deoxyglucose-6-phosphate phosphatase which confers resistance to 2-DOG (Randez-Gil et al. 1995, Yeast 11, 1233-1240).
   ii) Negative, or counter, selection markers enable the identification and/or survival of cells lacking a specified gene function, for example the selection of organisms with successfully deleted sequences which encompass the marker gene (Koprek T et al. (1999) The Plant Journal 19(6):719-726). TK thymidine kinase (TK) and diphtheria toxin A fragment (DT-A), codA gene encoding a cytosine deaminase (Gleve A P et al. (1999) Plant Mol Biol. 40(2):223-35; Pereat R I et al. (1993) Plant Mol. Biol 23(4): 793-799; Stougaard J; (1993) Plant J 3:755-761), the cytochrome P450 gene (Koprek et al. (1999) Plant J. 16:719-726), genes encoding a haloalkane dehalogenase (Naested H (1999) Plant J. 18:571-576), the iaah gene (Sundaresan V et al. (1995) Genes & Development 9:1797-1810) or the tms2 gene (Fedoroff N V & Smith D L 1993, Plant J 3: 273-289).
   iii) Reporter genes which encode readily quantifiable proteins and which may also, via intrinsic color or enzyme activity, ensure the assessment of the transformation efficacy or of the location or timing of expression. Very especially preferred here are genes encoding reporter proteins (see also Schenborn E, Groskreutz D. Mol Biotechnol. 1999; 13(1):29-44) such as:
      - "green fluorescence protein" (GFP) (Chui W L et al., Curr Biol 1996, 6:325-330; Leffel S M et al., Biotechniques. 23(5):912-8, 1997; Sheen et al.(1995) Plant Journal 8(5):777-784; Haseloff et al.(1997) Proc Natl Acad Sci USA 94(6):2122-2127; Reichel et al.(1996) Proc Natl Acad Sci USA 93(12):5888-5893; Tian et al. (1997) Plant Cell Rep 16:267-271; WO 97/41228).
      - chloramphenicoltransferase,
      - luciferase (Millar et al., Plant Mol Biol Rep 1992 10:324-414; Ow et al. (1986) Science, 234:856-859); permits the detection of bioluminescence,
      - beta-galactosidase, encodes an enzyme for which a variety of chromogenic substrates are available,
      - beta-glucuronidase (GUS) (Jefferson et al., EMBO J. 1987, 6, 3901-3907) or the uidA gene, which encodes an enzyme for a variety of chromogenic substrates,
      - R locus gene product: protein which regulates the production of anthocyanin pigments (red coloration) in plant tissue and thus makes possible the direct analysis of the promotor activity without the addition of additional adjuvants or chromogenic substrates (Dellaporta et al., In: Chromosome Structure and Function: Impact of New Concepts, 18th Stadler Genetics Symposium, 11:263-282, 1988),
      - beta-lactamase (Sutcliffe (1978) Proc Natl Acad Sci USA 75:3737-3741), enzyme for a variety of chromogenic substrates (for example PADAC, a chromogenic cephalosporin),
      - xylE gene product (Zukowsky et al. (1983) Proc Natl Acad Sci USA 80:1101-1105), catechol dioxygenase capable of converting chromogenic catechols,
      - alpha-amylase (Ikuta et al. (1990) Bio/technol. 8:241-242),
      - tyrosinase (Katz et al. (1983) J Gene Microbiol 129:2703-2714), enzyme which oxidizes tyrosine to give DOPA and dopaquinone which subsequently form melanine, which is readily detectable,
      - aequorin (Prasher et al. (1985) Biochem Biophys Res Commun 126(3):1259-1268), can be used in the calcium-sensitive bioluminescence detection.

The above-mentioned nucleic acids encoding markers and reporter genes can be comprised within the nucleic acid to be excised according to the invention. The same applies, e.g., for the T-DNA region or part thereof.

The recombination construct and any vectors derived from it may comprise further functional elements. The term "further functional elements" is to be understood in the broad sense. It preferably refers to all those elements which affect the generation, multiplication, function, use or value of the recombination system according to the invention, recombination construct or cells or organisms comprising them. The following may be mentioned by way of example, but not by limitation, of the further functional elements.
- Replication origins that ensure replication of the expression cassettes or vectors according to the invention in, for example, E. coli. Examples that may be mentioned are ORI (origin of DNA replication), the pBR322 ori or the P15A ori (Sambrook et al.: Molecular Cloning. A Laboratory Manual, 2.sup.nd ed. Cold Spring Harbor Laboratory Press, Cold Spring Harbor, N.Y., 1989).
- Multiple cloning regions (MCS) enable and facilitate the insertion of one or more nucleic acid sequences.
- Sequences which make possible homologous recombination or insertion into the genome of a host organism.
- Elements, for example border sequences, which make possible the agrobacterium-mediated transfer in plant cells for the transfer and integration into the plant genome, such as, for example, the right or left border of the T-DNA or the vir region.

All of the above-mentioned expression cassettes or further functional elements may be located, as mentioned, between the homology or repeated sequences A and B of the nucleic acid sequence to be excised. However, they may also be located outside them. This is advantageous in particular in the case of border sequences.

The method of the invention is useful for obtaining plants from which genome a nucleic acid sequence has been excised. In addition to the "whole" plants or the "mature" plants, progeny, propagation material (such as leaves, roots, seeds-including embryo, endosperm, and seed coat -, seedlings, fruit, pollen, shoots and the like), parts (organs, shoot vegetative organs/structures - e. g. leaves, stems and tubers -, roots, flowers, cuttings, and floral organs/structures, e.g. bracts, sepals, petals, stamens, carpels, anthers and ovules), tissues (e.g. vascular tissue, ground tissue, and the like), cells (e.g. guard cells, egg cells, trichomes and the like), cell cultures, and harvested material, derived from a plant which is obtained by the method according to the invention is also comprised.

"Mature plants" are to be understood as meaning plants at any developmental stage beyond the seedling. "Seedling" is to be understood as meaning a young, immature plant in an early developmental stage. The "progeny" (or descendant) includes, inter alia, a clone, a seed, a fruit, selfed or hybrid progeny and descendants, and any propagule of any of these, such as cuttings and seed, which may be used in reproduction or propagation, sexual or asexual. Also encompassed is a plant that is a sexually or asexually propagated offspring, clone or descendant of such a plant, or any part or propagule of said plant, offspring, clone or descendant.

The term "cell" or "plant cell" as used herein refers to a single cell. The term "cells" refers to a population of cells. The population may be a pure population comprising one cell type. Likewise, the population may comprise more than one cell type. In the present invention, there is no limit on the number of cell types that a cell population may comprise. The cells may be synchronized or not synchronized. A plant cell within the meaning of this disclosure may be isolated (e.g., in suspension culture) or comprised in a plant tissue, plant organ or plant at any developmental stage.

The term "tissue" with respect to a plant (or "plant tissue") means arrangement of multiple plant cells including differentiated and undifferentiated arrangements. Plant tissues may constitute part of a plant organ (e.g., the epidermis of a plant leaf) but may also constitute tumor tissues (e.g., callus tissue) and various types of cells in culture (e.g., single cells, protoplasts, embryos, calli, protocorm-like bodies, etc.). Plant tissue may be in planta, in organ culture, tissue culture, or cell culture.

Included are all genera and species of higher and lower plants of the plant kingdom. The class of plants that can be used in the method of the invention is generally as broad as the class of higher and lower plants amenable to transformation techniques, including angiosperms (monocotyledonous and dicotyledonous plants), gymnosperms, ferns, and multicellular algae. It includes plants of a variety of ploidy levels, including aneuploid, polyploid, diploid, haploid and hemizygous.

The method according to the invention may preferably be used for the following plant families: Amaranthaceae, Brassicaceae, Carophyllaceae, Chenopodiaceae, Compositae, Cucurbitaceae, Labiatae, Leguminosae-Papilionoideae, Liliaceae, Linaceae, Malvaceae, Rosaceae, Saxifragaceae, Scrophulariaceae, Solanacea, Tetragoniacea and transgene combinations thereof.

Annual, perennial, monocotyledonous and dicotyledonous plants are preferred host organisms for the generation of transgenic plants. The use of the method according to the invention is furthermore advantageous in all ornamental plants, useful or ornamental trees, flowers, cut flowers, shrubs or turf. Plants which may be mentioned by way of example but not by limitation are angiosperms, bryophytes such as, for example, Hepaticae (hepaticas) and Musci (mosses); pteridophytes such as ferns, horsetail and clubmosses; gymnosperms such as conifers, cycads, ginkgo and Gnetaeae; algae such as Chlorophyceae, Phaeophpyceae, Rhodophyceae, Myxophyceae, Xanthophyceae, Bacillariophyceae (diatoms) and Euglenophyceae. Plants for the purposes of the invention comprise by way of example and not by way of limitation the families of the Rosaceae such as rose, Ericaceae such as rhododendrons and azaleas, Euphorbiaceae such as poinsettias and croton, Caryophyllaceae such as pinks, Solanaceae such as petunias, Gesneriaceae such as African violet, Balsaminaceae such as touch-me-not, Orchidaceae such as orchids, Iridaceae such as gladioli, iris, freesia and crocus, Compositae such as marigold, Geraniaceae such as geraniums, Liliaceae such as drachaena, Moraceae such as ficus, Araceae such as philodendron and many others.

Flowering plants which may be mentioned by way of example but not by limitation are the families of the Leguminosae such as pea, alfalfa and soya; Gramineae such as rice, maize, wheat; Solanaceae such as tobacco and many others; the family of the Umbelliferae, particularly the genus Daucus (very particularly the species carota (carrot)) and Apium (very particularly the species graveolens dulce (celery)) and many others; the family of the Solanacea, particularly the genus Lycopersicon, very particularly the species esculentum (tomato) and the genus Solanum, very particularly the species tuberosum (potato) and melongena (aubergine) and many others; and the genus Capsicum, very particularly the species annum (peppers) and many others; the family of the Leguminosae, particularly the genus Glycine, very particularly the species max (soybean) and many others; and the family of the Cruciferae, particularly the genus Brassica, very particularly the species napus (oilseed rape), campestris (beet), oleracea cv Tastie (cabbage), oleracea cv Snowball Y (cauliflower) and oleracea cv Emperor (broccoli); and the genus Arabidopsis, very particularly the species thaliana and many others; the family of the Compositae, particularly the genus Lactuca, very particularly the species sativa (lettuce) and many others.

The transgenic plants are selected in particular among monocotyledonous crop plants, such as, for example, cereals such as wheat, barley, sorghum and millet, rye, triticale, maize, rice or oats, and sugar cane. Further preferred are trees such as apple, pear, quince, plum, cherry, peach, nectarine, apricot, papaya, mango, and other woody species including coniferous and deciduous trees such as poplar, pine, sequoia, cedar, oak, etc. Especially preferred are Arabidopsis thaliana, Nicotiana tabacum, oilseed rape, soybean, corn (maize), wheat, linseed, potato and tagetes. The transgenic plants are furthermore selected in particular from among dicotyledonous crop plants such as, for example, Brassicaceae such oilseed rape, cress, Arabidopsis, cabbages or canola, Leguminosae such as soya, alfalfa, peas, beans or peanut. Solanaceae such as potato, tobacco, tomato, aubergine or peppers, Asteraceae such as sunflower, Tagetes, lettuce or Calendula. Cucurbitaceae such as melon, pumpkin/squash or courgette, and linseed, cotton, hemp. Flax, red pepper, carrot, sugar beet and the various tree, nut and wine species.

Especially preferred for the method of the present invention are maize, Arabidopsis thaliana, Nicotiana tabacum and oilseed rape and all genera and species which are used as food or feedstuffs, such as the above-described cereal species, or which are suitable for the production of oils, such as oil crops (such as, for example, oilseed rape), nut species, soya, sunflower, pumpkin/squash and peanut.

Most preferred plants for the method of the present invention are maize, *Arabidopsis,* sorghum, rice, rapeseed, tobacco, wheat, rye, barley, oat, potato, tomato, sugar beet, pea, sugarcane, asparagus, soy, alfalfa, peanut, sunflower and pumpkin.

The transgenic plant line which is generated in step b) of the method of the invention, or cells or parts of this transgenic plant line, are used in step c) to perform a transient assay. This assay serves to analyze the functionality of the DSBI enzyme. The assay may avoid a time-consuming assessment of the activity of the DSBI enzyme and may allow instead a simple and rapid evaluation of the DSBI functionality.

The transient assay is an intrachromosomal homologous recombination (ICHR) assay. This assay is used to monitor the frequency of intrachromosomal HR.

Preferably, the transient assay of step c) of the method according to the invention comprises a transient transformation of a reporter construct. This transformation can be performed by any of the transformation methods described above with respect to the transformation of the (DSBI encoding) construct, preferably by biolistic bombardment or PEG-mediated protoplast transfection.

Preferably, the reporter construct comprises a nucleic acid sequence to be excised, wherein the nucleic acid sequence comprises at least one recognition sequence which is specific for the enzyme of step a) of the method according to the invention for the site-directed induction of DNA double strand breaks, and wherein the nucleic acid sequence is bordered at both ends by a homology sequence which allows for homologous recombination.

By way of example, but not of limitation, one concept of those reporter constructs will be explained in the following. The transient assay system can employ, for example, a "recombination trap" consisting of overlapping parts of a recombinant gene, for example a beta-glucuronidase (GUS) gene, comprised within the reporter construct. The overlap between two fragments of the gene, e.g. the GUS gene, can be removed by HR, leading to restoration of the functional gene. Such HR events can be detected, e.g. in the case of the GUS gene as blue spots or sectors, when plants or plant parts or cells are histochemically stained.

Preferably, the reporter construct is selected from the group consisting of a GUS construct, a green fluorescent protein (GFP) construct, a chloramphenicol transferase construct, a luciferase construct, a beta-galactosidase construct, an R-locus gene product construct, a beta-lactamase construct, a xyl E gene product construct, an alpha amylase construct, a tyrosinase construct and an aequorin construct. The method of detection of those gene products is well known to the skilled person, and is described in the literature cited above.

After the transient assay of step c), the plant line which is generated in step b) of the method according to the invention is crossed with a plant line containing a nucleic acid sequence to be excised, wherein the nucleic acid sequence to be excised comprises at least one recognition sequence which is specific for the enzyme of step a) for the site-directed induction of DNA double strand breaks, and wherein the nucleic acid sequence to be excised is bordered at both sides by a repeated sequence which allows for a DNA repair mechanism (step d)).

"DNA repair" is a process by which a DNA damage, e.g. a double strand break, is identified and corrected. In the present invention, preferably this repair mechanism is homologous recombination (HR). Alternatively, the mechanism to repair the introduced double strand break may be nonhomologous end joining (NHEJ), precise ligation (PJ), or other mechanisms so that the sequence of interest is fully excised.

Non-homologous end joining (NHEJ) is a pathway that can be used to repair double-strand breaks in DNA. NHEJ is referred to as "non-homologous" because the break ends are directly ligated without the need for a homologous template, in contrast to homologous recombination, which requires a homologous sequence to guide repair. The term "non-homologous end joining" was coined in 1996 by Moore J.K. and Haber J.E. (Mol Cell Biol. 1996 May;16(5):2164-73). NHEJ typically utilizes short homologous DNA sequences, termed microhomologies, to guide repair. Microhomologies in the single-stranded overhangs that are often present on the ends of double-strand breaks are used to promote restorative repair. When these overhangs are compatible, NHEJ almost always repairs the break accurately, with no sequence loss. Imprecise repair leading to loss of nucleotides can also occur, but is much less common. A number of proteins are involved in NHEJ. The Ku heterodimer, consisting of Ku70 and Ku80, forms a complex with the DNA dependent protein kinase catalytic subunit (DNA-PKcs), which is present in mammals but absent in yeast. The DNA Ligase IV complex, consisting of the catalytic subunit DNA Ligase IV and its cofactor XRCC4, performs the ligation step of repair. The recently discovered protein XLF, also known as Cernunnos, is also required for NHEJ.

The term "crossing" means the mating between two plants (eventually representing two plant lines) wherein the two individual plants are of not-identical genetic background. In other words, the two parental types have different genetic constitution. For the cross-pollination with the plants comprising the nucleic acid to be excised, both the T₀ lines and the homozygous T₁ lines can be used.

The crossing may be performed via pollination. In general, pollination is the transfer of pollen from the male reproductive structure of a flower to the female reproductive structure of a flower. More precisely, the pollination is the transfer of pollen from an anther (of the stamen) to the stigma (of a pistil). The pollination, which represents the sexual reproduction in plants, results in fertilization and, usually, seed production. In general, pollination may occur on a single plant (self-pollination) or between different plants or plant varieties (cross-pollination).

In a preferred embodiment, the "nucleic acid sequence to be excised" comprises the T-DNA region or part thereof. Another possibility is that the nucleic acid sequence to be excised encodes a selection marker. This selection marker is preferably selected from the group consisting of negative selection markers, markers conferring resistance to a biocidal metabolic inhibitor, to an antibiotic or to a herbicide, positive selection markers and counter-selection markers.

Most preferably, the selection marker is selected from the group consisting of acetohydroxy acid synthase, D-serine deaminase, phosphinothricin acetyltransferase, 5-enolpyruvylshikimate-3-phosphate synthase, glyphosates degrading enzymes, dalapono inactivating dehalogenases, sulfonylurea- and imidazolinone-inactivating acetolactate synthases, bromoxynilo degrading nitrilases, Kanamycin- or G418-resistance genes, neomycin phosphotransferase, 2-desoxyglucose-6-phosphate phosphatase, hygromycin phosphortransferase, dihydrofolate reductase , D-amino acid metabolizing enzyme, D-amino acid oxidase, gentamycin acetyl transferase, streptomycin phosphotransferase, aminoglycoside-3-adenyl transferase, bleomycin resistance determinant, isopentenyltransferase, beta-glucoronidase, mannose-6-phosphate isomerase, UDP-galactose-4-epimerase, cytosine deaminase, cytochrome P-450 enzymes, indoleacetic acid hydrolase, haloalkane dehalogenase and thymidine kinase.

Finally, after the crossing step d), a tissue culture regeneration through callus formation is performed. This step allows for recovering a transgenic plant containing a DNA double strand break inducing enzyme.

The method of the present invention also comprises the identification of a single copy transgenic line following step b) or c). Preferably a single copy transgenic plant line is used for the further steps of the method of the invention. In the context of the present invention, the term "single copy" refers to the double strand break inducing enzyme.

The identification of a single copy transgenic line may be performed via any standard molecular technique that is known to the person skilled in the art. Preferably, the single copy identification is performed via quantitative PCR or Southern hybridization.

Quantitative PCR is a technique used to simultaneously quantify and amplify a specific part of a given DNA molecule. It is used to determine whether or not a specific sequence is present in the sample, and if it is present, the number of copies in the sample. The procedure follows the general pattern of polymerase chain reaction, but the DNA is quantified after each round of amplification. Two common methods of quantification are the use of fluorescent dyes that intercalate with double-strand DNA, and modified DNA oligonucleotide probes that fluoresce when hybridized with a complementary DNA. The techniques include SYBR Green quantitative PCR, Probe-based quantitative PCR and Quantitative Reverse Transcriptase PCR.

Details about PCR technologies may be found, e.g. in "PCR - Polymerase-Kettenreaktion. Das Methodenbuch" (Hans-Joachim Müller, Spektrum Akademischer Verlag, June 2001), "Molekularbiologische Diagnostik" (Frank Thiemann Hoppenstedt Publishing, 2002) or "Der Experimentator: Molekularbiologie / Genomics" (Cornel Mülhardt, Spektrum Akademischer Verlag, April 2006).

"Southern hybridization" or "Southern blot" is a method of enhancing the result of an agarose gel electrophoresis by marking specific DNA sequences. By way of a general example, but not of limitation, the method comprises the following steps:
1. DNA fragments are electrophoresed on a gel to separate DNA (e.g. deriving from a PCR) based on size.
2. If DNA is larger than 15 kb, prior to blotting, the gel may be treated with a dilute acid, such as dilute HCl which acts to depurinate the DNA fragments. This breaks the DNA into smaller pieces that will be able to complete the transfer more efficiently than larger fragments.
3. The gel from the DNA electrophoresis is treated with an alkaline solution (typically containing sodium hydroxide) to cause the double-stranded DNA to denature, separating it into single strands. Denaturation is necessary so that the DNA will stick to the membrane and be hybridized by the probe (see below).
4. A sheet of nitrocellulose (or, alternatively, nylon) membrane is placed on top of the gel. Pressure is applied evenly to the gel (either using suction, or by placing a stack of paper towels and a weight on top of the membrane and gel). This causes the DNA to move from the gel onto the membrane by capillary action, where it sticks.
5. The membrane is then baked (in the case of nitrocellulose) or exposed to ultraviolet radiation (nylon) to permanently crosslink the DNA to the membrane.
6. The membrane is now treated with a hybridization probe - an isolated DNA molecule with a specific sequence that pairs with the appropriate sequence (the appropriate sequence is the complementary sequence of what the restriction enzyme recognized). The probe DNA is labelled so that it can be detected, for example by incorporating radioactivity or tagging the molecule with a fluorescent or chromogenic dye. In some cases, the hybridization probe may be made from RNA, rather than DNA.
7. After hybridization, excess probe is washed from the membrane, and the pattern of hybridization is visualized on X-ray film, or equivalent technology, by autoradiography in the case of a radioactive or fluorescent probe, or by development of color on the membrane itself if a chromogenic detection is used.

The method was first described by Southern, E.M. (1975): "Detection of specific sequences among DNA fragments separated by gel electrophoresis", J Mol Biol., 98:503-517. The skilled person knows how to perform a Southern blot, for example according to the description in T. Maniatis, E. F. Fritsch and J. Sambrook, Molecular Cloning: A Laboratory Manual, Cold Spring Harbor Laboratory, Cold Spring Harbor, NY (1989).

In another preferred embodiment of the present invention, the method comprises the analysis of the transgene expression level following step b) or c). The term "transgene" refers in this context to the double strand break inducing enzyme. In this context, the term "expression" includes transcription and translation. Preferably, a high or at least a medium expressing plant line is used for the further steps of the method of the invention. This step is performed in addition to the identification of a single copy transgenic line, most preferably subsequent to it.

This analysis can be performed via any standard molecular technique that is known to the person skilled in the art. Preferably, the analysis of the transgene expression level is performed via RT-PCR or Northern hybridization.

Reverse transcription polymerase chain reaction (RT-PCR) is a technique for amplifying a defined piece of an RNA molecule. The RNA strand is first reverse transcribed into its DNA complement or complementary DNA, followed by amplification of the resulting DNA using polymerase chain reaction. This can either be a 1 or 2 step process. Polymerase chain reaction itself is the process used to amplify specific parts of a DNA molecule, via the temperature-mediated enzyme DNA polymerase.

In the first step of RT-PCR, called the "first strand reaction", complementary DNA is made from a mRNA template using dNTPs and an RNA-dependent DNA polymerase, reverse transcriptase, through the process of reverse transcription. The above components are combined with a DNA primer in a reverse transcriptase buffer for an hour at about 37°C. After the reverse transcriptase reaction is complete, and complementary DNA has been generated from the original single-stranded mRNA, standard polymerase chain reaction, termed the "second strand reaction" is initiated.
1. A thermostable DNA polymerase and the upstream and downstream DNA primers are added.
2. The reaction is heated to temperatures above about 37°C to facilitate sequence specific binding of DNA primers to the cDNA (copy DNA).
3. Further heating allow the thermostable DNA polynerase ("transcriptase") to make double-stranded DNA from the primer bound cDNA.
4. The reaction is heated to approximately 95°C to separate the two DNA strands.
5. The reaction is cooled enabling the primers to bind again and the cycle repeats.

After approximately 30 cycles, millions of copies of the sequence of interest are generated. The original RNA template is degraded by RNase H, leaving pure cDNA (plus spare primers).

This process can be simplified into a single step process by the use of wax beads containing the required enzymes for the second stage of the process which are melted, releasing their contents, on heating for primer annealing in the second strand reaction. Northern blot techniques may be used to study the RNA's gene expression further.

The "Northern blot" or "Northern hybridization" is a technique used to study gene expression. It takes its name from the similarity of the procedure to the Southern blot procedure, used to study DNA, with the key difference that RNA, rather than DNA, is the substance being analyzed by electrophoresis and detection with a hybridization probe. A notable difference in the procedure (as compared with the Southern blot) is the addition of formaldehyde in the agarose gel, which acts as a denaturant. As in the Southern blot, the hybridization probe may be made from DNA or RNA.

The skilled person knows how to perform a Nouthern blot, for example according to the description in T. Maniatis, E. F. Fritsch and J. Sambrook, Molecular Cloning: A Laboratory Manual, Cold Spring Harbor Laboratory, Cold Spring Harbor, NY (1989).

In another preferred embodiment of the present invention, the method comprises the pollination of the transgenic plant line following step b) or c), wherein the pollination is either self-pollination or cross-pollination with a wild-type plant line. Self-pollination is preferred. Preferably, the lines that were previously identified to have a single copy DSBI transgene and/or to have a high or at least a medium DSBI expression are used for the pollination step. After the self-pollination of the so-called "T₀ lines" (the lines arising from step b) of the method of the invention), the seeds obtained are called "T₁ seeds".

The term "wild-type", "natural" or of "natural origin" means with respect to an organism, polypeptide, or nucleic acid sequence, that said organism is naturally occurring or available in at least one naturally occurring organism which is not changed, mutated, or otherwise manipulated by man.

Preferably, the seeds and/or seedlings obtained through the pollination (the "T₁" seedlings) are analyzed for their zygosity. In the present case, this analysis determines the presence of the DSBI transgene and serves to identify the homozygous and the hemizygous plants. The term "homozygous" refers to two DNA sequences in the organism each located in the same genomic location, one on each homologous chromosome, "heterozygous", or interchangeably hemizygous describes the presence of only a single copy of a gene (e.g. a transgene) on a single chromosome in an otherwise diploid (or polyploid) organism.

The zygosity analysis may be performed according to any standard molecular technique which is known to the person skilled in the art, for example via quantitative PCR, Southern hybridization and/or fluorescence *in situ* hybridization. The latter is defined as the use of a nucleic acid probe to detect and identify specific complementary sequences of DNA in chromosomes or RNA eukaryotic cells and tissues. The detection is performed via fluorescence, e.g. a probe coupled to a fluorescent dye.

In a further preferred embodiment, the homozygous lines which are identified after the zygosity analysis are selected for the crossing of step d) of the method according to the invention.

The seeds and/or seedlings (called "F₁") obtained by step e) of the method according to the invention are analyzed for DNA double strand break mediated homologous recombination.

Preferably, this homologous recombination analysis of the seeds and/or seedlings is determined by standard molecular techniques including PCR analysis, colorimetric or biochemical assays, or DNA sequencing. This analysis may be performed by the method as described for step c) of the method according to the invention. Alternatively or additionally, a PCR analysis may be performed. The selection of the primers depends on the nucleic acid sequence to be excised. The skilled person knows how to design an adequate PCR reaction, e.g. as described in T. Maniatis, E. F. Fritsch and J. Sambrook, Molecular Cloning: A Laboratory Manual, Cold Spring Harbor Laboratory, Cold Spring Harbor, NY (1989).

The method as defined above can also be reversed, which means that it is directed to the reciprocal process. In this case, the transformation of step a) is performed with a construct encoding a nucleic acid sequence to be excised, the transient assay of step c) is performed in order to assess the functionality of the recognition sequence and the repeated sequence of the construct of step a), and the generated transgenic plant line is crossed with a plant line containing a DNA double strand break inducing enzyme. Therefore, the present invention is also directed to a method for excising a nucleic acid sequence from the genome of a plant or of a plant cell, comprising:
a) transforming a plant cell with a construct encoding a nucleic acid sequence to be excised, wherein the nucleic acid sequence to be excised comprises at least one recognition sequence which is specific for a DNA double strand break inducing enzyme for the site-directed induction of DNA double strand breaks, and wherein the nucleic acid sequence to be excised is bordered at both sides by a repeated sequence which allows for a DNA repair mechanism,
b) generating a transgenic plant line from the cell of step a),
c) performing a transient assay with the plant line of step b) or cells or parts thereof to analyze the functionality of the recognition sequence and the repeated sequence of the construct of step a), wherein the transient assay is an intrachromosomal homologous recombination assay and wherein the method further comprises the identification of a single copy transgenic line following step b) or c);
d) crossing the plant line of step b) with a plant line containing a DNA double strand break inducing enzyme, and
e) performing a tissue culture regeneration through callus formation of F1 immature embryos, wherein the seeds and/or seedlings obtained by step e) are analyzed for DNA double strand break mediated repair mechanisms including homologous recombination and lines in which double strand break-induced homologous recombination has occurred are identified.

All of the terms and procedures defined above apply in the same way to this reciprocal method, with the exception of the transient assay, which is preferably performed with a construct encoding a DNA double strand break inducing enzyme.

As an alternative to the crossing of step d), another approach of obtaining marker excision events is the "re-transforming" of the plant line - which was generated in step b) and analyzed in step c) - with a construct.

In the case that the transforming of step a) was performed with a construct encoding a DNA double strand break inducing enzyme, the re-transformation is performed with a construct encoding a nucleic acid sequence to be excised. Therefore, the present invention is directed to a method for excising a nucleic acid sequence from the genome of a plant or of a plant cell as defined above, wherein the crossing of step d) is replaced by a re-transforming of the plant line of step b) with a construct encoding a nucleic acid sequence to be excised, wherein the nucleic acid sequence to be excised comprises at least one recognition sequence which is specific for the enzyme of step a) for the site-directed induction of DNA double strand breaks, and wherein the nucleic acid sequence to be excised is bordered at both sides by a repeated sequence which allows for a DNA repair mechanism, wherein the nucleic acid sequence to be excised comprises at least one recognition sequence which is specific for the enzyme of step a) for the site-directed induction of DNA double strand breaks, and wherein the nucleic acid sequence to be excised is bordered at both sides by a repeated sequence which allows for a DNA repair mechanism.

In the "reciprocal" method, where the transforming of step a) was performed with a construct encoding a nucleic acid sequence to be excised, the re-transforming is performed with a construct encoding a DNA double strand break inducing enzyme. Therefore, the present invention is also directed to a method for excising a nucleic acid sequence from the genome of a plant or of a plant cell as defined above, wherein the crossing of step d) is replaced by a re-transforming of the plant line of step b) with a construct encoding a DNA double strand break inducing enzyme.

The re-transformation process of the transgenic plant line with the second construct may be performed according to the description above, i.e. by any of the above-mentioned transformation methods. The same applies to the structure of the second construct (genetic control elements, promoters, enhancers, polyadenylation signals, ribosome binding sites etc.), which may be designed according to the above description.

In this alternative method, the skilled person is aware that another (a second) selection marker system should preferably be used for the second transformation. If, for example, the selection marker system for the first construct was based on the "ahas" gene, the second selection marker system could be the "dsdA" gene comprised within the expression cassette of the construct encoding the nucleic acid sequence to be excised, or vice versa. Any other combination of any of the above-mentioned selection markers or any selection marker that is known to the skilled person can be used likewise.

Preferably, retransformation is performed on the transgenic plant containing the excision target DNA sequence, especially when the excision target DNA sequence is the first selection marker gene. For example, immature embryos derived from the first transgenic line containing the ahas selection marker gene that is the excision target is dissected, infected and co-cultivated with an agrobacterium strain containing a plasmid comprising of a second transformation cassette with the dsdA gene as the selection marker. The similar transformation steps are followed as if the first transgenic line is wildtype in reference to the second selectable marker gene. If the first transgenic line contains the first T-DNA with the ahas gene, for example, applying only D-serine for the dsdA gene can do the selection. Thus produced plants are analysed for the excision events.

The plant or progeny, propagation material, part, tissue, cell or cell culture obtained by the method according to the invention may be used as aliment, fodder or seeds or for the production of pharmaceuticals or chemicals.

The plants obtained by the method according to the invention may be consumed by humans or animals and may therefore also be used as food or feedstuffs, for example directly or following processing known in the art. Here, the deletion of, for example, resistances to antibiotics and/or herbicides, as are frequently introduced when generating the transgenic plants, makes sense for reasons of customer acceptance, but also product safety.

The above-described plants and structures derived from them may be used for the production of pharmaceuticals or chemicals, especially fine chemicals. Here again, the deletion of, for example, resistances to antibiotics and/or herbicides is advantageous for reasons of customer acceptance, but also product safety.

A "pharmaceutical" is understood as meaning a drug, a chemical drug, or a medicine, which is used in medical treatment, prevention or vaccination. "Fine chemicals" is understood as meaning enzymes, vitamins, amino acids, sugars, fatty acids, natural and synthetic flavors, aromas and colorants widely usable. Especially preferred is the production of tocopherols and tocotrienols, and of carotenoids. Culturing the transformed host organisms, and isolation from the host organisms or from the culture medium, is performed by methods known to the skilled worker. The production of pharmaceuticals such as, for example, antibodies or vaccines, is described by Hood E E, Jilka J M. (1999) Curr Opin Biotechnol. 10(4):382-386; Ma J K and Vine N D (1999) Curr Top Microbiol Immunol. 236:275-92).

Various further aspects and embodiments of the present invention will be apparent to those skilled in the art in view of the present disclosure. Certain aspects and embodiments of the invention will now be illustrated by way of example. It is to be understood that this invention is not limited to the particular methodology, protocols, cell lines, plant species or genera, constructs, and reagents described as such.

### References:

Nutter RC, Scheets K, Panganiban LC, Lommel SA. The complete nucleotide sequence of the maize chlorotic mottle virus genome. Nucleic Acids Res. 1989 Apr 25;17(8):3163-77.
Scheets K, Khosravi-Far R, Nutter RC. Transcripts of a maize chlorotic mottle virus cDNA clone replicate in maize protoplasts and infect maize plants. Virology. 1993 Apr;193(2):1006-9.
Scheets K. Maize chlorotic mottle machlomovirus and wheat streak mosaic rymovirus concentrations increase in the synergistic disease corn lethal necrosis. Virology. 1998 Mar 1;242(1):28-38.
Scheets K. Maize chlorotic mottle machlomovirus expresses its coat protein from a 1.47-kb subgenomic RNA and makes a 0.34-kb subgenomic RNA. Virology. 2000 Feb 1;267(1):90-101.
Czako M, Wenck AR, Marton L (1996) Negative selection markers for plants. In: Gresshoff PM (ed) Technology transfer of plant biotechnology. CRC press, Boca Raton, pp 67-93.
Jefferson RA (1987) Assaying chimeric genes in plants: the GUS gene fusion system. Plant Mol Biol Rep 5:387-405.
Schrott M (1995) Selectable marker and reporter genes. In: Potrukus I (ed) Gene transfer to plants. Springer, Berlin, Heidelberg, New York, pp 325-336.
Kozak, M (1987) An analysis of 5'-noncoding sequences from 699 vertebrate messenger RNAs Nucleic Acids Research, 15:20 8125-8148.

### EXAMPLES

### Materials and General Methods

Unless indicated otherwise, chemicals and reagents in the Examples were obtained from Sigma Chemical Company (St. Louis, MO), restriction endonucleases were from New England Biolabs (Beverly, MA) or Roche (Indianapolis, IN), oligonucleotides were synthesized by MWG Biotech Inc. (High Point, NC), and other modifying enzymes or kits regarding biochemicals and molecular biological assays were from Clontech (Palo Alto, CA), Pharmacia Biotech (Piscataway, NJ), Promega Corporation (Madison, WI), or Stratagene (La Jolla, CA). Materials for cell culture media were obtained from Gibco/BRL (Gaithersburg, MD) or DIFCO (Detroit, MI). The cloning steps carried out for the purposes of the present invention, such as, for example, restriction cleavages, agarose gel electrophoresis, purification of DNA fragments, transfer of nucleic acids to nitrocellulose and nylon membranes, linking DNA fragments, transformation of E. coli cells, growing bacteria, multiplying phages and sequence analysis of recombinant DNA, are carried out as described by Sambrook (1989). The sequencing of recombinant DNA molecules is carried out using ABI laser fluorescence DNA sequencer following the method of Sanger (Sanger 1977).

### 1. Double strand break (DSB) mediated homologous recombination for marker excision in maize

Homing endonuclease (HEN)-expression in plant cells can enhance intrachromosomal homologous recombination (ICHR). The expression level of the HEN transgene in the HEN plants plays an important role in influencing ICHR rate. In a conventional way, the ICHR assays are performed on tissues from progeny plants derived from crosses between HEN-expressing plants and plants containing the target sequences (Figure 9A). Compared to Arabidopsis, as a plant species representing small size and short generation time, evaluation of this system in maize requires large amount of efforts, time, and resources including the greenhouse space as well as long generation time. To overcome these discrepancies and obtain successful marker excision in maize in an efficient and effective manner, the following methods were developed (Figures 9B and 9C).

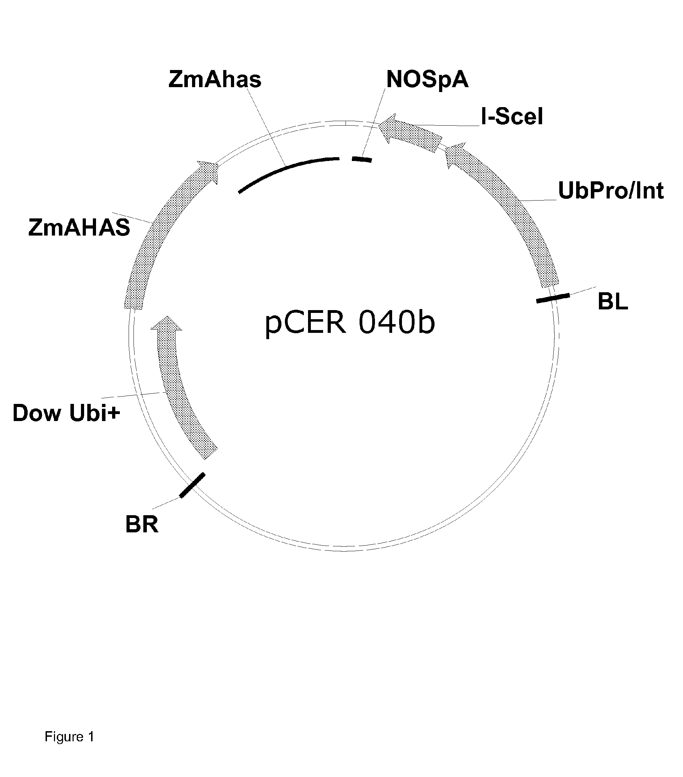

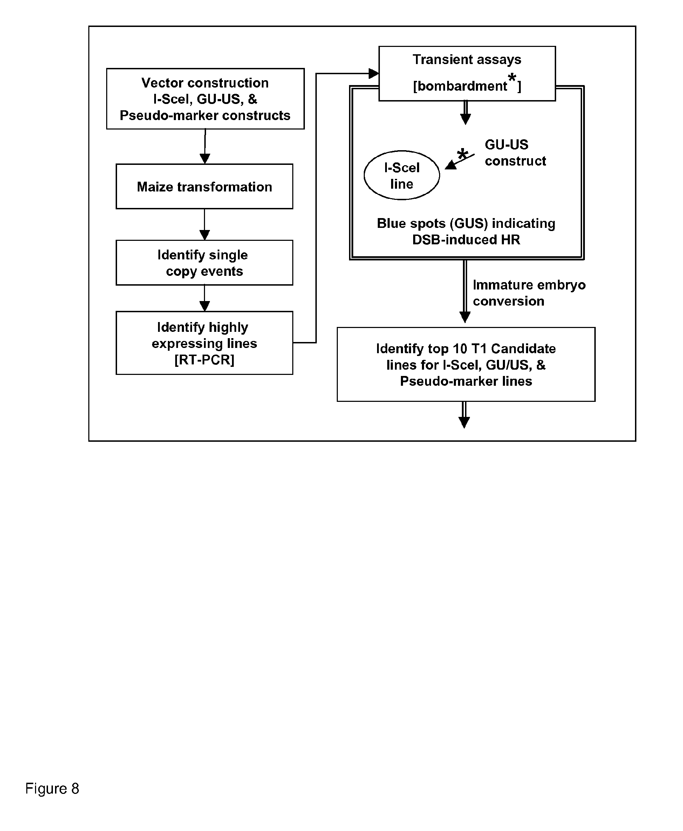

In maize, the selected T0 plants (HEN lines as well as excision capable lines) were selfed to obtain T1 seeds. The T1 seedlings were examined for a zygocity test using TaqMan assays in order to identify homozygous lines. T0 lines showing medium to high levels of transgene expression were selected to increase efficiency of DSB-mediated HR and limit the number of plants to be used for crossing (Figure 8). First, single copy lines were identified using a TaqMan semi-quantitative PCR assay. These single copy lines were tested in order to identify medium to high expression of the introduced gene (I-SceI or GU-US, Figures 1-4) at the mRNA levels using TaqMan real-time RT-PCR. Transgene expression levels were normalized to the expression level of an endogenous gene (single copy gene is preferable). Leaf tissues from some of the strongest I-SceI expressing lines were transformed with a plasmid encoding the GU-US gene for transient ICHR assays via biolistic transformation. Successful ICHR is indicated in this assay by the detection of blue GUS positive spots on bombarded leaf samples. This process can also be conducted by transferring I-SceI construct into leaf tissues of the transgenic GU-US lines. This transient assay system facilitated the identification of the HEN transgenic lines that were the best candidates for successful DSB-mediated HR. The selected transgenic lines were used for further experimentation to obtain DSB-mediated HR plants.

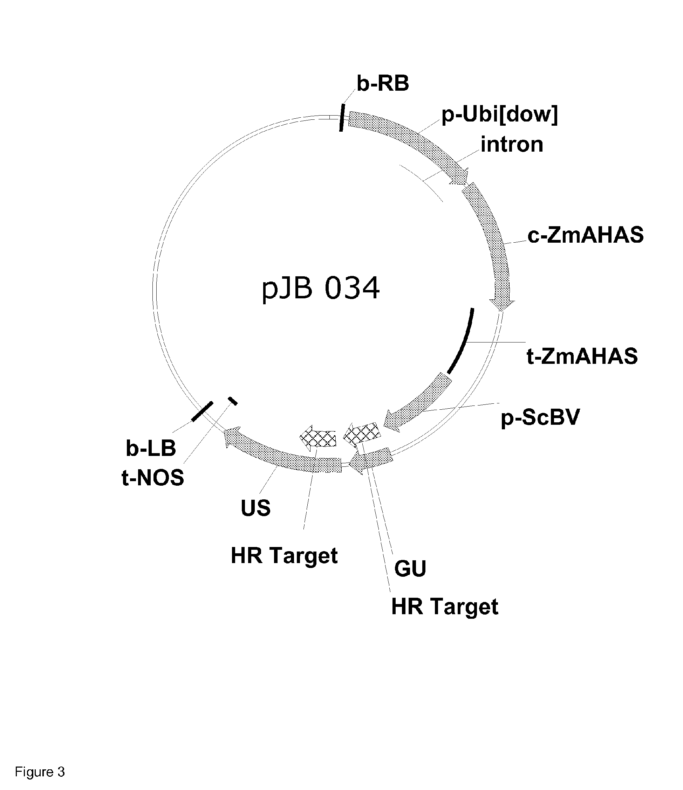

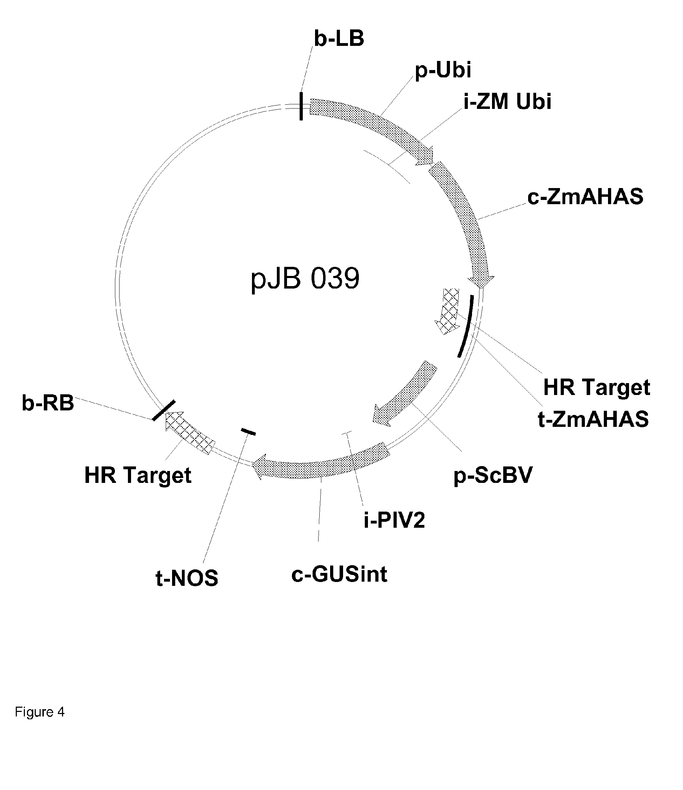

The selected HEN homozygous lines were cross pollinated with the plants comprising their complementary constructs (Figures 3 and 4); that is, the I-SceI lines were crossed with lines harboring the reporter constructs (GU-US) and the reporter lines were crossed with lines harboring the I-SceI constructs. As a control following the conventional method, the resulting progeny seeds comprising both I-SceI and GU-US constructs were analyzed for DSB-induced HR directly, and planted for evaluation of recombination in the whole plants (Figure 9A). In the progeny seeds comprising both I-SceI and reporter constructs, DSB-mediated HR was observed strongly in endosperm and sporadically in scutellum, but not in the embryo axis under the control of constitutive promoters (e.g. maize ubiquitin promoter in combination with maize ubiquitin intron) for expression of I-SceI and excision capable gene or expression cassette. Since scutellum is the target tissue for maize transformation and regeneration via *Agrobacterium,* the immature embryos of the F1 plants were used for regeneration process to recover DSB-mediated HR plants via embryognic callus culture. This process allowed propagating and differentiating the tissues that showed DSB-mediated HR. Therefore even though DSB-mediated HR did not occur in the embryo axis, the probability of identifying DSB-mediated HR lines was significantly increased (Figure 9B). In addition, the I-SceI constructs were re-transformed into the immature embryos of the homozygous GU-US lines to improve DSB-mediated HR, since this process will go through regeneration process (Figure 9C). Table 1 summarizes the basic features and the timeframes of these new approaches.

**Table 1. Summary of three approaches for obtaining marker-free event with HEN I-SceI gene in corn. ¹ Approaches based on Figure 9. ² A generation time is needed to segregate the I-SceI gene from the final excision-target marker-free event.**

| Approaches¹ | Timeline² to obtain the excision target marker-free evens | Features exemplified in this invention |
|---|---|---|
| A. | Minimum 19 months | Low chance of obtaining marker excision event, and/or requirement for large scale seed screening (due to low frequency of full excision in a seed). |
| B | 8 month | Increased chance of obtaining marker-excision event (due to the potential of recovering excision event at a single cell level, and the tissue culture process promotes the recombination activities). |
| C | 9months | Increased chance of obtaining marker-excision event (due to the high potential of obtaining the fully excised event based on single cell transformation) |

As described above, when a strong, constitutive, ubiquitous promoter was used to express the HEN gene and GU-US, no DSB-mediated HR, (indicated by blue spots) was detected in embryo axis in maize upon analysis of mature kernel. To achieve DSB-mediated HR in embryo, the super promoter was chosen, since this promoter in maize shows strong expression in the whole embryo (scutellum and embryo axis) during germination, calli (including embryogenic calli) during regeneration. The expression levels in these tissues can be enhanced by addition of intron-mediated enhancement (IME)-conferring intron between the super promoter and I-*Sce*I gene.

The I-SceI homing endonuclease gene sequence was optimized to improve expression and mRNA stability. The sequence was optimized using a 50% mix of maize and soybean preferred codons. RNA instability motifs, codon repeats, cryptic splice sites, unwanted restriction sites and mRNA secondary structures were identified and removed to arrive at the final optimized sequence. The synthetic sequence was synthesized by Entelechon GmbH, Regensburg, Germany. The gene was synthesized with adenine at -3 (+1 nucleotide for adenine of ATG as a translational start codon) Kozak consensus (Kozak, 1987), selected restriction sites and Gateway attachment regions (Figure 22).
SEQ ID NO: 26 coding sequence of codon optimized I-SceI
SEQ ID NO: 27 optimized I-SceI CDS with attachment regions

With the method according to the invention, fully recombined maize was generated in the T₁ generation for both the excision of an entire reporter gene cassette and for recombination of the GU/US reporter at the rate of 1.8-9.6% and at 5.4-16.4% efficiency at 95% confidence, respectively.

The following flowchart is provided to give a brief and simplified overview over the examples described in the following. It is not to be understood as limiting.
A. Agrobacterium-mediated transformation of Maize immature embryos
   a) with I-Sce I construct (selectable marker cassette: ahas)
   b) with GUS pseudo marker excision construct (marker: ahas)
   c) with GU-US reporter construct (marker: ahas)
   Agrobacterium inoculation, co-cultivation, selection, and plant regeneration
   1. Inoculating the immature embryos with agrobacterium cell suspension;
   2. Performing co-cultivation of immature embryos and agrobacterium on the medium without antibiotics and selection agents;
   3. Transferring cultures to the recovery medium that contains antibiotics, but without the selection agents; Embryogenic callus production initiates from the scutellum at this stage;
   4. Selecting transgenic embryogenic calli on medium with selection agent;
   5. Recovering plantlets on regeneration medium with the selection agent;
   6. Transferring young T₀ seedlings to rooting medium with the selection agents;
   7. Performing TaqMan copy number analysis, and identify single copy events;
   8. Transferring rooted, TaqMan-positive, single copy plants to soil;
   9. Determining the transgene expression level via RT-PCR;
   10. To lines are self-pollinated to obtain T₁ seeds (harvest and storage). T₁ seedlings are examined for homozygocity via TaqMan assay
B. Semi-transient assay system for the proof of concept on DSB-mediated HR:
   GU-US plasmid is introduced in the I-Sce I expressing Maize lines
   a) in leaf tissue via particle gun or
   b) in protoplasts via PEG-mediated transformation
      determination of the transient expression levels of the reporter gene
      = transient ICHR assay (intrachromosomal homologous recombination) detection of the blue = positive spots
      → identification of the HEN transgenic T₀ lines that are the best candidates
C. Cross pollination
   of a selected homozygous transgenic I-Sce I line with a line containing a selection marker bordered by excision sites (or with a GU-US or a GUS construct):
   I-Sce I x GU-US = F1 progenies (embryos / seeds) are obtained
D. Immature embryo conversion
   (a process to recover a plant from an immature embryo via in vitro conversion/germination of an immature embryo to a full seedling without callus formation. F1 immature embryos are placed onto the rooting medium without auxin 2,4-D, and immature embryos are then converted into seedlings.)
   (to avoid the time-consuming screening of F1 plants which are derived from the mature seeds)
   F1 immature embryos are placed on rooting medium containing the selection agent against the selection marker linked to the I-Sce I gene → seedlings are recovered
E. Plant Regeneration via embryogenic calli
   This is a process for recovering potentially marker-excised plants through a tissue culture process via callus formation. F1 immature embryos are cultured on the recovery (to promote callus formation), the selection (to promote embryogenic callus formation and to select cells containing integrated T-DNA), regeneration (to convert mature calli to plantlets) and then the rooting (to recover full seedlings) media. The major advantage of this process is to recover a F1 plant from a single cell, hence to separate/select a marker-excised cell line.
F. Re-transformation of a transgenic line
   This is a technique applied to marker excision in addition to the routine crossing between two transgenic lines (the marker-excision target line and the HEN line, for example). To conduct re-transformation, immature embryos of the transgenic line A (e.g. the excision target line with first selection marker gene in the T-DNA) is transformed again with the agrobacterium strain containing HEN gene in its T-DNA, or immature embryos of the transgenic line B (e.g. the HEN line) can be transformed again with the agrobacterium strain containing the excision target T-DNA (or vice versa). The marker-excised seedlings can be recovered from the re-transformation process.
G. Plant analysis for the DSB-mediated HR or marker excision
   molecular screening for identification of the marker-free (= full marker-excision) plants
   a) GUS histochemical staining assay of leaf and kernels
   b) PCR analysis for marker excision

### 1.1 Vector Construction

### 1.1.1 Homing endonuclease (I-SceI) constructs

I-*Sce*I was PCR amplified from vector pCB586-4 using primers 1 and 2.
SEQ ID NO: 7: Primer 1 (*Asc*I-ATG-I-*Sce*I 5'):
   5'-AGGCGCGCCATGAAAAACATCAAAAAAAACCA
SEQ ID NO: 8: Primer 2 (*Sbj*I-TAA- *I-SceI* 3'):
   5'-GCTCCTGCAGGTTATTTCAGGAAAGTTTC

The resulting PCR product was digested with *Asc*I and *Sbf*I and cloned into *Asc*I and *Sbf*I digested vector pLM065 to produce vector pJB010, which comprises an expression cassette wherein the maize Ubiquitin promoter drives the expression of I-*Sce*I.

Vector pCER 040b is a binary expression vector where the maize Ubiquitin promoter drives the expression of I-*Sce*I, and was generated by ligation of the T4 DNA polymerase filled-in *Pme*I-*Xba*I fragment of pJB010 into T4 DNA Polymerase filled in *Asc*I-*Pac*I digested pEG085.

Vector pCER 041 is a binary expression vector where the sugarcane bacilliform virus (ScBV) promoter drives the expression of I-*Sce*I*,* and was generated by ligation of the T4 DNA polymerase filled-in *Pac*I-*Asc*I fragment of pJB010 into T4 DNA Polymerase filled in *Eco*RV*-Asc*I digested pEG085.

### 1.1.2 GU-US construct

Vector pCB642-2 encodes an expression cassette wherein the GUS ORF comprises an internal duplication of 610 base pairs, with an I-SceI recognition site situated between the duplicated regions (GU-US). The fragment encoding the GU-US and NOS terminator was isolated from pCB642-2 by digestion with *Hin*dIII and *Spe*I, and was cloned into *Hin*dIII and *Spe*I digested pBluescript to generate pJB028.

The binary GU/US expression vector pJB034 was generated by ligation of the T4 DNA Polymerase filled in *Spe*I*-Xho*I fragment from pJB028 into T4 DNA Polymerase filled in *Eco*RV*-Asc*I digested pEG085.

### 1.1.3 Pseudo-marker excision construct

Vector pJB035 was generated by ligating the *Asc*I fragment of pBPSMM247b comprising the GUS expression cassette (p-ScBV:GUS:t-NOS) into *Asc*I-digested pUC001.

In order to introduce I-*Sce*I sites flanking the GUS expression cassette in pJB035, oligos encoding the recognition sequence were generated. Annealing of oligos 3 and 4 resulted in a double-stranded I-*Sce*I recognition site with a *Sal*d-compatible overhang on one end and an *Xba*I-compatible overhang on the other.
SEQ ID NO: 9: Oligo 3 (*Sal*I I-*Sce*I): 5'-TCGATAGGGATAACAGGGTAAT
SEQ ID NO: 10: Oligo 4 (*Xba*I-I-*Sce*I): 5'-CTAGATTACCCTGTTATCCCTA

An I-*Sce*I site was added downstream of the GUS expression cassette by digesting pJB035 with *Sal*I and *Xba*I and ligating in annealed oligos 3 and 4, thereby generating pJB036.

Oligos 5 and 6 were generated in order to produce a double stranded I-*Sce*I sequence with *Pac*I compatible ends.
SEQ ID NO:11: Oligo 5 (*Pac*I-I-*Sce*I 5'): 5'-TAGGGATAACAGGGTAAT
SEQ ID NO: 12: Oligo 6 (*Pac*I-I-*Sce*I 3'): 5'-TACCCTGTTATCCCTAAT

Annealed oligos 5 and 6 were ligated into *Pac*I-digested pJB036 to generate pJB037.

The final pseudo-marker excision vector required a duplicated DNA sequence flanking the I-*Sce*I sites in order to serve as a target sequence for homologous recombination (HR target). For this purpose, a portion of the maize AHAS terminator was duplicated. The region was excised via the 850 bp *Eco*RI*-Kpn*I fragment from vector pEG085. This *Eco*RI*-Kpn*I fragment was cloned into *Eco*RI and *Kpn*I digested pJB037 to generate pJB038, which comprises the [I-*Sce*I:p-ScBV:GUS:t-NOS:I-*Sce*I:HR target] pseudo-marker cassette.

The pseudo-marker binary vector was generated by ligation of the 5.2Kb *Hpa*I-*Pme*I fragment from pJB038 into T4 DNA Polymerase filled in *Pac*I *- Asc*I digested pEG085, to generate pJB039.

### 1.2 Agrobacterium-mediated corn transformation and regeneration

### 1.2.1 Plant tissue culture and bacterial culture media

Unless indicated otherwise, chemicals and reagents in the Examples were obtained from Sigma Chemical Company (St. Louis, MO). Materials for cell culture media were obtained from Gibco/BRL (Gaithersburg, MD) or DIFCO (Detroit, MI). The cloning steps carried out for the purposes of the present invention, such as, for example, transformation *of E. coli* cells, growing bacteria, multiplying phages and sequence analysis of recombinant DNA, are carried out as described by Sambrook (1989). The following examples are offered by way of illustration and not by way of limitation.

### Media Recipes

Imazethapyr (Pursuit) stock solution (1 mM) is prepared by dissolving 28.9 mg of Pursuit into 100 ml of DMSO (Sigma), and stored at 4°C in the dark. Acetosyringone stock is prepared as 200 mM solution in DMSO and stored at -20°C. D-serine stock solution is prepared in the double distilled water, filter-sterilized and store at 4°C.

**Table 2. Maize YP Media (for growing Agrobacterium)**

| Media Components | Supplier/Catalog # | Final Concentration |
|---|---|---|
| Yeast extract | Sigma Y1626 | 5 g/L |
| Peptone (from meat) | EM V298413 | 10 g/L |
| NaCl | Sigma S5886 | 5 g/L |

Adjust pH to 6.8 with 1 M NaOH. For solid medium add 3 g agar (EM Science) per 250 mL bottle. Aliquot 100 mL media to each 250 mL bottle, autoclave, let cool and solidify in bottles. For plate preparation, medium in bottle is melted in microwave oven, and the bottle is placed in water bath and cool to 55°C. When cooled, add spectinomycin (Sigma S-4014) to a final concentration of 50 mg/L mix well and pour the plates.

**Table 3. Maize LS-inf Medium**

| Media Components | Supplier/Catalog # | Final Concentration |
|---|---|---|
| MS (Murashige and Skoog basal media) | Sigma M-5524 | 4.3 g/L |
| Vitamin assay casamino acids (Difco) | Difco vitamin assay | 1.0 g/L |
| Glucose | Sigma G7528 | 36 g/L |
| Sucrose | Sigma S5391 | 68.5 g/L |
| 2,4-D (stock at 0.5 mg/mL) | Sigma D7299 | 1.5 mg/L |
| Nicotinic acid (stock 0.5 mg/mL) sterile | Sigma N4126 | 0.5 mg/L |
| Pyridoxine HCl (0.5 mg/mL) sterile | Sigma P8666 | 0.5 mg/L |
| Thiamine HCl (1.0 mg/mL) sterile | Sigma T4625 | 1.0 mg/L |
| Myo-inositol (100 mg/mL) sterile | Sigma I5125 | 100 mg/L |

Adjust pH to 5.2 with 1 M HCl, filter sterilize, dispense in 100 mL aliquots, add acetosyringone (100 µM) to the medium right before used for *Agrobacterium* infection (50 µL to 100 mL media - 200 mM stock).

**Table 4. Maize 1.5LSAs Medium (for co-cultivation)**

| Media Components | Supplier/Catalog # | Final Conc. |
|---|---|---|
| MS (Murashige and Skoog basal media) | Sigma M-5524 | 4.3 g/L |
| Glucose | Sigma G7528 | 10 g/L |
| Sucrose | Sigma S5391 | 20 g/L |
| 2,4-D (stock at 0.5 mg/mL) | Sigma D7299 | 1.5 mg/L |
| Nicotinic acid (stock 0.5 mg/mL) sterile | Sigma N4126 | 0.5 mg/L |
| Pyridoxine HCl (0.5 mg/mL) sterile | Sigma P8666 | 0.5 mg/L |
| Thiamine HCl (1.0 mg/mL) sterile | Sigma T4625 | 1.0 mg/L |
| Myo-inositol (100 mg/mL) sterile | Sigma I5125 | 100 mg/L |
| L-proline (stock 350 mg/mL) | Sigma P5607 | 700 mg/L |
| MES (stock 250 mg/mL) | Sigma M3671 | 500 mg/L |

Adjust pH media to 5.8 with 1 M NaOH. Weigh 4 g Sigma Purified Agar per bottle (8g/L) and dispense 500 mL media per bottle, autoclave. When cooled add AgNO3 (stock at 15 mM) to a final concentration of 15 µM and L-cysteine (stock at 150 mg/ml) to a final concentration 300 mg/l. Pour into 100 x 20 mm Petri plates. Medium containing acetosyringone should be used freshly without long-term storage.

**Table 5. Maize Recovery Medium: IM medium**

| Media Components | Supplier/Catalog # | Final Conc. |
|---|---|---|
| MS(Murashige and Skoog basal media) | Sigma M-5524 | 4.3g/L |
| Sucrose | Sigma S5391 | 30g/L |
| 2,4D(stock 0.5mg.ml) | Sigma D7299 | 1.5mg/mL |
| Casein hydrolysate | V919638 | 100mg/L |
| Proline | Sigma P5607 | 2.9g/L |

Measure ∼ ¾ of the total volume ddH₂O desired, add sucrose and salts, and dissolve under stirring. After all ingredients are dissolved, adjust to final volume with ddH₂O and to pH 5.8 using 1M KOH. Aliquot 500 mls of liquid medium into a 1L bottle with 0.9 g gelrite, autoclave for 20 minutes (liquid cycle). After autoclaving place bottles into a water-bath to cool to 55°C and add MS Vitamins (to a final concentration of 1.0 mg/mL), silver nitrate (to final concentration of 15 µM) and Timentin (to final concentration of 150 mg/L). Pour media into 100 X 20 mm petri plates and allow media to remain in the laminar hood overnight to prevent excess condensation.

**Table 6a. Selection Media**

| Media Components | Supplier/Catalog # | Final Concentration |
|---|---|---|
| MS (Murashige and Skoog basal media) | Sigma M-5524 | 4.3 g/L |
| Sucrose | Sigma S5391 | 20 g/L |
| 2,4-D (stock at 2.0 mg/mL) | Sigma D7299 | 0.5 mg/L |
| Nicotinic acid (stock 0.5 mg/mL) sterile | Sigma N4126 | 0.5 mg/L |
| Pyridoxine HCl (0.5 mg/mL) sterile | Sigma P8666 | 0.5 mg/L |
| Thiamine HCl (1.0 mg/mL) sterile | Sigma T4625 | 1.0 mg/L |
| Myo-inositol (100 mg/mL) sterile | Sigma I5125 | 100 mg/L |
| L-proline (stock 350 mg/mL) | Sigma P5607 | 700 mg/L |
| MES (stock 250 mg/mL) | Sigma M3671 | 500 mg/L |

Adjust pH of media to pH 5.8 with 1 M NaOH. Add Sigma Purified Agar (8g/L), dispense 500 mL medium per 1 L bottle, autoclave, when cooled add (Table 6b):

| Medium type | Post autoclaving components | Supplier/Catalog # | Final Concentration |
|---|---|---|---|
| Selection with Pursuit | Timentin (stock at 200 mg/ml) | Bellamy DS | 150 mg/L |
| | Pursuit (stock at 1 mM) | AC263, 499 | 500 nM |
| | Picloram (2 mg/mL) | Sigma Z0876 | 2 mg/L |
| Selection with D-Serine | Timentin (stock at 200 mg/mL) | Bellamy DS | 150 mg/L |
| | D-Serine (Stock at 1M) | AlfaAesar A11353 | 10 mM |
| | Picloram (2 mg/mL) | Sigma Z0876 | 2 mg/L |

**Table 7a. Maize Regeneration Media**

| Media Components | Supplier/Catalog # | Final Concentration |
|---|---|---|
| MS (Murashige and Skoog basal media) | Sigma M-5524 | 4.3 g/L |
| Sucrose | Sigma S5391 | 20 g/L |
| Nicotinic acid (stock 0.5 mg/mL) sterile | Sigma N4126 | 0.5 mg/L |
| Pyridoxine HCl (0.5 mg/mL) sterile | Sigma P8666 | 0.5 mg/L |
| Thiamine HCl (1.0 mg/mL) sterile | Sigma T4625 | 1.0 mg/L |
| Myo-inositol (100 mg/mL) sterile | Sigma I5125 | 100 mg/L |
| L-proline (stock 350 mg/mL) | Sigma P5607 | 700 mg/L |
| MES (stock 250 mg/mL) | Sigma M3671 | 500 mg/L |

Adjust pH media to 5.8 with 1 M NaOH. Weigh 4 g Sigma Purified Agar (Sigma A7921) per bottle (8g/L). Dispense 500 mL media per bottle, autoclave and let solidify in bottles. For use, microwave to melt media, when cooled, add (Table 7b):

| Type of media | Post autoclaving components | Supplier/Catalog # | Final Concentration |
|---|---|---|---|
| Regeneration medium with Pursuit | Timentin (200 mg/mL) | Bellamy DS | 150 mg/L |
| | Pursuit (stock at 1 mM) | AC263, 499 | 500 nM |
| | Zeatin (stock at 5 mg/mL) | Sigma Z0876 | 2.5 mg/L |
| Regeneration medium with D-Serine | Timentin (200 mg/mL) | Bellamy DS | 150 mg/: |
| | D-Serine (stock at 1mM) | AlfaAesar A11353 | 15 mM |
| | Zeatin (stock at 5 mg/mL) | Sigma Z0876 | 2.5 mg/L |

Pour into 100 x 20 mm Petri plates

**Table 8. Maize Rooting Media**

| Media Components | Supplier/Catalog # | Final Concentration |
|---|---|---|
| ½ MS (Murashige and Skoog basal media) | Sigma M-5524 | 2.15g/L |
| Sucrose | Sigma S5391 | 20 g/L |
| Nicotinic acid (stock 0.5 mg/mL) sterile | Sigma N4126 | 0.5 mg/L |
| Pyridoxine HCl (0.5 mg/mL) sterile | Sigma P8666 | 0.5 mg/L |
| Thiamine HCl (1.0 mg/mL) sterile | Sigma T4625 | 1.0 mg/L |
| Myo-inositol (100 mg/mL) sterile | Sigma I5125 | 100 mg/L |
| L-proline (stock 350 mg/mL) | Sigma P5607 | 700 mg/L |
| MES (stock 250 mg/mL) | Sigma M3671 | 500 mg/L |

Adjust pH of media to pH 5.8 with 1 M NaOH, add 1 g Gelrite per bottle (2g/L), dispense 500 mL media per bottle, autoclave, pour into disposable Phyatrays after adding the selection agents.

| Type of media | Post autoclaving components | Supplier/Catalog # | Final Concentration |
|---|---|---|---|
| Rooting medium with Pursuit | Timentin (200 mg/ml) | Bellamy DS | 150 mg/L |
| | Pursuit (stock at 1mM) | AC263, 499 | 500 nM |
| | Zeatin (stock at 5 mg/mL) | Sigma Z0876 | 2.5 mg/L |
| Rooting medium with D-Serine | Timentin (200 mg/mL) | Bellamy DS | 150 mg/L |
| | D-Serine (stock at 1mM) | AlfaAesar A11353 | 10 mM |
| | Zeatin (stock at 5 mg/mL) | Sigma Z0876 | 2.5 mg/L |

### 1.3 Preparation of donor plants for transformation experiments

### 1.3.1 Deposit under the Budapest Treaty

A deposit was made under the Budapest Treaty for the following material:
1. Seed of Zea mays line BPS553; Patent Deposit Designation PTA-6170.
2. Seed of Zea mays line BPS631; Patent Deposit Designation PTA-6171.

The deposit was made with the American Type Culture Collection (ATCC),

Manassas, VA 20110-2209 USA on August 26, 2004.

### 1.3.2 Preparation of hybrid donor plants

The following *Zea mays* inbred lines are employed for the following steps:
1. HiIIA: HiII parent A; deposit No.: T0940A, Maize Genetics and Genomics Database), available from Maize Genetics Cooperation - Stock Center USDA/ARS & Crop Sci/UIUC, S-123 Turner Hall, 1102 S. Goodwin Avenue, Urbana IL USA 61801-4798; http://www.maizegdb.org/stock.php.
2. A188: Agronomy & Plant Genetics, 411 Borlaug Hall, Univ of Minnesota, Saint Paul MN 55108.
3. BPS533 (ATCC Patent Deposit Designation PTA-6170).
4. BPS631 (ATCC Patent Deposit Designation PTA-6171).

F1 seeds of corn genotype HiIIAxA188 are produced by crossing HiIIA (female parent) with inbred line A188 (male), and planted in the greenhouse as pollen donor. F2 seeds of (HiIIAxA188) are produced by self-pollination of F1 (HiIIAxA188) plants either in the greenhouse or in the field, and planted in the greenhouse as the pollen donor. Hybrid immature embryos of BPS553x(HiIIAxA188) or BPS631x(HiIIAxA188) are produced using inbred line BPS553 (ATCC Patent Deposit Designation PTA-6170) or BPS631 (ATCC Patent Deposit Designation PTA-6171) as the female parents, and either F1 or F2 (HiIIAxA188) plants as the male parent in the greenhouse.

Seeds are sowed in pots containing Metromix. Once the seeds become germinated and rooted, one seedling/pot is maintained for immature embryo production, and the second seedling is discarded; alternatively seeds are started in a 4x4 inch pots, and seedlings are transplanted to 10-inch pots two weeks after sowing the seeds. Approximately one tablespoon of Osmocote 14-14-14 (a type of slow releasing fertilizer) is added to the surface of each pot. The temperature in the greenhouse is maintained at 24°C night and 28°C day. Watering is done automatically, but is supplemented daily manually as needed. Twice a week, the plants are watered with a 1:15 dilution of Peters 20-20-20 fertilizer.

### 1.3.3 Preparation of inbred donor plants

Seeds of inbred lines BPS553 or BPS631 are sown either directly in 4-inch pots, and the seedlings are transplanted to 10-inch pots two weeks after sowing the seeds. Alternatively, seeds are directly sown into 10-inch pots. Self- or sib-pollination is performed. The growing conditions are same as above for the hybrid line.

### 1.3.4 Hand-Pollination

Every corn plant is monitored for ear shoots, and when appeared, they are covered with a small white ear shoot bag (Lawson). Once the ear shoots have started to produce silks, the silks are cut and covered again with the ear shoot bag. The tassel of the same plant is bagged with a brown paper bag (providing that the tassel has entered anthesis). The next morning, the tassel is shaken to remove pollen and anthers into the bag. The bag is then removed and pollen is shaken over the silks of the ear shoot. Pollinating is done between 8 and 10 a.m. in the morning. Secure the brown paper bag over the ear shoot and around the corn stalk. After pollination, the tassel is removed from the plant to reduce pollen (allergens to many people) in the greenhouse.

To ensure synchronized pollinations for the same genotypes, and hence to avoid weekend harvesting/transformation, ear shoots of those early flowering plants are cut back again. A group of plants, *e.g.* > 5 to 10 plants are then pollinated on the same day. However, this practice is dependent on the quality/quantity of pollens on a plant. Sib-pollination is needed for the inbred lines. For instance either BPS553 or BPS631 can be either selfed or sib-pollinated between the same genotype).

### 1.3.5 Harvest and Pre-treat Ears

Ears from corn plants (the first ear that comes out is the best) are harvested 8 to 14 (average 10) days after pollination (DAP). Timing of harvest varies depending on growth conditions and maize variety. The size of immature embryos is a good indication of their stage of development. The optimal length of immature embryos for transformation is about 1 to 1.5 mm, including the length of the scutellum. The embryo should be translucent, not opaque. If the ear is ready, but cannot be used for transformation that day, the ear can be harvested, put in the pollination bag, and stored in a plastic bag in 4°C fridge for 1 to 3 days.

### 1.4 Agrobacterium mediated transformation

### 1.4.1 Preparation of Agrobacterium

*Agrobacterium* glycerol stock is stored at -80°C. Inoculums of *Agrobacterium* are streaked from glycerol stocks onto YP agar medium (A-1) containing appropriate antibiotics (e.g. 50 mg/L spectinomycin and/or 10 mg/L tetracycline, or 100 mg/l kanamycin). The bacterial cultures are incubated in the dark at 28°C for 1 to 3 days, or until single colonies are visible. The obtained plate can be stored at 4°C for 1 month and used as a master plate to streak out fresh cells. Fresh cells should be streaked onto YP agar with the appropriate antibiotic from a single colony on the master plate, at least 2 days in advance of transformation. These bacterial cultures can be incubated in the dark at 28°C for 1 to 3 days.

Alternatively frozen *Agrobacterium* stock can be prepared by streaking *Agrobacterium* cells from frozen stock to a plate B-YP-002 (YP+50 mg/L spectinomycin + 10 mg/L tetracycline), and growing at 28°C for 2 to 3 days. Save it as master plate and store at 4C for up to a month. From the master plate, streak a loop of agro cells to a flask containing 25 mL liquid B-YP-000 medium supplemented with 50 mg/L Spectinomycin + 10 mg/l tetracycline. Grow on a shaker set at 300 rpm and 28°C 2 to 3 days. Prepare frozen agro stock by mixing 1 part of the above agro culture with 1 part of sterile 30% glycerol. Vortex to mix well and dispense 10 µL the *Agrobacterium*/glycerol mixture to a 50 µL Eppendorf tube. Store at -80°C.

One loop full (2 mm in diameter) of bacterial culture is suspended in 1.0 to 1.8 mL LS-inf medium supplemented with 200 nM acetosyringone. This yields a bacterial suspension with approximate optical density (OD₆₀₀) between 0.5 to 2.0. Vortex for 0.5 to 3 hours. Vortexing is performed by fixing (e.g. with tape) the microfuge tube horizontally (instead of vertically) on the platform of a vortexer to ensure better disperse *Agrobacterium* cells into the solution. Mix 100 µL of *Agrobacterium* cell suspension with 900 uL of LS-inf solution in a curvet, and measure OD₆₀₀. Adjust OD of original *Agrobacterium* solution to 0.6 to 2.0 with LS-Inf (with 100 nM acetosyringone) solution. The *Agrobacterium* suspension must be vortexed in the LS-inf + acetosyringone media for at least 0.5 to 3 hours prior to infection. Prepare this suspension before starting harvesting embryos.

Alternatively *Agrobacterium* suspensions for corn transformation can be preparedas as follows: Two days before transformation, from -80°C stock, streak *Agrobacteria* from one tube to a plate containing B-YP-002 (solidified YP+50 mg/L spectinomycin + 10 mg/l tetracycline) and grow at 28°C in the dark for two days. About 1 to 4 hrs before transformation, place one scoop of bacterial cells to 1.5 mL M-LS-002 medium (LS-inf + 200 µM acetosyrigone) in a 2 mL Eppendorf tube. Vortex the tube to dispense the bacterial cells to solution and shake the tube at 1000rpm for 1 to 4 hrs. The OD₆₀₀ should be in the range of 0.6 to 1.0 or about 10⁸cfu/mL.

For the purpose of the following examples *Agrobacterium tumefaciens* strain LBA4404 or disarmed *Agrobacterium* strain K599 (NCPPB 2659)) transformed with binary vector plasmid pBPSMM232 were employed. pBPSMM232 contains the *ahas* gene (as selection marker) and the gus reporter gene.

### 1.4.2 Surface sterilization of corn ear and isolation of immature embryos

The ears are harvested from the greenhouse 8 to 12 days after pollination. All husk and silks are removed and ears are transported in the brown pollination bag back to the tissue culture lab. The cob is moved into the sterile hood. A large pair of forceps is inserted into the basal end of the ear and the forceps are used as a handle for handling the cob. Optionally, when insects/fungus are present on the ear, the ear should be first sterilized with 20% commercial bleach for 10 min (alternatively 30% Clorox solution for 15 min), and then rinsed with sterilized water three times. While holding the cob by the forceps, the ear is completely sprayed with 70% ethanol and then rinsed with sterile ddH₂O.

### 1.4.3 Inoculation method-1: The Modified "Tube" method

The cob with the forceps handle is placed in a large Petri plate. A dissecting scope may be used. The top portion (2/3's) of kernels are cut off and removed with a #10 scalpel (for safety consideration, the cut on the kernels is made by cutting away from your hand that holds the handle of the forceps). The immature embryos are then excised from the kernels on the cob with a scalpel (#11 scalpel): the scalpel blade is inserted on an angle into one end of the kernel. The endosperm is lifted upwards; the embryo is lying underneath the endosperm. The excised embryos are collected in a microfuge tube (or a small Petri plate) containing roughly 1.5 to 1.8 mL of *Agrobacterium* suspension in LS-inf liquid medium containing acetosyrigone (see above; A-2). Each tube can contain up to 100 embryos. The tube containing embryos is hand-mixed several times, and let the tube/plate stand at room temperature (20 to 25°C) for 30 min. Remove excess bacterial suspension from the tube/plate with a pipette. Transfer the immature embryos and bacteria in the residue LS-inf medium to a Petri plate containing co-cultivation agar medium. Transfer any immature embryos that remain in the microfuge tube by a sterile loop. Remove excess bacterial suspension with a pipette. A small amount of liquid must be left in the plate to avoid drying out the embryos while plating. Place the immature embryos on the co-cultivation medium with the flat side down (scutellum upward). Do not embed the embryos into medium. Leave the plate cover open in the sterile hood for about 15 min for evaporating excess moisture covering immature embryos. Seal the Petri dishes with 3 M micropore tape. About 100 embryos can be placed on a Petri plate for co-cultivation. Seal the plate and wrap with a sheet of aluminum foil. Incubate the plates in the dark at 22°C for 2 to 3 days. Take 3 to 5 immature embryos for GUS staining if a GUS construct is used to assess transient GUS expression.

### 1.4.4 Method-2: The "Drop" method

Excised immature embryos are directly put on the co-cultivation medium (Appendix A-3) with the flat side down (scutellum upward). Each plate (20x100 mm plate) can hold up to 100 immature embryos. Put 5 µL of diluted *Agrobacterium* cell suspension to each immature embryo with a repeat pipettor. Remove excess moisture covering immature embryos by leaving the plate cover open in the hood for about 15 min. Seal the plate with 3 M micropore tape and wrap with aluminum foil. Incubate the plate in the dark at 22°C for 2 to 3 days. Take 3-5 immature embryos for GUS staining if a GUS construct is used to assess transient GUS expression.

### 1.4.5 Recovery

After co-cultivation, transfer the embryos to recovery media (A-4) and incubate the plates in dark at 27°C for about 5 to 10 days. Keep scutellum side up and do not embed into the media.

### 1.4.6 Selection

Transfer immature embryos to 1^{st} selection media (A-6). Roughly 25 to 50 immature embryos can be placed on each plate. Be careful to maintain the same orientation of the embryos (scutellum up). Do not embed the embryos in the media. Seal the Petri plates with white tape. Incubate in the dark at 27°C for 10 to 14 days (First selection). Subculture all immature embryos that produce variable calli to 2nd selection media (A-6). Try to avoid transferring slimy or soft calli. At this stage, use scissors to remove any shoots that have formed (try to remove the entire embryo from the scutellum if possible and discard it). Firmly place the callus on the media - do not embed into the media. Wrap the plates in 3M Micropore tape and put in the dark at 27°C. Incubate for 2 weeks under the same conditions for the first selection (Second selection). Using 2 pairs of fine forceps, excise the regenerable calli from the scutellum under a stereoscopic microscope. The regenerable calli is whitish/yellowish in color, compact, not slimy and may have some embryo-like structures. Transfer calli to fresh the 2nd selection media (A-6), wrap in 3M Micropore tape and incubate in the dark at 27°C for 2 weeks. Firmly place the callus on the media - do not embed into the media. Be careful to group and mark the calli pieces that came from the same embryo.

### 1.4.7 Regeneration and transplanting of transformed plants

Excise the proliferated calli (whitish with embryonic structures forming), in the same manner as for 2^{nd} selection and transfer to regeneration media (A-7) in 25x100 mm plates. Firmly place the callus on the media - do not embed into the media. Wrap the plates in 3M Micropore tape and put in the light at 25 or 27°C. Be careful to group the calli pieces that came from the same embryo and number them by embryo.

Incubate under light (ca. 2,000 lux; 14/10hr light/dark) at 25 or 27°C for 2 to 3 weeks, or until shoot-like structures are visible. Transfer to fresh regeneration media if necessary. Transfer calli sections with regenerated shoots or shoot-like structures to a Phytatray or Magenta boxes containing rooting medium (A-8) and incubate for 2 weeks under the same condition for the above step, or until rooted plantlets have developed. After 2 to 4 weeks on rooting media, transfer calli that still have green regions (but which have not regenerated seedlings) to fresh rooting Phytatrays. Seedling samples are taken for TaqMan analysis to determine the T-DNA insertion numbers.

Transfer rooted seedlings to Metromix soil in greenhouse and cover each with plastic dome for at least 1 week, until seedlings have established. Maintain the plants with daily watering, and supplementing liquid fertilizer twice a week. When plants reach the 3 to 4 leaf-stages, they are fertilized with Osmocote. If needed putative transgenic plants containing ahas gene are sprayed with 70 to 100 g/ha Pursuit^{™} by a licensed person, and grown in the greenhouse for another two weeks. Non-transgenic plants should develop herbicidal symptoms or die in this time. Survived plants are transplanted into 10" pots with MetroMix and 1 teaspoon Osmocote^{™}.

At the flowering stage, the tassels of transgenic plants are bagged with brown paper bags to prevent pollen escape, and the ear shoots are also covered with the ear bag for preventing pollen contamination. Pollination is performed on the transgenic plants. It is best to do self-pollination on the transgenic plants. If silking and anthesis are not synchronized, a wild-type pollen donor or recipient plant with same genetic background as the transgenic T₀ plant should be available for performing cross-pollination. T₁ seeds are harvested, dried and stored properly with adequate label on the seed bag. After harvesting the transgenic T₁ seeds, T₀ plants including the soil and pot should be bagged in autoclave bags and autoclaved (double bagging).

### 1.5 Identification of single copy transgenic lines (T0) showing high expression of transgene

### 1.5.1 Identification of single copy lines

Single copy lines were identified using TaqMan copy assays (Applied Biosystems Catalog # 4326270).

### 1.5.2 Identification of high expressing lines for transgenes at the mRNA levels

### 1.5.2.1 Sampling

The nucleic acid samples that were used to determine copy number (see Example 1.5.1, above) were used to assay for transgene (pCER040b & pCER041:I-SceI, pJB034:GU-US, and JB039:GUS) mRNA expression levels.

The T0 leaf nucleic acid samples used for copy number analysis were DNase treated using the DNA-free kit from Ambion (catalog #1906), as described by the manufacturer.

### 1.5.2.2 Expression Analysis Reaction Set-Up

Once samples have been treated with DNase, expression analysis was performed. For analysis of each sample, two reactions were run, one for the gene of interest (either the NOS terminator, GUS reporter gene or I-SceI gene) and one for an endogenous gene control used to quantify RNA concentration in the reaction. The expression of the endogenous gene should remain constant throughout assay conditions so as to accurately reflect relative concentration of the gene of interest. For these experiments a maize gene was identified that shows stable expression levels under normal greenhouse growth conditions (BPS-NC clone ID 62054718). Primers 12 and 13 were used to analyze expression levels of this gene.
SEQ ID NO: 13: Primer 12 (Forward primer Endo):
   5'-TCTGCCTTGCCCTTGCTT-3'
SEQ ID NO: 14: Primer 13 (Reverse primer Endo):
   5'-CAATTGCTTGGCAGGTCTTATTT-3'

The NOS terminator primers anneal before the transcriptional stop in the terminator. The sequences of the primers are below.
SEQ ID NO: 15: Primer 14 (Forward primer NOS):
   5'-TCCCCGATCGTTCAAACATT-3'
SEQ ID NO: 16: Primer 15 (Reverse primer NOS):
   5'-CCATCTCATAAATAACGTCATGCAT-3'

The GUS reporter gene primers anneal in the middle of the gene sequence. The sequences of the primers are below.
SEQ ID NO: 17: Primer 16 (Forward primer GUS):
   5'-TTACGTGGCAAAGGATTCGAT-3'
SEQ ID NO: 18: Primer 17 (Reverse primer GUS):
   5'-GCCCCAATCCAGTCCATTAA-3'

The I-SceI gene primers anneal within the open reading frame. The sequences of the primers are below.
SEQ ID NO: 19: Primer 18 (Forward primer I-SceI):
   5'- GACCAGGTATGTCTGCTGTACGA-3'
SEQ ID NO: 20: Primer 19 (Reverse primer I-Scel):
   5'- CAGGTGGTTAACACGTTCTTTTTT-3'

The reactions were run in a 96-well optical plate (Applied Biosystems, 4314320), with endogenous control and gene of interest reactions run on the same plate simultaneously. Semi-quantitiave RT-PCR using SYBR Green (Eurogentec #RTSNRT032X-1) was performed on the samples using standard procedures known in the art. Reactions were performed on the Perkin Elmer GeneAmp 5700 (serial # 100001042), as described by the manufacturer.

The thermocycler parameters used were as follows:
Stage 1: 30 min at 48°C (Reps: 1)
Stage 2: 10 min at 95°C (Reps:1)
Stage 3: 15 sec at 95°C and 1 min at 60°C (Reps:40)

The default dissociation protocol was used:
15 sec at 95°C
20 sec at 60°C
20 min, 35°C slow ramp (60-95°C)

### 1.5.2.3 Data Analysis Results on the GeneAmp5700 for Transgene

The results of the endogenous control reactions were used to confirm the quality and integrity of mRNA samples. Transgene expression was categorized as high, medium, or low in the T0 generation Maize plants based on Ct values generated by GeneAmp5700. High level were regarded as Ct values in the range of 18 to 23, medium levels (Ct values: 24 to 26), low levels (Ct values: 26 to 30). Samples that produced Ct values above 30 were considered to show no transgene expression. Table 9 shows a summary of the number of transgenic lines for each construct, grouped by experimentally determined mRNA expression levels.

**Table 9. Number of T0 plants in each category of expression level for each transgene construct.**

| Construct | **High** | Medium | Low | Total tested |
|---|---|---|---|---|
| pJB034 | 36 | 28 | 19 | 83 |
| pJB039 | 56 | 50 | 18 | 124 |
| pJBcer040b | 20 | 2 | 2 | 24 |
| pJBcer041 | 20 | 38 | 29 | 87 |

### 1.6 Proof of concept on double strand break (DSB)-mediated homologous recombination via semi-transient assay system

### 1.6.1 Transient expression assay for proof of concept on DSB-mediated recombination

A transient expression assay was used to provide proof of concept data for the DSB-mediated homologous recombination system in plant cells. A GU-US reporter construct (e.g. pJB034) was introduced to maize leaf tissue or protoplasts via biolistic bombardment or PEG-mediated transformation, respectively. The functional GUS open reading frame can only be generated from pJB034 upon homologous recombination of the GU-US locus. The results from these experiments are summarized in Table 10 and in Figure 4. GUS staining was detected at significantly higher levels when leaf tissue from I-*Sce*I-expressing maize plants was bombarded with pJB034 as compared with maize leaves that did not express I-*Sce*I.

**Table 10. GUS staining results from transient bombardment assays**

| | pJB035 (GUS) | pJB034 (GU-US) |
|---|---|---|
| WT maize | +++ | - |
| CER040b transgenic maize | +++ | ++ |
| CER041 transgenic maize | +++ | + |

### 1.6.2 Biolistic transformation

The plasmid constructs are isolated using Qiagen plasmid kit (cat# 12143). DNA is precipitated onto 0.6 µM gold particles (Bio-Rad cat# 165-2262) according to the protocol described by Sanford *et al.* (1993) and accelerated onto target tissues *(e.g.* two week old maize leaves, BMS cultured cells, *etc.)* using a PDS-1000/He system device (Bio-Rad). All DNA precipitation and bombardment steps are performed under sterile conditions at room temperature.

Two mg of gold particles (2 mg/3 shots) are resuspended in 100% ethanol followed by centrifugation in a Beckman Microfuge 18 Centrifuge at 2,000 rpm in an Eppendorf tube. The pellet is rinsed once in sterile distilled water, centrifuged, and resuspended in 25 µL of 1 µg/µL total DNA. The following reagents are added to the tube: 220 µL H₂0, 250 µL 2.5M CaCl₂, 50µL 0.1M spermidine, freebase. The DNA solution is briefly vortexed and placed on ice for 5 min followed by centrifugation at 500 rpm for 5 min in a Beckman Microfuge 18 Centrifuge. The supernatant is removed. The pellet is resuspended in 600 µL ethanol followed by centrifugation for 1 min at 14,000 rpm. The final pellet is resuspended in 36 µL of ethanol and used immediately or stored on ice for up to 4 hr prior to bombardment. For bombardment, two-week-old maize leaves are cut in approximately 1 cm in length and located on 2 inches diamenter sterilized Whatman filter paper. In the case of BMS cultured cells, 5 mL of one-week-old suspension cells are slowly vacuum filtered onto the 2 inches diameter filter paper placed on a filter unit to remove excess liquid. The filter papers holding the plant materials are placed on osmotic induction media (N6 1-100-25, 0.2 M mannitol, 0.2 M sorbitol) at 27°C in darkness for 2-3 hours prior to bombardment. A few minutes prior to shooting, filters are removed from the medium and placed onto sterile opened Petri dishes to allow the calli surface to partially dry. To keep the position of plant materials, a sterilized wire mesh screen is laid on top of the sample. Each plate is shot with 10 µL of gold-DNA solution once at 2,200 psi for the leaf materials and twice at 1,100 psi for the BMS cultured cells. Following bombardment, the filters holding the samples are transferred onto MS basal media and incubated for 2 days in darkness at 27°C prior to transient assays. Determine transient expression levels of the reporter gene following the protocols in the art as described above.

### 1.6.3 Protoplast transfection

Isolation of protoplasts is conducted by following the protocol developed by Sheen (1990). Maize seedlings are kept in the dark at 25°C for 10 days and illuminated for 20 hours before protoplast preparation. The middle part of the leaves are cut to 0.5 mm strips (about 6 cm in length) and incubated in an enzyme solution containing 1 % (w/v) cellulose RS, 0.1% (w/v) macerozyme R10 (both from Yakult Honsha, Nishinomiya, Japan), 0.6 M mannitol, 10 mM Mes (pH 5.7), 1 mM CaCl₂, 1 mM MgCl₂, 10 mM β-mercaptoethanol, and 0.1% BSA (w/v) for 3 hr at 23°C followed by gentle shaking at 80 rpm for 10 min to release protoplasts. Protoplasts are collected by centrifugation at 100 x g for 2 min, washed once in cold 0.6 M mannitol solution, centrifuged, and resuspended in cold 0.6 M mannitol (2 x 10⁶/mL).

A total of 50 µg plasmid DNA in a total volume of 100 µL sterile water is added into 0.5 mL of a suspension of maize protoplasts (1 x 10⁶ cells/mL) and mix gently. 0.5 mL PEG solution (40 % PEG 4000, 100 mM CaNO₃, 0.5 mannitol) is added and prewarmed at 70°C with gentle shaking followed by addition of 4.5 mL MM solution (0.6 M mannitol, 15 mM MgCl₂, and 0.1 % MES). This mixture is incubated for 15 minutes at room temperature. The protoplasts are washed twice by pelleting at 600 rpm for 5 min and resuspending in 1.0 mL of MMB solution [0.6 M mannitol, 4 mM MES (pH 5.7), and brome mosaic virus (BMV) salts (optional)] and incubated in the dark at 25°C for 48 hr. After the final wash step, collect the protoplasts in 3 mL MMB medium, and incubate in the dark at 25 °C for 48 hr. Determine transient expression levels of the reporter gene following the protocols in the art.

### 1.7 Recovery marker-free Transgenic plants through direct conversion, and through callus culture of F1 hybrid immature embryos

After crossing a transgenic plant containing selection marker bordered by excision sites, and a second transgenic plant containing I-SceI gene, each of the F1 progenies (embryos/seeds) may have: (1) all of the cells in an embryo with intact GOI and selection marker (cell with intact selection marker); (2) all of the cells with GOI, but without selection marker (full marker excision occurred); and (3) some of the cells have the selection marker excised resulting in a mixed genotype.

Depending on the stage excision occurs during and after pollination, there are possibly different genotypes in an embryo/seed. If selection marker excision occurs right at the single cell stage of pollination, a fully excised plant is expected. However, if marker excision event occurs at a later - multi-cell stage during embryo development, one zygote/embryo may contain mixed cell types - cells with and without selection marker.

Conventionally, mature seeds of F1 progeny are harvested, and planted in soil to obtain individual seedlings for future screening. Molecular techniques, such as PCR analysis, and Southern blot analysis are applied to identify the full excision plant.

### 1.7.1 Screening based on the conversion of F1 immature embryos

In order to save time as compared to the conventional approach through screening mature seeds, a process of converting F1 immature embryos on the rooting medium (A-8) containing the selection agent against the selection marker linked to I-Sce-I gene is applied. For instance, D-serine is applied in the rooting medium if dsdA selection marker is used for generating I-Sce-I plant. Immature embryos are dissected and placed onto the rooting medium (A-8), and then incubated at 27°C chamber with 16 hr photoperiod. Seedlings recovered are then subjected to molecular screening for identifying the full marker excision plant. For a comparison between the conventional method and the method of immature embryo conversion, about 80 days are saved with the application of immature embryos conversion assuming both methods have the same rate of obtaining full excision events (Table 11).

### 1.7.2 Screening based on the callus culture of F1 immature embryos

For increasing the chance of obtaining the full excision event, a process of culturing F1 immature embryos is applied in this invention.

As compared with the approaches of screening F1 plants derived from the mature seeds and from immature embryos, we hypothesize that cell division associated with embryogenic callus culture of immature embryos promotes the activities of marker excision due to the active DNA multiplication and repair activities in the embryogenic callus initiation process. Since regeneration through corn embryogenic callus culture is single cell-based process, a regenerated seedling, therefore, contains a single cell type - e.g. either full excised genotype or non-excised genotype. For example, if the excision occurs only in the scutellum in the F1 immature embryo/seed, it is impossible to recover the full excision plant through screening plants derived from mature seeds or converted immature embryos. In contrast, callus culture of chimeric immature embryo may result in recovering the full excision plant.

**Table 11. Comparison of time required for conventional and tissue culture regeneration approaches in obtaining a full marker excision plant.**

| | Conventional method (through mature seeds) | Time required (days) | Regenerating plants through callus culture from F1 immature embryos | Time required (days) | Recover a full marker excision plant through immature embryo conversion | Time require (days) |
|---|---|---|---|---|---|---|
| Step1 | Producing F1 progeny between excision target and I-SceI parents by crossing pollination | 70 | Producing F1 progeny between excision target and I-SceI parents by crossing pollination | 70 | Producing F1 progeny between excision target and I-SceI parents by crossing pollination | 70 |
| Step2 | Obtaining mature F1 seeds | 50 | Obtaining immature embryos (1.0-1.8 mm) | 10 | Obtaining immature embryos (2 - 4 mm) | 14 |
| Step 3 | Germinating seeds to obtain F1 seedlings | 14 | Regenerating plants from scutulum of immature embryo through tissue culture | 60 | Generating plants from immature embryos via embryo conversion | 14 |
| Step 4 | Selecting marker-free plants | 7 | Identifying marker-free plants | 7 | Selecting marker free plants | 7 |
| Step 5 | Making F2 progeny | 120 | | | Making F2 immature embryos | 70 |
| Step 6 | Germinating F2 seeds to obtain F2 seedlings | 14 | | | Generating F2 plants from immature embryos via embryo conversion | 14 |
| Step 7 | Identifying marker-free plants | 7 | | | Identifying marker-free plants | 7 |
| | Total | 277 | | 147 | | 196 |

F1 immature embryos of 1 to 1.8 mm in length are dissected onto the recovery medium (A-4, IM medium), and incubated at 27°C in dark for about 5 to 10 days, and then the derived calli are transferred to and cultured on the selection medium containing the selection agent against the second selection marker gene linked with Sce-I gene for about 14 days. The calli are further cultured on the selection medium for another 14 days, and then transferred to the regeneration medium (A-7) and incubated in a tissue culture chamber under 16 hr/day photoperiod for about 7 to 14 days. The regenerated plants are then transferred to the rooting medium (A-8) under the same condition as the regeneration step. The seedlings are then subjected to molecular screening for identifying the full marker-excision plant.

### 1.7.3 Plant analysis for DSB-mediated homologous recombination or marker excision

### 1.7.3.1 GUS histochemical assay

One method that was used to monitor recombination events was histochemical GUS staining in leaf and kernel tissues from pJB034-containing plants. The pJB034 construct comprises an interrupted β-glucuronidase (GUS) reporter gene containing an internal partial sequence duplication such that the functional open reading frame can only be reconstituted via homologous recombination between the repeated sequence. The expression of functional GUS protein in plant tissues can be visualized by means of a chromogenic substrate such as 5-bromo-4-chloro-3-indolyl-β-D-glucuronic acid using methods known to those in the art.

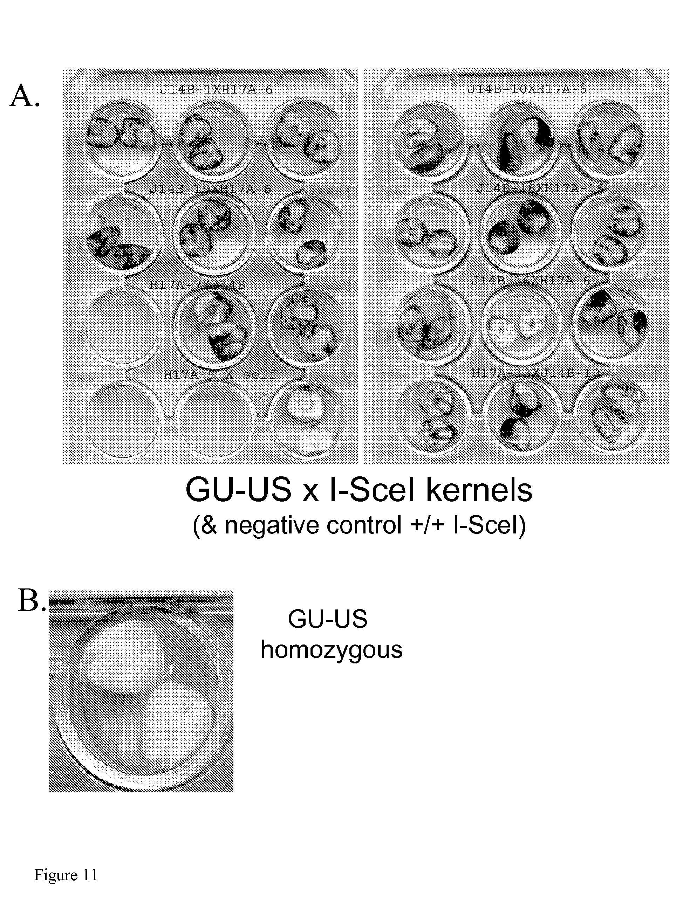

Plant tissues from [JB034 x I-*Sce*I] and [JB034 x I-*Sce*I NULL] plants were analyzed for GUS expression via histochemical staining. The results are summarized in Table 12 and Figure 11. Tissues from plants generated via JB034 x I*-Sce*I crosses showed significantly more GUS expression in leaf and kernels than tissues from plants generated from JB034 x NULL crosses. Crosses with null plants *(i.e.* plants that do not express I*-Sce*I) showed no detectable GUS staining.

For all combinations, reciprocal crosses were performed with regard to the maternal or paternal transmission of the I*-Sce*I and JB034 constructs; no quantitative or qualitative differences in GUS expression were detectable between maternally or paternally supplied transgenes. Larger amounts of GUS staining were generally observed in plants derived from crosses with CER040b (pUbi::I-*Sce*I) as compared with CER041 (ScBV::I-*Sce*I) plants.

**Table 12. Results of GUS staining of tissues from JB034 X I-SceI plants**

| JB034 crossed with: | GUS Staining | Comments |
|---|---|---|
| JB034 (self) | - | No staining in any tissues |
| CER040b (Ubi::I-SceI) | +++ | Strong staining in leaves and kernels (endosperm and scutellum) |
| CER041 (ScBV::I-SceI) | ++ | Strong staining in leaves and less intense staining in kernels (endosperm) |
| Null | - | No staining in any tissues |

Leaf tissue from different JB034 x I-*Sce*I plants varied in the amount of GUS staining that was visualized: from spotty GUS staining representing recombination events that happened relatively late in the leaf development, streaks of GUS staining representing tissue developed from cells that had previously undergone recombination, to fully blue leaves representing recombination events that occurred at a developmental stage preceding leaf formation. Since recombination occurs at the cellular level, it was possible that different leaves from the same plant would yield different GUS staining patterns.

### 1.7.3.2 PCR analysis for marker excision

PCR was used to provide molecular characterization of recombination events from plants comprising either pJB034 or pJB039 reporter constructs.

Plants from JB034 crosses were analyzed by PCR using primers 7 and 8, which are based in the ScBV promoter and a region of the GUS ORF that is downstream of the repeated region, respectively.
SEQ ID NO: 21: Primer 7 (ScBV fwd):
   5'-GATCGCAGTGCGTGTGTGACACC-3'
SEQ ID NO: 22: Primer 8 (GUS AS879 rev):
   5'-GTCCGCATCTTCATGACGACC-3'

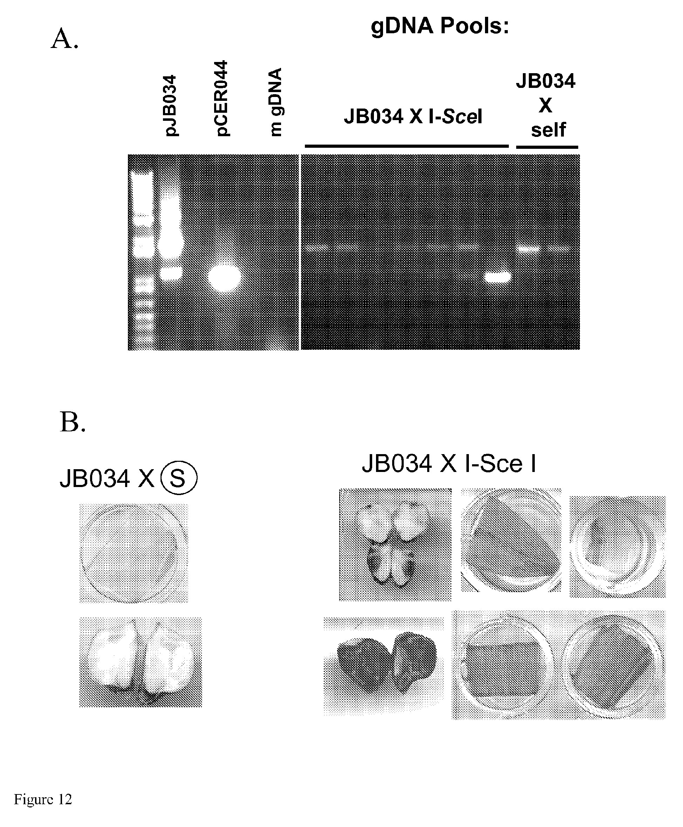

In order to maximize assay throughput, genomic DNAs were grouped into pools for PCR analyses. PCR amplification with primers 7 and 8 from the native JB034 construct yields a 1.7 kb product, while amplification from recombined JB034 generates a 1.0Kb PCR product. Figure 12 shows that PCR performed with genomic DNA from [JB034 x I-SceI] plants yielded both the 1.7 kb and 1.0 kb products; when the template was genomic DNA from selfed JB034 results in generation of only the 1.7 kb product expected from the unrecombined JB034 locus. Individual plants from the genomic DNA pools that yielded the excision-specific PCR product were subsequently analyzed separately.

Plants from JB039 crosses were analyzed by PCR using primers 9 and 10, which are based in the AHAS open reading frame and the region adjacent to the T-DNA Right border, respectively. PCR with primers 9 and 10 should result in a 6.7 kb product from a native JB039 template, and a 0.9 kb product from the proposed recombined JB039. PCR was carried out under conditions that would not allow efficient amplification of the 6.7 kb native product, so in order to confirm that the genomic DNA was intact for all samples, an additional PCR was performed in order to provide an easily amplifiable product from genomic DNA comprising the native JB039 construct. This confirmatory PCR used primers 9 and 11 generates a 1.2 kb product from native JB039, and no product from recombined JB039, due to the loss of the primer 11 homologous sequence.
SEQ ID NO: 23: Primer 9 (AHAS ORF fwd):
   5'-CTAATGGTGGGGCTTTCAAGG
SEQ ID NO: 24: Primer 10 (RB proximal rev):
   5'-CCTTAAGGCGATCGCGCTGAGGC
SEQ ID NO: 25: Primer 11 (distal AHAS term rev):
   5'-AGTGTACGGAATAAAAGTCC

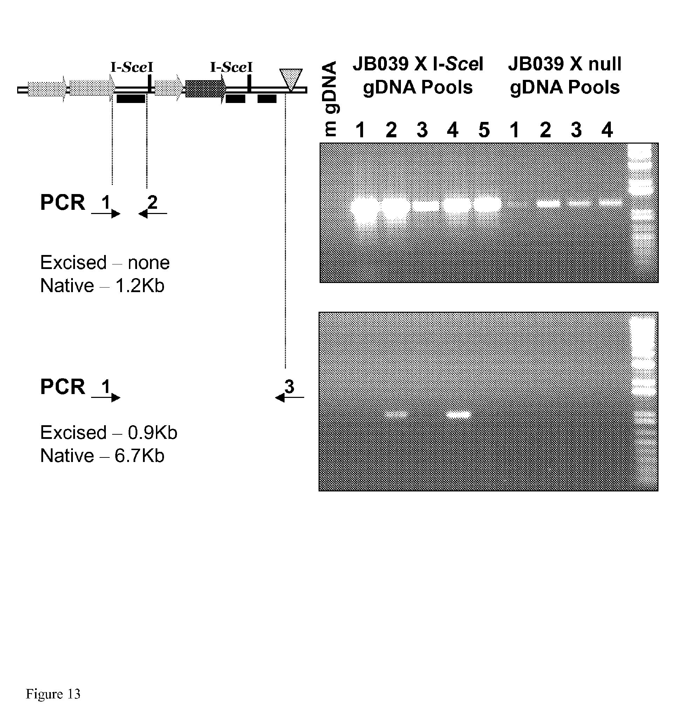

In order to efficiently analyze genomic DNA from as many plants as possible, the plant genomic DNAs from [JB039 x I-SceI] or [JB039 x null] plants were pooled and initially assayed as such. Figure 13 shows typical results of these PCR analyses. Individual plants from the genomic DNA pools that yielded the excision-specific PCR product were subsequently analyzed separately.

### 1.7.3.3 Plant analysis for marker excision

A series of 17 crosses were generated for the GU-US reporter construct (JB034). A total of 369 events from 8 crosses were obtained with the Ubiquitin I-SceI construct (CER40b: Zm.ubiquitin promoter::Zm.ubiquitin intron::I-SceI::NOS terminator) and 520 events from 9 crosses obtained with the ScBV I-SceI construct (CER41: ScBV promoter::I-SceI::NOS terminator). Histochemical screening of the Ubiquitin I-SceI cross produced 118 positive recombination events indicated by blue streaks and spots, an average of 15 recombined events per cross. PCR analysis of 354 of these events produced 58 positive PCR products, an average of 7 recombined events per cross. When the crosses were made using the ScBV I-SceI 14 histochemically positive events out of 520 were obtained, an average of 2 recombined events per cross. The PCR analysis identified 28 positive PCR products out of 516 events, an average of 4 recombined events per cross (Table 3). The weaker ScBV promoter resulted in fewer recombination events born out by both histochemical and PCR screening methods. A JB034 crossed to an I-SceI null line yielded 93 events of which 1 was histochemically and PCR positive. The positive event is possibly a spontaneous recombination event or the result of an error.

A similar series of crosses were made for the JB039 lines and the two I-SceI constructions. The 4 Pseudo GUS x Ubiquitin I-SceI crosses yielded 188 events of which 18 produced a positive PCR product, an average of 5 positive events per cross. The other 7 crosses using the ScBV-I-SceI construct yielded 434 events of which only 8 were PCR positive, an average of 1 recombined event per cross (Table 13). A subset of the events was histochemically stained however no white streak or spots could be distinguished in the intense blue background so further staining was abandoned. As with the interrupted GUS construct the use of the weaker ScBV promoter resulted in approximate 4 fold lower recombination events.

**Table 13. Molecular screening results from all regenerated plants. Positive¹ recombination was indicated by either blue spots or streaks and positive PCR events were indicated by an appropriate sized band. All results were chimeric in nature due to the pooling of tissue from same event and/or the incomplete excision obtained within any individual plant. The average² is expressed as events per cross.**

| GU-USxUbq-SceI 8 Crosses | Stained | PCR | Pseudo GUSxUbq-SceI 4 Crosses | PCR |
|---|---|---|---|---|
| Total Events | 369 | 354 | Total Events | 118 |
| Positive¹ | 118 | 58 | Positive | 18 |
| Average² | 15 | 7 | Average | 5 |

| GU-USxScBV-SceI 9 Crosses | Stained | PCR | Pseudo GUSxScBV-SceI 7 Crosses | |
|---|---|---|---|---|
| Total Events | 520 | 516 | Total Events | 434 |
| Positive | 14 | 28 | Positive | 8 |
| Average | 2 | 4 | Average | 1 |

| GU-USxI-SceI null 1 Cross | Stained | PCR | Pseudo GUSx I-SecI null 4 Crosses | |
|---|---|---|---|---|
| Total Events | 93 | 93 | Total Events | 172 |
| Positive | 1 | 1 | Positive | 0 |

A total of 132 out of 834 regeneration events containing the GU-US reporter construct, JB034 stained positive for recombination while 86 out of 870 events produced a PCR band indicative of the recombined gene. Of these positive events 50 were positive for both criteria (Table 14). The one positive event obtained in the I-SceI null cross was either a spontaneous event or an error.

**Table 14. Summary of events obtained with regeneration and screening results. Summary of the events obtained from each type of cross analyzed either histochemically or with PCR. GU-US events screened differed for each test as tissue for every plant for the histochemical analysis was not always available at the time of event sampling. The histochemical analysis of the excision events was discontinued and PCR analysis was performed exclusively.**

| Cross | Total # Events GUS Histochemical assays tested | # Events Stain | Total # Events PCR tested | # Events PCR + | # Events both Stained & PCR+ |
|---|---|---|---|---|---|
| JB034xI-SceI | 834 | 132 | 870 | 86 | 50 |
| JB034xI-SceInull | 93 | 1 | 93 | 1 | 1 |
| JB039xI-SceI | 28 | 0 | 622 | 26 | |
| JB039xI-SceInull | 76 | 0 | 172 | 0 | |

### 1.7.4 Retransformation strategy for producing plants with both I-SceI and excision target

We also evaluated another approach of obtaining putative marker excision events: re-transforming the excision target plant with a construct containing the I-SceI gene with a second selection marker. Since the tissue culture process may promote the excision activities (hypothesis), re-transformation experiments were conducted with several testing constructs (HEN constructs: JB084, LM319 or LM320).

To perform the re-transformation experiments, we followed the transformation procedure described above using the excision target plant (with *AHAS* as the selection marker) as the transformation donor material, and applying the selection agent against the selection cassette for the second transformation construct (e.g. D-Ser for dsdA gene).

When we used a strong, constitutive, ubiquitous promoter to express the HEN gene and GU-US, no DSB-mediated HR, (indicated by blue spots) was detected in embryo axis in maize upon analysis of mature kernel. To achieve DSB-mediated HR in embryo, the super promoter was chosen, since this promoter in maize shows strong expression in the whole embryo (scutellum and embryo axis) during germination, calli (including embryogenic calli) during regeneration. The expression levels in these tissues can be enhanced by addition of an intron-mediated enhancement (IME)-conferring intron between the super promoter and I-*Sce*I gene.

### 1.7.4.1 Embryo-specific promoter

In order to maximize I-*Sce*I expression in the embryos of developing maize kernels, vectors were generated that comprise the expression cassettes wherein the I-*Sce*I gene is driven by the super promoter, a promoter that has been described to drive high levels of expression in these tissues.

Vector pJB082 is a pUC based vector that comprises a p-Super::I-*Sce*I::t-Nos expression cassette, and was generated by the 3-way ligation of the T4 DNA polymerase filled in *Hin*dIII-*Bgl*II super promoter fragment from pLM266, the T4 DNA polymerase filled in *Asc*I-*Sb*I fragment of pJB010, and the T4 DNA polymerase filled in *Asc*I fragment of pCER039. How was JB084 finally assembled?

The binary vector pLM319 comprises the p-Super::I-*Sce*I::t-Nos cassette, and was generated by ligation of the T4 DNA polymerase filled in *Pac*I-*Pme*I fragment of pJB082 into T4 DNA polymerase filled in *Asc*I digested pLM151.

An expression cassette comprising p-Super::I-Ubi::I-*Sce*I::t-Nos was generated in a pUC vector backbone by ligating in the T4 DNA polymerase filled in *Bgl*II-*Asc*I ubiquitin intron fragment from pLM303 into the T4 DNA polymerase filled in *Sph*I digested pJB082, thereby generating pJB083. The binary vector pLM320 was generated by ligation of the T4 DNA polymerase filled in *Pac*I-*Pme*I p-Super::I-Ubi::I-*Sce*I::t-Nos fragment from pJB083 into T4 DNA polymerase filled in *Asc*I digested pLM151.

### 1.7.4.2 Retransformation of reporter events

A homozygous event for each reporter construct, JB034 and JB039 underwent embryo rescue and was re-transformed with the I-SceI gene driven by the Super promoter, JB084. This promoter is believed to give higher expression in the germinating embryo and scutellum layer, which may improve the recovery of recombined plants. Asimilar retransformation set was performed with RLM319 and RLM320 using embryos from JB034 homozygous events. A total of 112 embryos were transformed with RLM319 and 100 embryos were transformed with RLM320.

### 1.7.4.3 Plant analysis for marker excision

A total of 16 lines containing the pseudo-marker gene, JB039 were recovered from the retransformation experiment with JB084. Nine lines were stained at the five-leaf stage and examined for white patches. Three lines showed leaves with a half white half blue pattern. The rest showed fully blue leaves. Leaves from six lines including one that had previously shown the half white pattern were stained at pollination and all showed fully blue leaves. A total of 11 lines containing the interrupted GUS gene, JB034 were recovered from the re-transformation experiment with JB084. All were stained for recombination but none showed any blue staining.

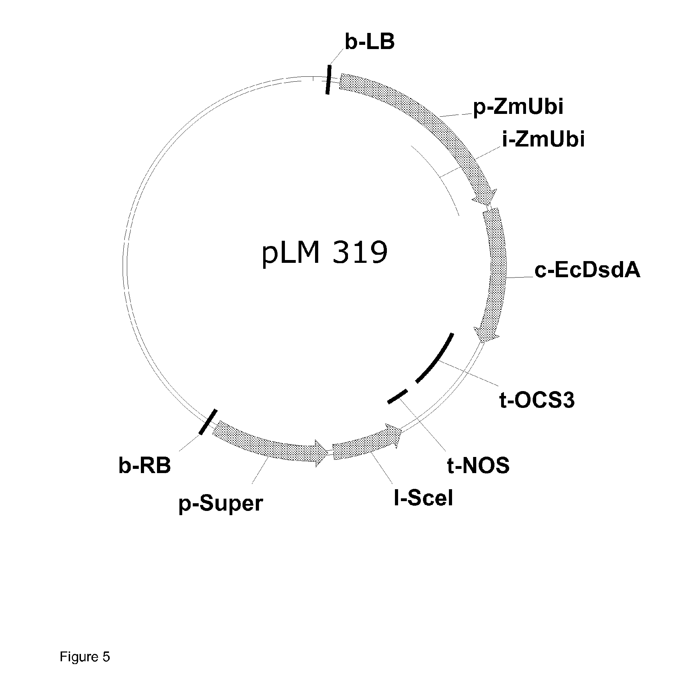

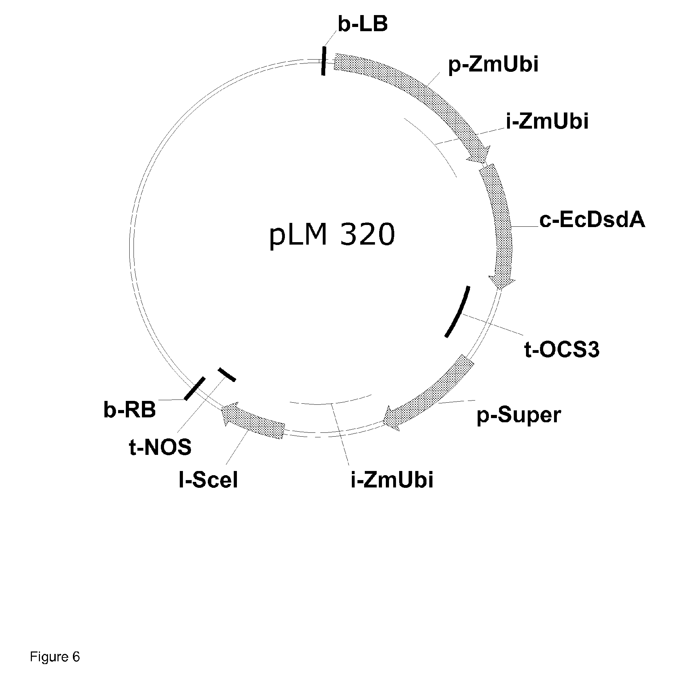

A total of 20 lines containing the interrupted GUS gene, JB034 were recovered from two retransformation experiments with LM319. A total of 28 lines containing the interrupted GUS gene, JB034 were recovered from two retransformation experiments with LM320. These retransformed plants were screened for the presence of the selectable marker and GUS stained to screen for recombination. Nine LM319 and 15 LM320 plants were GUS stained to screen for recombination. No homologous recombination positive events were recovered from the transformants produced using the Super promoter without the intron, JB084 or RLM319. Eleven positive events, with 3 being completely blue in tissue culture were identified from the transformation using the Super promoter with the intron, RLM320 (Table 15, Figures 5 and 6). Re-transformation data generated with the super promoter constructs indicated that super promoter in combination with intron (*i.e* Maize Ubiquitin intron in LM320) is effective in driving the expression of I-SceI gene to a functional level. Without this intron (LM319), the super promoter is ineffective.

Table 15. The construct used for each transformation and the number of confirmed lines. The GUS staining showed recombination occurred with the Super promoter coupled with the Ubiquitin intron while no recombinants were obtained using the super promoter without the intron.

| First Construct | Second Construct | embryos infected | Number of confirmed events | Recombined events/# of events | Fully recombined events |
|---|---|---|---|---|---|
| JB039 | JB084 | | 12 | 3/9 | 0 |
| JB034 | JB084 | | 11 | 0/11 | 0 |
| JB034 | LM319 | 112 | 7 | 0/9 | 0 |
| JB034 | LM320 | 100 | 12 | 11/15 | 3/15 |

JB034 transgenic plants were re-transformed with RLM320 or JB084 followed by selfing to set seed. None of the progeny containing JB084 showed homologous recombination. Leaves from a total of 15 T0 events containing RLM320 were tested for homologous recombination. Eleven out of 15 events showed homologous recombination via both GUS histochemical assay and PCR. Three out of 11 were fully recombined. Five out of the 11 events were tested in T1 generation. Three to four plants per event were analysed. Two out of a total of 12 T1 plants were fully recombined.

### 2. Application of minimaize as an efficient tool for determining frequency of marker excision in maize

In order to reduce the time to obtain the marker-free transgenic plants, a rapid cycling dwarf maize line can be utilized. This transformable dwarf line offers advantages over regular maize lines because it's small size and short life cycle - it completes a life cycle from seed to seed in about 60 days as compared to 120 days for the regular maize lines. This line is extremely useful in determining the HEN's marker excision efficiency in maize.

Transformation experiments are conducted mainly based the protocol with agrobacterium-mediated transformation procedure described in the Example 3. On the other hand, transformation experiments can also be conducted based on direct DNA delivery methods such as a biolistic transformation, e.g. particle bombardment known to the skilled in the art. Preparation of transformation donor materials also follows the procedure described above.

### BRIEF DESCRIPTION OF THE DRAWINGS

Figure 1. Vector pCER 040b. Binary I-SceI expression vector comprising the maize ubiquitin promoter/intron cassette driving the expression of I-SceI.

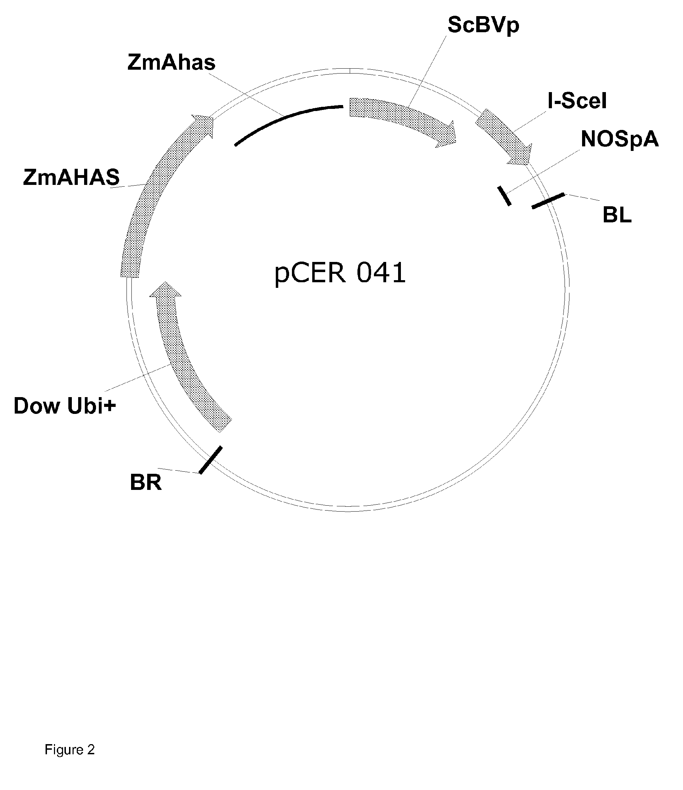

Figure 2. Vector pCER 041. Binary I-SceI expression vector comprising the ScBV promoter driving the expression of I-SceI
Figure 3. Vector pJB 034. Binary reporter vector comprising the GU-US reporter cassette.
Figure 4. Vector pJB 039. Binary reporter vector comprising the pseudo-marker excision cassette.
Figure 5. Vector pLM 319. Binary I-SceI expression vector comprising the Super promoter driving the expression of I-SceI.
Figure 6. Vector pLM 320. Binary I-SceI expression vector comprising I-SceI expression driven by the Super promoter in conjunction with the Zm ubiquitin intron.

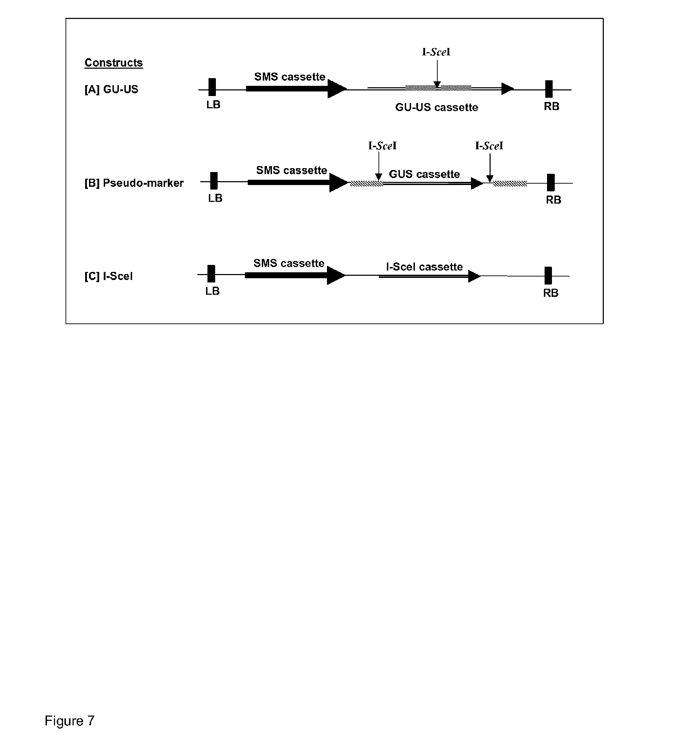

Figure 7. A diagram of the constructs used for DSB-induced homologous recombination. (A) GU-US construct encodes an expression cassette wherein the GUS ORF comprises an internal duplication (i.e. 650 bp of GUS coding sequence: hatched bars), with an I-*Sce*I recognition site located between the duplicated regions (GU-US). (B) Pseudo-marker excision vector comprises a duplicated DNA sequence (i.e. 850 bp of AHAS terminator region of the selectable marker cassette: gray bars) flanking the I-*Sce*I sites in order to serve as a target sequence for homologous recombination (HR target). (C) I-SceI construct comprises the I-SceI expression cassette. The T-DNA regions for all of these vectors also comprises a selectable marker cassette (SMS) in addition to the above described elements.
Figure 8. A selection process of identifying T0 lines that showing potential DSB-mediated HR using transient assays. Medium to high expressing lines comprising I-SceI (or GU-US) were transferred with GU-US (or I-SceI) construct. The lines showing GUS histochemical positive expression (blue spots) were selected. Young embryos in T0 plants were used for immature embryo conversion to identify homozygous lines, which sped up at least 1.5 months compared to the conventional maixze breeding timelin, because the seed development and maturation, seed-drying time is omitted. This transient assay process including immature embryo conversion allows not only a reduction in the overall time requirement but also an increased frequency of identification of candidate lines that show the potential for exhibiting a high rate of homologous recombination.

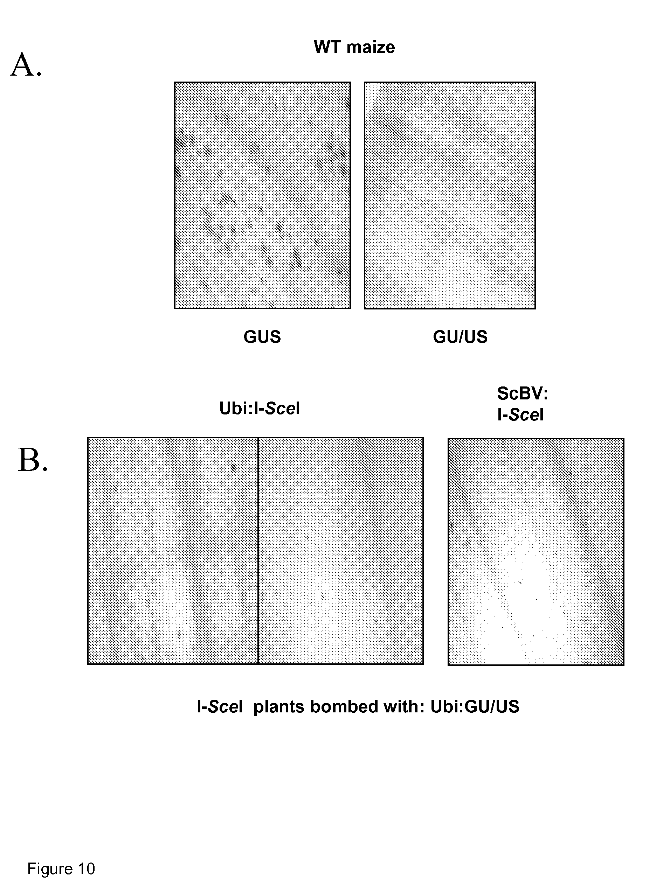

Figure 9. Approaches for identifying DSB-induced HR occurring in transgenic maize lines. Each method requires various range of time to obtain transgenic lines exhibiting DSB-mediated HR: Conventional method using crossing (A) requires minimum 19 months. Regeneration (B) and retransformation (C) methods require approximately 8-9 months. ***implies the transient assay system described in Figures 8 and 10.
Figure 10. Transient assay for DSB-induced homologous recombination in maize leaf tissue. (A) Wild type maize leaf tissue was bombarded with vectors comprising expression cassettes for either a functional GUS ORF (left) or the GU-US ORF (right), confirming that expression of GU-US does not result in detection of functional GUS by histochemical staining. (B) Bombardment of leaf tissues from I-SceI expressing plants with the GU-US expression cassette results in the generation of detectable GUS by histochemical staining. This result was seen in maize plants using both the ubiquitin promoter (left) and the ScBV promoter (right) to drive I-SceI expression.
Figure 11. Histochemical analysis of [JB034 X I-SceI] plant kernels. (A) Histochemical staining of kernels generated by crossing maize lines harboring the GU-US expression cassette with maize lines expressing I-SceI shows the generation of functional GUS. There is no GUS staining when seeds are analyzed from homozygous I-SceI expressing plants (bottom right well in left plate). (B) Histochemical staining of homozygous GU-US kernels demonstrates that in the absence of I-SceI expression, there is no generation of functional GUS protein.
Figure 12. Genomic PCR of [JB034 X I-SceI] plants, pooled samples. (A) Genomic DNA samples were prepared from [GU-US X I-SceI] plants and pooled. Similar pools of genomic DNA were prepared from self pollinated GU-US plants. Genomic PCR was performed as described in the examples. Positive control reactions using purified pJB034 vector generated the 1.7Kb product expected from the native construct; this reaction also generated a 1.0Kb product indicative of the recombined locus, indicating a low level of vector recombination during bacterial passages. Vector pCER044 is equivalent to vector pJB034 following homologous recombination at the GU-US locus, and yields the expected 1.0Kb PCR product. PCR amplification with genomic DNA from wild type maize plants does not result in the generation of any PCR product, demonstrating that the PCR products generated in these reactions are specific for the JB034 locus. Analysis of the genomic DNA pools shows that the 1.7Kb unrecombined product is produced with both the [JB034 X I-SceI] and the homozygous JB034 pools, but the recombined 1.0Kb product is only produced when the [JB034 X I-SceI] genomic DNA is used as a template. (B) Histochemical staining of kernel and leaf samples from homozygous JB034 plants (left) and [JB034 X I-SceI] plants (right), showing that homologous recombination of the GUS locus only is detectable in plants that express I-SceI.
Figure 13. Genomic PCR of [JB039 X I-SceI] plants, pooled samples. Genomic DNA pools were generated for [JB034 X I-SceI] (blue) and [JB034 C null] (red) plants. PCR was performed using primers 9 and 10 to confirm the presence of the JB039 template and with primers 9 and 11 to detect plants comprising cells that have undergone homologous recombination at this locus (left). PCR with primers 9 and 10 generates the expected 1.2Kb product from all samples, indicating that all genomic DNA pools comprise the native JB039 locus (top right). PCR with primers 9 and 11 generate the 0.9Kb product only in genomic DNA pools A2 and A4, each assembled from the [JB039 X I-SceI] crosses (bottom right).

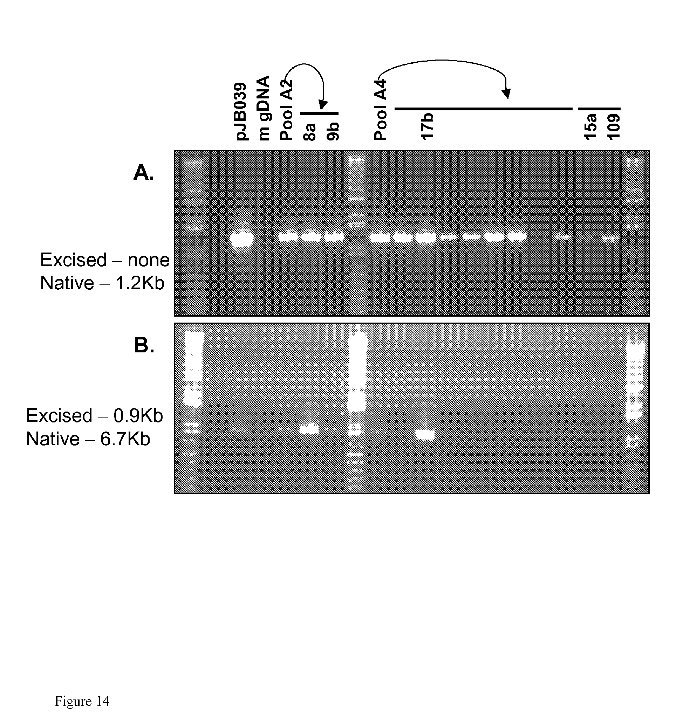

Figure 14. Genomic PCR of individual [JB039 x I-SceI] plants. Genomic DNA from individual [JB039 x I-SceI] plants was analyzed by PCR using primer combinations 9:10 (top) and 9:11 (bottom) as described in the examples. The control reactions (no DNA, vector control pJB039, and wild type maize genomic DNA) all produced the expected products from both primer sets. Analysis of genomic DNA pool A2 identified two individual plants that comprise the recombined JB039 locus, ie: plants 8a and 9b. Analysis of the plants that make up genomic DNA pool A4 shows that only plant 17b comprises the recombined locus. Lanes labeled 15a and 109 represent genomic DNA samples from individual [JB039 X I-SceI] plants that were not included in the previous genomic DNA pools.

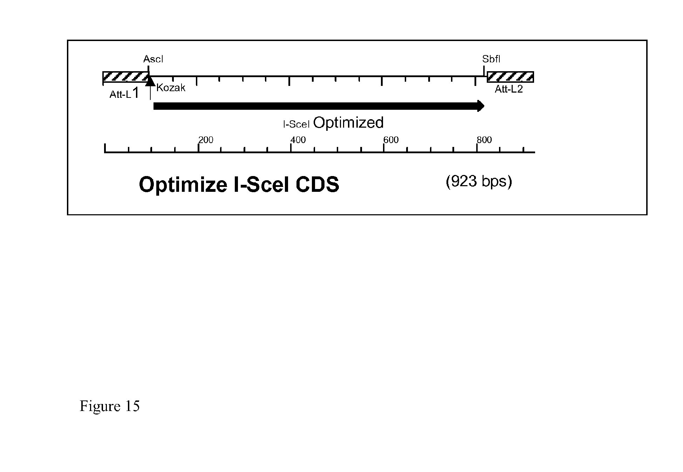

Figure 15. Graphic representation of the synthetic homing endonuclease I-SceI gene sequences. The Gateway attachment regions, Att-L1 and Att-L2 are depicted by the hashed boxes. The Kozak consensus is indicated by the vertical arrow and the open reading frame by the solid arrow. The location of selected restriction sites is indicated.

## Claims

1. A method for excising a nucleic acid sequence from the genome of a plant or of a plant cell, comprising:
a) transforming a plant cell with a construct encoding a DNA double strand break inducing enzyme,
b) generating a transgenic plant line from the cell of step a),
c) performing a transient assay with the plant line of step b) or cells or parts thereof to analyze the functionality of the transgenic DNA double strand break inducing enzyme, wherein the transient assay is an intrachromosomal homologous recombination assay and wherein the method further comprises the identification of a single copy transgenic line following step b) or c);
d) crossing the plant line of step b) with a plant line containing a nucleic acid sequence to be excised, wherein the nucleic acid sequence to be excised comprises at least one recognition sequence which is specific for the enzyme of step a) for the site-directed induction of DNA double strand breaks, and wherein the nucleic acid sequence to be excised is bordered at both sides by a repeated sequence which allows for a DNA repair mechanism, and
e) performing a tissue culture regeneration through callus formation of F1 immature embryos, wherein the seeds and/or seedlings obtained by step e) are analyzed for DNA double strand break mediated repair mechanisms including homologous recombination and lines in which double strand break-induced homologous recombination has occurred are identified.

2. A method for excising a nucleic acid sequence from the genome of a plant or of a plant cell, comprising:
a) transforming a plant cell with a construct encoding a nucleic acid sequence to be excised, wherein the nucleic acid sequence to be excised comprises at least one recognition sequence which is specific for a DNA double strand break inducing enzyme for the site-directed induction of DNA double strand breaks, and wherein the nucleic acid sequence to be excised is bordered at both sides by a repeated sequence which allows for a DNA repair mechanism,
b) generating a transgenic plant line from the cell of step a),
c) performing a transient assay with the plant line of step b) or cells or parts thereof to analyze the functionality of the recognition sequence and the repeated sequence of the construct of step a), wherein the transient assay is an intrachromosomal homologous recombination assay and wherein the method further comprises the identification of a single copy transgenic line following step b) or c);
d) crossing the plant line of step b) with a plant line containing a DNA double strand break inducing enzyme, and
e) performing a tissue culture regeneration through callus formation of F1 immature embryos, wherein the seeds and/or seedlings obtained by step e) are analyzed for DNA double strand break mediated repair mechanisms including homologous recombination and lines in which double strand break-induced homologous recombination has occurred are identified.

3. The method according to any of the preceding claims,
wherein the DNA double strand break inducing enzyme is selected from the group consisting of homing endonucleases, restriction endonucleases, group II endonucleases, recombinases, transposases and chimeric endonucleases.

4. The method according to any of the preceding claims,
wherein the construct of step a) comprises a promoter for the expression of the DNA double strand break inducing enzyme or for the expression of the nucleic acid sequence to be excised, respectively.

5. The method according to claim 4,
wherein the promoter is selected from the group consisting of constitutive promoters, development-dependent promoters, plant virus derived promoters, inducible promoters, chemically inducible promoters, biotic or abiotic stress inducible promoters, pathogen inducible promoters, tissue specific promoters, promoters with specificity for the embryo, scutellum, endosperm, embryo axis, anthers, ovaries, pollen, meristem, flowers, leaves, stems, roots, seeds, fruits and/or tubers, promoters which enable seed specific expression in monocotyledons including maize, barley, wheat, rye and rice, super promoters, and functional combinations of such promoters.

6. The method according to any of the preceding claims,
wherein the nucleic acid to be excised comprises the sequence of the T-DNA region or part thereof or encodes a selection marker or part thereof.

7. The method according to any of the preceding claims,
wherein the plant is selected from the group consisting of maize, *Arabidopsis,* sorghum, rice, rapeseed, tobacco, wheat, rye, barley, oat, potato, tomato, sugar beet, pea, sugarcane, asparagus, soy, alfalfa, peanut, sunflower and pumpkin.

8. The method according to any of the preceding claims,
as far as they relate to claim 1 or dependent claims, wherein the crossing of step d) is replaced by a re-transforming of the plant line of step b) with a construct encoding a nucleic acid sequence to be excised, wherein the nucleic acid sequence to be excised comprises at least one recognition sequence which is specific for the enzyme of step a) for the site-directed induction of DNA double strand breaks, and wherein the nucleic acid sequence to be excised is bordered at both sides by a repeated sequence which allows for a DNA repair mechanism.

9. The method according to any of the preceding claims, as far as they relate to claim 2 or dependent claims, wherein the crossing of step d) is replaced by a re-transforming of the plant line of step b) with a construct encoding a DNA double strand break inducing enzyme.

## Patentansprüche

1. Verfahren zum Ausschneiden einer Nukleinsäuresequenz aus dem Genom einer Pflanze oder Pflanzenzelle, umfassend:
a) Transformieren einer Pflanzenzelle mit einem Konstrukt, das ein DNA-Doppelstrangbruch induzierendes Enzym codiert ,
b) Erzeugen einer transgenen Pflanzenlinie aus der Zelle aus Schritt a),
c) Durchführen eines transienten Testverfahren mit der Pflanzenlinie aus Schritt b) oder Zellen oder Teilen davon zur Analyse der Funktionalität des transgenen DNA-Doppelstrangbruch induzierenden Enzyms, wobei es sich bei dem transienten Testverfahren um ein Testverfahren für intrachromosomale homologe Rekombination handelt und wobei das Verfahren ferner die Identifizierung einer Einzelkopie einer transgenen Linie nach Schritt b) oder c) umfasst;
d) Kreuzen der Pflanzenlinie aus Schritt b) mit einer Pflanzenlinie enthaltend eine auszuschneidende Nukleinsäuresequenz , wobei die auszuschneidende Nukleinsäuresequenz wenigstens eine Erkennungssequenz umfasst, die für das Enzym aus Schritt a) für die ortsgerichtete Induktion von DNA-Doppelstrangbrüchen spezifisch ist, und wobei an die auszuschneidende Nukleinsäuresequenz auf beiden Seiten eine Wiederholungssequenz grenzt, die einen DNA-Reparaturmechanismus erlaubt, und
e) Durchführen einer Gewebekulturregeneration über Kallusbildung unreifer F1-Embryos, wobei die durch Schritt e) erhaltenen Samen und/oder Sämlinge auf durch DNA-Doppelstrangbruch vermittelte Reparaturmechanismen, einschließlich homologe Rekombination, analysiert und Linien, in denen durch Doppelstrangbruch induzierte homologe Rekombiation stattgefunden hat, identifiziert werden.

2. Verfahren zum Ausschneiden einer Nukleinsäuresequenz aus dem Genom einer Pflanze oder Pflanzenzelle, umfassend:
a) Transformieren einer Pflanzenzelle mit einem eine Konstrukt, das eine auszuschneidende Nukleinsäuresequenz codiert, wobei die auszuschneidende Nukleinsäuresequenz wenigstens eine Erkennungssequenz umfasst, die für ein DNA-Doppelstrangbruch induzierendes Enzym für die orts-gerichtete Induktion von DNA-Doppelstrangbrüchen spezifisch ist, und wobei an die auszuschneidende Nukleinsäuresequenz auf beiden Seiten eine Wiederholungssequenz grenzt, die einen DNA-Reparaturmechanismus erlaubt,
b) Erzeugen einer transgenen Pflanzenlinie aus der Zelle aus Schritt a),
c) Durchführen eines transienten Testverfahren mit der Pflanzenlinie aus Schritt b) oder Zellen oder Teilen davon zur Analyse der Funktionalität der Erkennungssequenz und der Wiederholungssequenz des Konstrukts aus Schritt a), wobei es sich bei dem transienten Testverfahren um ein Testverfahren für intrachromosomale homologe Rekombination handelt und wobei das Verfahren ferner die Identifizierung einer Einzelkopie einer transgenen Linie nach Schritt b) oder c) umfasst;
d) Kreuzen der Pflanzenlinie aus Schritt b) mit einer Pflanzenlinie, enthaltend ein DNA-Doppelstrangbruch induzierendes Enzym und
e) Durchführen einer Gewebekulturregeneration über Kallusbildung unreifer F1-Embryos, wobei die durch Schritt e) erhaltenen Samen und/oder Sämlinge auf durch DNA-Doppelstrangbruch vermittelte Reparaturmechanismen, einschließlich homologe Rekombination, analysiert und Linien, in denen durch Doppelstrangbruch induzierte homologe Rekombiation stattgefunden hat, identifiziert werden.

3. Verfahren nach einem der vorhergehenden Ansprüche, wobei das DNA-Doppelstrangbruch induzierende Enzym aus der aus Homing-Endonukleasen, Restriktionsendonukleasen, Gruppe-II-Endonukleasen, Rekombinasen, Transposasen und chimären Endonukleasen bestehenden Gruppe ausgewählt ist.

4. Verfahren nach einem der vorhergehenden Ansprüche, wobei das Konstrukt aus Schritt a) einen Promotor für die Expression des DNA-Doppelstrangbruch induzierenden Enzyms bzw. für die Expression der auszuschneidenden Nukleinsäuresequenz umfasst.

5. Verfahren nach Anspruch 4,
wobei der Promotor aus der aus konstitutiven Promotoren, entwicklungsabhängigen Promotoren, von Pflanzenviren stammenden Promotoren, induzierbaren Promotoren, chemisch induzierbaren Promotoren, durch biotischen oder abiotischen Stress induzierbaren Promotoren, durch Krankheitserreger induzierbaren Promotoren, gewebespezifischen Promotoren, Promotoren mit Spezifität für den Embryo, das Scutellum, das Endosperm, die Embryoachse, die Antheren, die Fruchtknoten, den Pollen, das Meristem, die Blüten, die Blätter, die Stängel, die Wurzeln, die Samen, die Früchte und/oder die Knollen, Promotoren, die die samenspezifische Expression in Monokotyledonen, einschließlich Mais, Gerste, Weizen, Roggen und Reis ermöglichen, Superpromotoren und funktionellen Kombinationen solcher Promoteren bestehenden Gruppe ausgewählt ist.

6. Verfahren nach einem der vorhergehenden Ansprüche, wobei die auszuschneidende Nukleinsäuresequenz die Sequenz des T-DNA-Bereichs oder einen Teil davon umfasst oder einen Selektionsmarker oder einen Teil davon codiert.

7. Verfahren nach einem der vorhergehenden Ansprüche, wobei die Pflanze aus der aus Mais, *Arabidopsis,* Sorghum, Reis, Raps, Tabak, Weizen, Roggen, Gerste, Hafer, Kartoffel, Tomate, Zuckerrübe, Erbse, Zuckerrohr, Spargel, Soja, Alfalfa, Erdnuss, Sonnenblume und Kürbis bestehenden Gruppe ausgewählt ist.

8. Verfahren nach einem der vorhergehenden Ansprüche, soweit sie sich auf Anspruch 1 oder abhängige Ansprüche beziehen, wobei das Kreuzen in Schritt d) durch ein Neutransformieren der Pflanzenlinie aus Schritt b) mit einem Konstrukt. das eine auszuschneidende Nukleinsäuresequenz codiert ersetzt wird, wobei die auszuschneidende Nukleinsäuresequenz wenigstens eine Erkennungssequenz umfasst, die für das Enzym aus Schritt a) für die ortsgerichtete Induktion von DNA-Doppelstrangbrüchen spezifisch ist, und wobei an die auszuschneidende Nukleinsäuresequenz auf beiden Seiten eine Wiederholungssequenz grenzt, die einen DNA-Reparaturmechanismus erlaubt.

9. Verfahren nach einem der vorhergehenden Ansprüche, soweit sie sich auf Anspruch 2 oder abhängige Ansprüche beziehen, wobei das Kreuzen in Schritt d) durch ein Neutransformieren der Pflanzenlinie aus Schritt b) mit einem ein DNA-Doppelstrangbruch induzierendes Enzym codierenden Konstrukt ersetzt wird.

## Revendications

1. Procédé d'excision d'une séquence d'acide nucléique du génome d'une plante ou d'une cellule de plante, comprenant :
a) la transformation d'une cellule de plante avec une construction codant pour une enzyme induisant une rupture de double brin d'ADN,
b) la génération d'une lignée de plante transgénique à partir de la cellule de l'étape a),
c) la conduite d'un essai transitoire avec la lignée de plante de l'étape b) ou des cellules ou parties de celle-ci pour analyser la fonctionnalité de l'enzyme induisant une rupture de double brin d'ADN transgénique, l'essai transitoire étant un essai de recombinaison homologue intrachromosomique et le procédé comprenant en outre l'identification d'une lignée transgénique à copie unique après l'étape b) ou c) ;
d) le croisement de la lignée de plante de l'étape b) avec une lignée de plante contenant une séquence d'acide nucléique à exciser, la séquence d'acide nucléique à exciser comprenant au moins une séquence de reconnaissance qui est spécifique pour l'enzyme de l'étape a) pour l'induction dirigée de ruptures de double brin d'ADN, et la séquence d'acide nucléique à exciser étant bordée sur les deux côtés par une séquence répétée qui permet un mécanisme de réparation d'ADN, et
e) la conduite d'une régénération de culture de tissu par formation de cal d'embryons immatures F1, les graines et/ou semis obtenus par l'étape e) étant analysés pour des mécanismes de réparation médiés par rupture de double brin d'ADN comprenant une recombinaison homologue et des lignées dans lesquelles une recombinaison homologue induite par rupture de double brin s'est produite sont identifiées.

2. Procédé d'excision d'une séquence d'acide nucléique du génome d'une plante ou d'une cellule de plante, comprenant:
a) la transformation d'une cellule de plante avec une construction codant pour une séquence d'ADN à exciser, la séquence d'acide nucléique à exciser comprenant au moins une séquence de reconnaissance qui est spécifique une enzyme induisant une rupture de double brin d'ADN pour l'induction dirigée de ruptures de double brin d'ADN, et la séquence d'acide nucléique à exciser étant bordée sur les deux côtés par une séquence répétée qui permet un mécanisme de réparation d'ADN,
b) la génération d'une lignée de plante transgénique à partir de la cellule de l'étape a),
c) la conduite d'un essai transitoire avec la lignée de plante de l'étape b) ou des cellules ou parties de celle-ci pour analyser la fonctionnalité de la séquence de reconnaissance et la séquence répétée de la construction de l'étape a), l'essai transitoire étant un essai de recombinaison homologue intrachromosomique et le procédé comprenant en outre l'identification d'une lignée transgénique à copie unique après l'étape b) ou c) ;
d) le croisement de la lignée de plante de l'étape b) avec une lignée de plante contenant une enzyme induisant une rupture de double brin d'ADN, et
e) la conduite d'une régénération de culture de tissu par formation de cal d'embryons immatures F1, les graines et/ou semis obtenus par l'étape e) étant analysés pour des mécanismes de réparation médiés par rupture de double brin d'ADN comprenant une recombinaison homologue et des lignées dans lesquelles une recombinaison homologue induite par rupture de double brin s'est produite sont identifiées.

3. Procédé selon l'une quelconque des revendications précédentes,
dans lequel l'enzyme induisant une rupture de double brin d'ADN est choisie dans le groupe constitué d'endonucléases de homing, endonucléases de restriction, endonucléases du groupe II, recombinases, transposases et endonucléases chimériques.

4. Procédé selon l'une quelconque des revendications précédentes,
dans lequel la construction de l'étape a) comprend un promoteur pour l'expression de l'enzyme induisant une rupture de double brin d'ADN ou pour l'expression de la séquence d'acide nucléique à exciser, respectivement.

5. Procédé selon la revendication 4,
dans lequel le promoteur est choisi dans le groupe constitué de promoteurs constitutifs, promoteurs dépendants du développement, promoteurs dérivés de virus de plante, promoteurs inductibles, promoteurs chimiquement inductibles, promoteurs inductibles par stress biotique ou abiotique, promoteurs inductibles par agent pathogène, promoteurs tissu-spécifiques, promoteurs ayant une spécificité pour l'embryon, le scutellum, l'endosperme, l'axe de l'embryon, les anthères, les ovaires, le pollen, le méristème, les fleurs, les feuilles, les tiges, les racines, les graines, les fruits et/ou les tubercules, des promoteurs qui permettent une expression spécifique dans les graines chez des monocotylédones comprenant le maïs, l'orge, le blé, le seigle et le riz, des superpromoteurs, et des combinaisons fonctionnelles de tels promoteurs.

6. Procédé selon l'une quelconque des revendications précédentes,
dans lequel l'acide nucléique à exciser comprend la séquence de la région d'ADN-T ou une partie de celle-ci ou code pour un marqueur de sélection ou une partie de celui-ci.

7. Procédé selon l'une quelconque des revendications précédentes,
dans lequel la plante est choisie dans le groupe constitué des maïs, *Arabidopsis,* sorgo, riz, colza, tabac, blé, seigle, orge, avoine, pomme de terre, tomate, betterave sucrière, pois, canne à sucre, asperge, soja, luzerne, arachide, tournesol et potiron.

8. Procédé selon l'une quelconque des revendications précédentes,
dans la mesure où il est lié à la revendication 1 ou aux revendications dépendantes, dans lequel le croisement de l'étape d) est remplacé par une re-transformation de la lignée de plante de l'étape b) avec une construction codant pour une séquence d'acide nucléique à exciser, la séquence d'acide nucléique à exciser comprenant au moins une séquence de reconnaissance qui est spécifique pour l'enzyme de l'étape a) pour l'induction dirigée de ruptures de double brin d'ADN, et la séquence d'acide nucléique à exciser étant bordée sur les deux côtés par une séquence répétée qui permet un mécanisme de réparation d'ADN.

9. Procédé selon l'une quelconque des revendications précédentes,
dans la mesure où il est lié à la revendication 2 ou les revendications dépendantes, dans lequel le croisement de l'étape d) est remplacé par une re-transformation de la lignée de plante de l'étape b) avec une construction codant pour une enzyme induisant une rupture de double brin d'ADN.
